# EUROPEAN PATENT SPECIFICATION

(11) **EP 2 550 958 B1**
(45) Date of publication and mention of the grant of the patent: **10.10.2018**
(21) Application number: 12177932.6
(22) Date of filing: 05.08.2010
(51) Int. Cl.: A61K 9/16, A61K 9/00, A61K 38/00, C07K 14/47

(54) **Controlled release formulations of lipocalin muteins**
Retard-Formulierungen von Lipocalin-Muteinen
Formulations à libération contrôlée de mutéines de la lipocaline

(30) Priority: 05.08.2009 US 231365 P
(43) Date of publication of application: 30.01.2013
(62) Divisional of application: 10747840.6
(73) Proprietor: Pieris Pharmaceuticals GmbH, 85354 Freising-Weihenstephan (DE)
(72) Inventor: Hohlbaum, Andreas, 9000 Ghent (BE); Huelsmeyer, Martin, 67354 Roemerberg (DE); Gille, Hendrik, 50823 Köln (DE); Mantripragada, Sankaram, Bhima, Firestone, CO Colorado 80528 (US); Campbell, Kathleen, Marie, Firestone, CO Colorado 80504 (US)
(74) Representative: Schiweck Weinzierl Koch Patentanwälte Partnerschaft mbB

(56) References cited:
- WO-A1-2009/095447
- WO-A2-2005/019256
- WO-A2-2007/107563
- WO-A2-2008/015239
- US-A1- 2009 274 623

### SEQUENCE LISTING

<110> Pieris AG
<120> Controlled Release Formulations of Lipocalin Muteins
<130> PIE14005PCTEPD1
<140> Divisional of EP 10 747 840.6
   <141> 2012-07-25
<150> US 61/231,365
   <151> 2009-08-05
<160> 110
<170> PatentIn version 3.5
<210> 1
   <211> 154
   <212> PRT
   <213> Artificial
<220>
   <223> Mutein of human tear lipocalin with binding affinity for IL-4R alpha
<400> 1
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala Tyr Tyr Gly Ser Val
               20                  25                  30
   Thr Pro Met Thr Leu Thr Thr Leu Glu Gly Gly Asn Leu Glu Ala Lys
           35                  40                  45
   Val Thr Met Gln Arg Ile Gly Arg Ser Gln Glu Val Lys Ala Val Leu
       50                  55                  60
   Glu Lys Thr Asp Glu Pro Gly Lys Tyr Thr Ala Ser Gly Gly Arg His
   65                  70                  75                  80
   Val Ala Tyr Ile Ile Arg Ser His Val Lys Asp His Tyr Ile Phe Tyr
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 2
   <211> 154
   <212> PRT
   <213> Artificial
<220>
   <223> Mutein of human tear lipocalin with binding affinity for IL-4R alpha
<400> 2
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala Tyr Tyr Lys Ser Val
               20                  25                  30
   Thr Pro Met Thr Leu Thr Thr Leu Glu Gly Gly Asn Leu Glu Ala Lys
           35                  40                  45
   Phe Thr Ala Gln Arg Asn Gly Arg Trp Gln Glu Leu Lys Leu Val Leu
       50                  55                  60
   Glu Lys Thr Asp Glu Pro Gly Lys Tyr Ala Ala Ser Gly Gly Arg His
   65                  70                  75                  80
   Val Ala Tyr Ile Ile Arg Ser His Val Lys Asp His Tyr Ile Phe Tyr
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 3
   <211> 154
   <212> PRT
   <213> Artificial
<220>
   <223> Mutein of human tear lipocalin with binding affinity for IL-4R alpha
<400> 3
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala Tyr Tyr Glu Ser Val
               20                  25                  30
   Thr Pro Met Thr Leu Thr Thr Leu Glu Gly Gly Asn Leu Glu Ala Lys
           35                  40                  45
   Phe Thr Leu Gln Arg Arg Gly Arg Trp Gln Glu Gly Lys Leu Val Leu
       50                  55                  60
   Glu Lys Thr Asp Glu Pro Gly Lys Tyr Thr Ala Ser Gly Gly Arg His
   65                  70                  75                  80
   Val Ala Tyr Ile Ile Arg Ser His Val Lys Asp His Tyr Ile Phe Tyr
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 4
   <211> 154
   <212> PRT
   <213> Artificial
<220>
   <223> Mutein of human tear lipocalin with binding affinity for IL-4R alpha
<400> 4
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala Tyr Tyr Ser Ser Val
               20                  25                  30
   Thr Pro Met Thr Leu Thr Thr Leu Glu Gly Gly Asn Leu Glu Ala Lys
           35                  40                  45
   Phe Thr Ala Gln Arg Ser Gly Arg Trp Gln Glu Tyr Lys Leu Val Leu
       50                  55                  60
   Glu Lys Thr Asp Glu Pro Gly Lys Tyr Thr Ala Ser Gly Gly Arg His
   65                  70                  75                  80
   Val Ala Tyr Ile Ile Arg Ser His Val Lys Asp His Tyr Ile Phe His
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 5
   <211> 154
   <212> PRT
   <213> Artificial
<220>
   <223> Mutein of human tear lipocalin with binding affinity for IL-4R alpha
<400> 5
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala His Tyr Ser Ser Val
               20                  25                  30
   Thr Pro Met Thr Leu Thr Thr Leu Glu Gly Gly Asn Leu Glu Ala Lys
           35                  40                  45
   Leu Thr Leu Gln Arg Ala Gly Arg Trp Gln Glu Gly Lys Ile Val Leu
       50                  55                  60
   Glu Lys Thr Asp Glu Pro Gly Lys Tyr Thr Ala Ser Gly Gly Arg His
   65                  70                  75                  80
   Val Ala Tyr Ile Ile Arg Ser His Val Lys Asp His Tyr Ile Phe Tyr
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 6
   <211> 154
   <212> PRT
   <213> Artificial
<220>
   <223> Mutein of human tear lipocalin with binding affinity for IL-4R alpha
<400> 6
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala Tyr Tyr Asp Ser Val
               20                  25                  30
   Thr Pro Met Thr Leu Thr Thr Leu Glu Gly Gly Asn Leu Glu Ala Lys
           35                  40                  45
   Gly Thr Leu Gln Arg Lys Gly Arg Pro Gln Glu Met Lys Leu Val Leu
       50                  55                  60
   Glu Lys Thr Asp Glu Pro Gly Lys Tyr Thr Ala Ser Gly Gly Arg His
   65                  70                  75                  80
   Val Ala Tyr Ile Ile Arg Ser His Val Lys Asp His Tyr Ile Phe Tyr
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 7
   <211> 154
   <212> PRT
   <213> Artificial
<220>
   <223> Mutein of human tear lipocalin with binding affinity for IL-4R alpha
<400> 7
   Ala Ser Asp Glu Glu Ile Gln Asp Val Ser Gly Thr Trp Tyr Leu Lys
   1               5                   10                  15
   Ala Met Thr Val Asp Ser Arg Cys Pro Arg Ala Tyr Tyr Gly Ser Val
               20                  25                  30
   Thr Pro Met Thr Leu Thr Thr Leu Glu Gly Gly Asn Leu Glu Ala Lys
           35                  40                  45
   Leu Thr Leu Gln Arg Ser Gly Arg Trp Gln Glu Ser Lys Val Val Leu
       50                  55                  60
   Glu Lys Thr Asp Glu Pro Gly Lys Tyr Thr Ala Ser Gly Gly Arg His
   65                  70                  75                  80
   Val Ala Tyr Ile Ile Arg Ser His Val Lys Asp His Tyr Ile Phe Tyr
                   85                  90                  95
   Ser Glu Gly Leu Cys Pro Gly Gln Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 8
   <211> 154
   <212> PRT
   <213> Artificial
<220>
   <223> Mutein of human tear lipocalin with binding affinity for VEGF
<400> 8
   Ala Ser Asp Glu Glu Ile Gln Asp Val Ser Gly Thr Trp Tyr Leu Lys
   1               5                   10                  15
   Ala Met Thr Val Asp Ser Gly Ala Leu Arg Cys Leu Ala Gly Ser Val
                   20                  25                  30
   Thr Pro Met Thr Leu Thr Thr Leu Glu Gly Gly Asn Leu Glu Ala Lys
           35                  40                  45
   Val Thr Met His Ile Lys Gly Arg Ser Gln Glu Val Lys Ala Val Leu
       50                  55                  60
   Glu Lys Thr Asp Glu Pro Gly Lys Tyr Thr Ala Ile Gly Gly Ile His
   65                  70                  75                  80
   Val Ala Tyr Ile Ile Arg Ser His Val Lys Asp His Tyr Ile Phe Tyr
                   85                  90                  95
   Ser Glu Gly Cys Leu Asn Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 9
   <211> 154
   <212> PRT
   <213> Artificial
<220>
   <223> Mutein of human tear lipocalin with binding affinity for VEGF
<400> 9
   Ala Ser Asp Glu Glu Ile Gln Asp Val Ser Gly Thr Trp Tyr Leu Lys
   1               5                   10                  15
   Ala Met Thr Val Asp Pro Gly Ala Leu Arg Cys Leu Ala Gly Ser Val
               20                  25                  30
   Thr Pro Thr Thr Leu Thr Thr Leu Glu Gly Gly Asn Leu Glu Ala Lys
           35                  40                  45
   Val Thr Met His Ile Glu Gly Arg Ser Gln Glu Val Lys Val Val Leu
       50                  55                  60
   Gly Lys Thr Asp Glu Pro Gly Lys Tyr Thr Ala Ile Gly Gly Ile His
   65                  70                  75                  80
   Val Ala Tyr Ile Ile Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 10
   <211> 154
   <212> PRT
   <213> Artificial
<220>
   <223> Mutein of human tear lipocalin with binding affinity for VEGF
<400> 10
   Ala Ser Asp Glu Glu Ile Gln Asp Val Ser Gly Thr Trp Tyr Leu Lys
   1               5                   10                  15
   Ala Met Thr Val Asp Ser Gly Ala Leu Arg Cys Leu Ala Gly Ser Val
               20                  25                  30
   Thr Pro Thr Thr Leu Thr Thr Leu Glu Gly Gly Asn Leu Glu Val Lys
           35                  40                  45
   Val Thr Met His Ile Lys Gly Arg Ser Gln Glu Val Lys Ala Val Leu
       50                  55                  60
   Gly Lys Thr Asp Glu Pro Gly Lys Tyr Thr Ala Ile Gly Gly Ile His
   65                  70                  75                  80
   Val Ala His Ile Thr Arg Ser His Val Lys Asp His Tyr Val Phe Tyr
                   85                  90                  95
   Ser Glu Gly Cys Leu Asn Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 11
   <211> 154
   <212> PRT
   <213> Artificial
<220>
   <223> Mutein of human tear lipocalin with binding affinity for VEGF
<400> 11
   Ala Ser Asp Glu Glu Ile Gln Asp Val Ser Gly Thr Trp Tyr Leu Lys
   1               5                   10                  15
   Ala Met Thr Val Asp Pro Gly Ala Leu Arg Cys Leu Ala Gly Ser Ala
               20                  25                  30
   Thr Pro Met Ala Leu Thr Thr Leu Glu Gly Gly Asn Leu Glu Ala Arg
           35                  40                  45
   Val Thr Val Arg Ile Lys Gly Arg Ser Gln Glu Val Lys Ala Ile Leu
       50                  55                  60
   Glu Lys Thr Asp Glu Pro Gly Arg Tyr Thr Ala Ile Gly Gly Ile His
   65                  70                  75                  80
   Val Ala Tyr Ile Thr Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 12
   <211> 154
   <212> PRT
   <213> Artificial
<220>
   <223> Mutein of human tear lipocalin with binding affinity for VEGF
<400> 12
   Ala Ser Asp Glu Glu Ile Gln Asp Val Ser Gly Thr Trp Tyr Leu Lys
   1               5                   10                  15
   Ala Met Thr Val Asp Pro Gly Ala Leu Arg Cys Leu Ala Gly Ser Val
               20                  25                  30
   Ala Pro Met Ala Leu Thr Thr Leu Glu Gly Gly Asn Leu Glu Ala Lys
           35                  40                  45
   Val Thr Met His Ile Lys Gly Arg Ser Gln Glu Val Lys Ala Ile Leu
       50                  55                  60
   Glu Lys Thr Asp Glu Pro Gly Lys Tyr Thr Ala Ile Gly Gly Ile His
   65                  70                  75                  80
   Val Ala Arg Ile Ile Gly Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 13
   <211> 154
   <212> PRT
   <213> Artificial
<220>
   <223> Mutein of human tear lipocalin with binding affinity for VEGF
<400> 13
   Ala Ser Asp Glu Glu Ile Gln Asp Val Ser Gly Thr Trp Tyr Leu Lys
   1               5                   10                  15
   Ala Met Thr Val Asp Pro Gly Ala Leu Arg Cys Leu Ala Gly Ser Val
               20                  25                  30
   Thr Pro Met Thr Leu Thr Thr Leu Glu Gly Gly Asn Leu Glu Ala Lys
           35                  40                  45
   Val Val Val His Ile Lys Gly Arg Ser Gln Glu Val Arg Ala Val Leu
       50                  55                  60
   Gly Lys Thr Asp Glu Pro Gly Lys Tyr Thr Ala Ile Gly Gly Ile His
   65                  70                  75                  80
   Val Ala Tyr Ile Thr Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 14
   <211> 154
   <212> PRT
   <213> Artificial
<220>
   <223> Mutein of human tear lipocalin with binding affinity for VEGF
<400> 14
   Ala Ser Asp Glu Glu Ile Gln Asp Val Ser Gly Thr Trp Tyr Leu Lys
   1               5                   10                  15
   Ala Met Thr Val Asp Pro Gly Ala Leu Arg Cys Leu Ala Gly Ser Val
               20                  25                  30
   Thr Pro Met Thr Leu Thr Thr Leu Glu Gly Gly Asn Leu Glu Ala Lys
           35                  40                  45
   Val Thr Met His Ile Lys Gly Arg Pro Gln Glu Val Lys Ala Val Leu
       50                  55                  60
   Gly Lys Thr Asp Glu Pro Gly Lys Tyr Thr Ala Ile Gly Gly Ile His
   65                  70                  75                  80
   Val Ala Tyr Ile Val Arg Ser His Val Lys Asp His Tyr Ile Phe Tyr
                   85                  90                  95
   Ser Glu Gly Cys Leu Ser Glu Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 15
   <211> 154
   <212> PRT
   <213> Artificial
<220>
   <223> Mutein of human tear lipocalin with binding affinity for VEGF
<400> 15
   Ala Ser Asp Glu Glu Ile Gln Asp Val Ser Gly Thr Trp Tyr Leu Lys
   1               5                   10                  15
   Ala Met Thr Val Asp Ser Gly Ala Leu Arg Cys Leu Ala Gly Ser Val
               20                  25                  30
   Ile Pro Thr Thr Leu Thr Thr Leu Glu Gly Gly Asn Leu Glu Ala Lys
           35                  40                  45
   Val Thr Met His Ile Lys Gly Arg Ser Gln Glu Val Lys Ala Val Leu
       50                  55                  60
   Glu Lys Thr Asp Glu Pro Gly Lys Tyr Thr Ala Ile Gly Gly Ile His
   65                  70                  75                  80
   Val Ala His Ile Ile Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 16
   <211> 154
   <212> PRT
   <213> Artificial
<220>
   <223> Mutein of human tear lipocalin with binding affinity for VEGFR2
<400> 16
   Ala Ser Asp Glu Glu Ile Gln Asp Val Ser Gly Thr Trp Tyr Leu Lys
   1               5                   10                  15
   Ala Met Thr Val Asp Ser Ile Phe Pro Ser Gly Arg Ile Tyr Ser Val
               20                  25                  30
   Thr Pro Met Thr Leu Thr Thr Leu Glu Gly Gly Asn Leu Glu Ala Lys
           35                  40                  45
   Val Thr Met Lys Phe Arg Gly Arg Ser Gln Glu Val Lys Ala Val Leu
       50                  55                  60
   Glu Lys Thr Asp Glu Pro Gly Lys Tyr Thr Ala Ser Gly Gly Glu His
   65                  70                  75                  80
   Val Ala Tyr Ile Ile Arg Ser His Val Lys Asp His Tyr Ile Phe Tyr
                   85                  90                  95
   Ser Glu Gly Leu Ala Val Gly Thr Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 17
   <211> 154
   <212> PRT
   <213> Artificial
<220>
   <223> Mutein of human tear lipocalin with binding affinity for VEGFR2
<400> 17
   Ala Ser Asp Glu Glu Ile Gln Asp Val Ser Gly Thr Trp Tyr Leu Lys
   1               5                   10                  15
   Ala Met Thr Val Asp Ser Ile Phe Pro Ser Gly Arg Ile Tyr Ser Val
               20                  25                  30
   Thr Pro Met Thr Leu Thr Thr Leu Glu Gly Gly Asn Leu Glu Ala Lys
           35                  40                  45
   Val Thr Met Glu Phe Arg Gly Arg Ser Gln Glu Val Lys Ala Val Leu
       50                  55                  60
   Glu Lys Thr Asp Glu Pro Gly Lys Tyr Thr Ala Ser Gly Gly Glu His
   65                  70                  75                  80
   Val Ala Tyr Ile Ile Arg Ser His Val Lys Asp His Tyr Ile Phe Tyr
                   85                  90                  95
   Ser Glu Gly Leu Ala Val Arg Thr Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 18
   <211> 154
   <212> PRT
   <213> Artificial
<220>
   <223> Mutein of human tear lipocalin with binding affinity for VEGFR2
<400> 18
   Ala Ser Asp Glu Glu Ile Gln Asp Val Ser Gly Thr Trp Tyr Leu Lys
   1               5                   10                  15
   Ala Met Thr Val Asp Ser Ile Phe Pro Ser Gly Arg Ile Tyr Ser Val
               20                  25                  30
   Thr Pro Met Thr Leu Thr Thr Leu Glu Gly Gly Asn Leu Glu Ala Lys
           35                  40                  45
   Val Thr Met Ala Phe Arg Gly Arg Ser Gln Glu Met Lys Ala Val Leu
       50                  55                  60
   Glu Lys Thr Asp Glu Pro Gly Lys Tyr Thr Ala Ser Gly Gly Glu His
   65                  70                  75                  80
   Val Ala Tyr Ile Ile Arg Ser His Val Lys Asp His Tyr Ile Phe Tyr
                   85                  90                  95
   Ser Glu Gly Leu Ala Val Gly Thr Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 19
   <211> 154
   <212> PRT
   <213> Artificial
<220>
   <223> Mutein of human tear lipocalin with binding affinity for VEGFR2
<400> 19
   Ala Ser Asp Glu Glu Ile Gln Asp Val Ser Gly Thr Trp Tyr Leu Lys
   1               5                   10                  15
   Ala Met Thr Val Asp Ser Ile Phe Pro Ser Gly Arg Ile Tyr Ser Val
               20                  25                  30
   Thr Pro Met Thr Leu Thr Thr Leu Glu Gly Gly Asn Leu Glu Ala Lys
           35                  40                  45
   Val Thr Met Glu Phe Arg Gly Arg Ser Gln Glu Val Lys Ala Val Leu
       50                  55                  60
   Glu Lys Thr Gly Glu Pro Gly Lys Tyr Thr Ala Pro Gly Gly Glu His
   65                  70                  75                  80
   Val Ala Tyr Ile Ile Arg Ser His Val Lys Asp His Tyr Ile Phe Tyr
                   85                  90                  95
   Ser Glu Gly Leu Ala Val Gly Thr Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 20
   <211> 154
   <212> PRT
   <213> Artificial
<220>
   <223> Mutein of human tear lipocalin with binding affinity for VEGFR2
<400> 20
   Ala Ser Asp Glu Glu Ile Gln Asp Val Ser Gly Thr Trp Tyr Leu Lys
   1               5                   10                  15
   Ala Met Thr Val Asp Ser Ile Phe Pro Ser Gly Arg Ile Tyr Ser Val
               20                  25                  30
   Thr Pro Met Thr Leu Thr Thr Leu Glu Gly Gly Asn Leu Glu Ala Lys
           35                  40                  45
   Val Thr Met Glu Phe Arg Gly Arg Ser Gln Glu Val Lys Ala Val Leu
       50                  55                  60
   Glu Lys Thr Asp Glu Pro Gly Arg Tyr Thr Ala Ser Gly Gly Glu His
   65                  70                  75                  80
   Val Ala Tyr Ile Thr Arg Ser His Val Lys Asp His Tyr Ile Phe Tyr
                   85                  90                  95
   Ser Glu Gly Leu Ala Val Lys Thr Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 21
   <211> 154
   <212> PRT
   <213> Artificial
<220>
   <223> Mutein of human tear lipocalin with binding affinity for VEGFR2
<400> 21
   Ala Ser Asp Glu Glu Ile Gln Asp Val Ser Gly Thr Trp Tyr Leu Lys
   1               5                   10                  15
   Ala Met Thr Val Asp Ser Ile Phe Pro Ser Gly Arg Ile Tyr Ser Val
                   20                  25                  30
   Thr Pro Met Thr Leu Thr Thr Leu Glu Gly Gly Asn Leu Glu Ala Lys
           35                  40                  45
   Val Thr Met Glu Phe Arg Gly Arg Ser Gln Lys Val Lys Ala Val Leu
       50                  55                  60
   Glu Lys Thr Asp Glu Pro Gly Lys Tyr Thr Ala Ser Gly Gly Glu His
   65                  70                  75                  80
   Val Ala Tyr Ile Ile Lys Ser His Val Lys Asp His Tyr Ile Phe Tyr
                   85                  90                  95
   Ser Glu Gly Leu Ala Val Glu Thr Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala
   145                 150
<210> 22
   <211> 162
   <212> PRT
   <213> Artificial
<220>
   <223> Mutein of human tear lipocalin with binding affinity for VEGF
<400> 22
   Ala Ser Asp Glu Glu Ile Gln Asp Val Ser Gly Thr Trp Tyr Leu Lys
   1               5                   10                  15
   Ala Met Thr Val Asp Val Gly Ala Leu Arg Cys Leu Ala Gly Ser Val
               20                  25                  30
   Ile Pro Thr Thr Leu Thr Thr Leu Glu Gly Gly Asn Leu Glu Ala Lys
           35                  40                  45
   Val Thr Met His Ile Lys Gly Arg Ser Gln Glu Val Lys Ala Val Leu
       50                  55                  60
   Ser Lys Thr Asp Glu Pro Gly Ile Tyr Thr Ala Ile Gly Gly Ile His
   65                  70                  75                  80
   Val Ala Lys Ile Gly Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala Trp Ser His Pro Gln Phe
   145                 150                 155                 160
   Glu Lys
<210> 23
   <211> 162
   <212> PRT
   <213> Artificial
<220>
   <223> Mutein of human tear lipocalin with binding affinity for VEGF
<400> 23
   Ala Ser Asp Glu Glu Ile Gln Asp Val Ser Gly Thr Trp Tyr Leu Lys
   1               5                   10                  15
   Ala Met Thr Val Asp Val Gly Ala Leu Arg Cys Leu Ala Gly Ser Val
               20                  25                  30
   Ile Pro Thr Thr Leu Thr Thr Leu Glu Gly Gly Asn Leu Glu Ala Lys
           35                  40                  45
   Val Thr Met His Ile Lys Gly Arg Pro Gln Glu Val Lys Ala Val Leu
       50                  55                  60
   Thr Lys Thr Asp Glu Pro Gly Ala Tyr Thr Ala Ile Gly Gly Ile His
   65                  70                  75                  80
   Val Ala Gln Ile His Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala Trp Ser His Pro Gln Phe
   145                 150                 155                 160
   Glu Lys
<210> 24
   <211> 162
   <212> PRT
   <213> Artificial
<220>
   <223> Mutein of human tear lipocalin with binding affinity for VEGF
<400> 24
   Ala Ser Asp Glu Glu Ile Gln Asp Val Ser Gly Thr Trp Tyr Leu Lys
   1               5                   10                  15
   Ala Met Thr Val Asp Leu Gly Ala Leu Arg Cys Leu Ala Gly Ser Val
               20                  25                  30
   Ile Pro Thr Ser Leu Thr Thr Leu Glu Gly Gly Asp Leu Glu Ala Lys
           35                  40                  45
   Val Thr Met His Ile Lys Gly Arg Ser Gln Glu Val Lys Ala Val Leu
       50                  55                  60
   Ser Lys Thr Asp Glu Pro Gly Met Tyr Thr Ala Ile Gly Gly Ile His
   65                  70                  75                  80
   Val Ala Arg Ile Met Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala Trp Ser His Pro Gln Phe
   145                 150                 155                 160
   Glu Lys
<210> 25
   <211> 162
   <212> PRT
   <213> Artificial
<220>
   <223> Mutein of human tear lipocalin with binding affinity for VEGF
<400> 25
   Ala Ser Asp Glu Glu Ile Gln Asp Val Ser Gly Thr Trp Tyr Leu Lys
   1               5                   10                  15
   Ala Met Thr Val Asp Ile Gly Ala Leu Arg Cys Leu Ala Gly Ser Val
               20                  25                  30
   Thr Pro Met Thr Leu Thr Thr Leu Glu Gly Gly Asn Leu Glu Ala Lys
           35                  40                  45
   Val Val Val His Ile Lys Gly Arg Ser Gln Glu Val Arg Ala Val Leu
       50                  55                  60
   Ser Lys Thr Asp Glu Pro Gly Pro Tyr Thr Ala Ile Gly Gly Ile His
   65                  70                  75                  80
   Val Ala Lys Ile Lys Arg Ser His Val Lys Asp His Tyr Ile Phe Tyr
                   85                  90                  95
   Ser Glu Gly Cys Leu Ser Gly Val Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly Ser Ala Trp Ser His Pro Gln Phe
   145                 150                 155                 160
   Glu Lys
<210> 26
   <211> 238
   <212> PRT
   <213> Artificial
<220>
   <223> Fusion protein of ompA, a human tear lipocalin mutein with binding affinity for VEGF , albumin-binding domain (abd) and Strep-Tag II
<400> 26
   Met Lys Lys Thr Ala Ile Ala Ile Ala Val Ala Leu Ala Gly Phe Ala
   1               5                   10                  15
   Thr Val Ala Gln Ala Ala Ser Asp Glu Glu Ile Gln Asp Val Ser Gly
               20                  25                  30
   Thr Trp Tyr Leu Lys Ala Met Thr Val Asp Val Gly Ala Leu Arg Cys
           35                  40                  45
   Leu Ala Gly Ser Val Ile Pro Thr Thr Leu Thr Thr Leu Glu Gly Gly
       50                  55                  60
   Asn Leu Glu Ala Lys Val Thr Met His Ile Lys Gly Arg Ser Gln Glu
   65                  70                  75                  80
   Val Lys Ala Val Leu Ser Lys Thr Asp Glu Pro Gly Ile Tyr Thr Ala
                   85                  90                  95
   Ile Gly Gly Ile His Val Ala Lys Ile Gly Arg Ser His Val Lys Asp
               100                 105                 110
   His Tyr Ile Phe Tyr Ser Glu Gly Cys Leu Ser Gly Val Pro Val Pro
           115                 120                 125
   Gly Val Trp Leu Val Gly Arg Asp Pro Lys Asn Asn Leu Glu Ala Leu
       130                 135                 140
   Glu Asp Phe Glu Lys Ala Ala Gly Ala Arg Gly Leu Ser Thr Glu Ser
   145                 150                 155                 160
   Ile Leu Ile Pro Arg Gln Ser Glu Thr Ser Ser Pro Gly Ser Ala Gly
                   165                 170                 175
   Ala Val Asp Ala Asn Ser Leu Ala Glu Ala Lys Val Leu Ala Asn Arg
               180                 185                 190
   Glu Leu Asp Lys Tyr Gly Val Ser Asp Tyr Tyr Lys Asn Leu Ile Asn
           195                 200                 205
   Asn Ala Lys Thr Val Glu Gly Val Lys Ala Leu Ile Asp Glu Ile Leu
       210                 215                 220
   Ala Ala Leu Pro Ser Ala Trp Ser His Pro Gln Phe Glu Lys
   225                 230                 235
<210> 27
   <211> 152
   <212> PRT
   <213> Artificial
<220>
   <223> Mutein of human tear lipocalin with binding affinity for c-Met
<400> 27
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Met Thr Leu Thr Thr Leu Glu Gly Gly Asn Leu Glu Ala Lys
           35                  40                  45
   Val Thr Met Asn Gln Ile Gly Arg Ser Gln Glu Val Lys Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ile Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 28
   <211> 152
   <212> PRT
   <213> Artificial
<220>
   <223> Mutein of human tear lipocalin with binding affinity for c-Met
<400> 28
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Thr Leu Thr Thr Leu Glu Gly Gly Ser Leu Glu Ala Thr
           35                  40                  45
   Val Thr Leu Asn Gln Ile Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ser Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 29
   <211> 152
   <212> PRT
   <213> Artificial
<220>
   <223> Mutein of human tear lipocalin with binding affinity for c-Met
<400> 29
   Ala Ser Asp Glu Glu Ile Gln Asp Val Ser Gly Thr Trp Tyr Leu Lys
   1               5                   10                  15
   Ala Met Thr Val Asp Thr Gln Thr Pro Leu Ser Leu Tyr Val Ser Val
               20                  25                  30
   Thr Pro Ile Thr Leu Thr Thr Leu Glu Gly Gly Asn Leu Glu Ala Glu
           35                  40                  45
   Val Thr Leu Asn Gln Val Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ile Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 30
   <211> 152
   <212> PRT
   <213> Artificial
<220>
   <223> Mutein of human tear lipocalin with binding affinity for c-Met
<400> 30
   Ala Ser Asp Glu Glu Ile Gln Asp Val Ser Gly Thr Trp Tyr Leu Lys
   1               5                   10                  15
   Ala Met Thr Val Asp Pro Gln Met Pro Leu Ser Leu Tyr Val Ser Val
               20                  25                  30
   Thr Pro Met Thr Leu Thr Thr Leu Glu Gly Gly Asn Leu Glu Ala Glu
           35                  40                  45
   Val Thr Leu Asn Gln Val Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Thr Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
                       145                 150
<210> 31
   <211> 152
   <212> PRT
   <213> Artificial
<220>
   <223> Mutein of human tear lipocalin with binding affinity for c-Met
<400> 31
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Met Thr Leu Thr Thr Leu Glu Gly Gly Asn Leu Glu Ala Glu
           35                  40                  45
   Val Thr Leu Asn Gln Ile Gly Arg Ser Gln Glu Val Arg Ala Val Leu
       50                  55                  60
   Glu Lys Thr Asp Glu Pro Gly Arg Tyr Thr Ala Tyr Gly Gly Ala His
   65                  70                  75                  80
   Val Ala Tyr Ile Thr Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 32
   <211> 152
   <212> PRT
   <213> Artificial
<220>
   <223> Mutein of human tear lipocalin with binding affinity for c-Met
<400> 32
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Thr Leu Thr Ala Leu Glu Gly Gly Asn Leu Glu Ala Glu
           35                  40                  45
   Val Thr Leu Asn Gln Val Gly Arg Ser Gln Glu Val Lys Ala Val Leu
       50                  55                  60
   Glu Lys Thr Asp Glu Pro Gly Arg Tyr Thr Ala Tyr Gly Gly Ala His
   65                  70                  75                  80
   Val Ala Tyr Ile Thr Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 33
   <211> 152
   <212> PRT
   <213> Artificial
<220>
   <223> Mutein of human tear lipocalin with binding affinity for c-Met
<400> 33
   Ala Ser Asp Glu Glu Ile Gln Asp Val Ser Gly Thr Trp Tyr Leu Lys
   1               5                   10                  15
   Ala Met Thr Val Asp Thr Gln Val Pro Leu Ser Leu Tyr Val Ser Val
               20                  25                  30
   Thr Pro Ile Thr Leu Thr Thr Leu Glu Gly Gly Asn Leu Glu Ala Glu
           35                  40                  45
   Val Thr Met Asn Gln Ile Gly Arg Ser Gln Glu Val Arg Ala Val Leu
       50                  55                  60
   Glu Lys Thr Asp Glu Pro Gly Arg Tyr Thr Ala Tyr Gly Gly Ala His
   65                  70                  75                  80
   Val Ala Tyr Ile Thr Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 34
   <211> 152
   <212> PRT
   <213> Artificial
<220>
   <223> Mutein of human tear lipocalin with binding affinity for c-Met
<400> 34
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Cys Leu Thr Thr Leu Glu Gly Gly Ser Leu Glu Ala Thr
           35                  40                  45
   Val Thr Leu Asn Gln Ile Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ser Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 35
   <211> 152
   <212> PRT
   <213> Artificial
<220>
   <223> Mutein of human tear lipocalin with binding affinity for c-Met
<400> 35
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Thr Leu Thr Thr Leu Glu Gly Gly Ser Leu Glu Ala Thr
           35                  40                  45
   Val Thr Leu Asn Gln Ile Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ser Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Cys Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 36
   <211> 152
   <212> PRT
   <213> Artificial
<220>
   <223> Mutein of human tear lipocalin with binding affinity for c-Met
<400> 36
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Thr Leu Thr Thr Leu Glu Gly Gly Ser Leu Glu Ala Thr
           35                  40                  45
   Val Thr Leu Asn Gln Ile Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ser Arg Ser His Val Lys Cys His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 37
   <211> 152
   <212> PRT
   <213> Artificial
<220>
   <223> Mutein of human tear lipocalin with binding affinity for c-Met
<400> 37
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Thr Leu Thr Thr Leu Glu Gly Gly Ser Leu Glu Ala Thr
           35                  40                  45
   Val Thr Leu Asn Gln Ile Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ser Cys Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 38
   <211> 152
   <212> PRT
   <213> Artificial
<220>
   <223> Mutein of human tear lipocalin with binding affinity for c-Met
<400> 38
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
               20                  25                  30
   Thr Pro Ile Thr Leu Thr Thr Leu Glu Gly Gly Ser Leu Glu Ala Thr
           35                  40                  45
   Val Thr Leu Asn Gln Ile Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ser Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Cys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 39
   <211> 152
   <212> PRT
   <213> Artificial
<220>
   <223> Mutein of human tear lipocalin with binding affinity for c-Met
<400> 39
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Thr Pro Ile Thr Leu Thr Thr Leu Glu Gly Gly Asn Leu Glu Ala Met
           35                  40                  45
   Val Thr Leu Asn Gln Val Gly Arg Ser Gln Glu Val Leu Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 40
   <211> 152
   <212> PRT
   <213> Artificial
<220>
   <223> Mutein of human tear lipocalin with binding affinity for c-Met
<400> 40
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Thr Pro Leu Thr Leu Thr Thr Leu Glu Gly Gly Asn Leu Glu Ala Met
           35                  40                  45
   Val Thr Leu Asn Gln Val Gly Arg Ser Gln Glu Val Leu Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Thr Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 41
   <211> 152
   <212> PRT
   <213> Artificial
<220>
   <223> Mutein of human tear lipocalin with binding affinity for c-Met
<400> 41
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Ser Pro Ile Thr Leu Thr Thr Leu Glu Gly Gly Asn Leu Glu Ala Thr
   35 40 45
   Val Thr Leu Asn Gln Ile Gly Arg Ser Gln Glu Val Leu Ala Val Leu
   50 55 60
   Glu Lys Thr Asp Glu Pro Gly Lys Tyr Thr Ala Tyr Gly Gly Ala His
   65 70 75 80
   Val Ala Tyr Ile His Arg Ser His Val Lys Asp His Tyr Ile Phe Tyr
   85 90 95
   Ser Glu Gly Asp Thr Trp Gly Gly Pro Val Pro Gly Val Trp Leu Val
   100 105 110
   Gly Arg Asp Pro Lys Asn Asn Leu Glu Ala Leu Glu Asp Phe Glu Lys
   115 120 125
   Ala Ala Gly Ala Arg Gly Leu Ser Thr Glu Ser Ile Leu Ile Pro Arg
   130 135 140
   Gln Ser Glu Thr Ser Ser Pro Gly
   145 150
<210> 42
   <211> 152
   <212> PRT
   <213> Artificial
<220>
   <223> Mutein of human tear lipocalin with binding affinity for c-Met
<400> 42
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Thr Pro Ile Thr Leu Thr Thr Leu Glu Gly Gly Asn Leu Glu Ala Met
           35                  40                  45
   Val Thr Leu Asn Gln Val Gly Arg Ser Gln Glu Val Leu Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Cys Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 43
   <211> 158
   <212> PRT
   <213> Artificial
<220>
   <223> Mutein of human tear lipocalin with binding affinity for c-Met
<400> 43
   His His Leu Leu Ala Ser Asp Glu Glu Ile Gln Asp Val Ser Gly Thr
   1               5                   10                  15
   Trp Tyr Leu Lys Ala Met Thr Val Asp Arg Glu Phe Pro Glu Met Asn
               20                  25                  30
   Leu Glu Ser Val Thr Pro Met Thr Leu Thr Thr Leu Glu Gly Gly Asn
           35                  40                  45
   Leu Glu Ala Lys Val Thr Met Leu Ile Ser Gly Arg Cys Gln Glu Val
       50                  55                  60
   Lys Ala Val Leu Glu Lys Thr Asp Glu Pro Gly Lys Tyr Thr Ala Asp
   65                  70                  75                  80
   Gly Gly Lys His Val Ala Tyr Ile Ile Arg Ser His Val Lys Asp His
                   85                  90                  95
   Tyr Ile Phe Tyr Cys Glu Gly Glu Leu His Gly Lys Pro Val Arg Gly
               100                 105                 110
   Val Lys Leu Val Gly Arg Asp Pro Lys Asn Asn Leu Glu Ala Leu Glu
           115                 120                 125
   Asp Phe Glu Lys Ala Ala Gly Ala Arg Gly Leu Ser Thr Glu Ser Ile
       130                 135                 140
   Leu Ile Pro Arg Gln Ser Glu Thr Cys Ser Pro Gly Ser Asp
             145                 150                 155
<210> 44
   <211> 158
   <212> PRT
   <213> Artificial
<220>
   <223> Mutein of human tear lipocalin with binding affinity for c-Met
<400> 44
   Ala Ser Asp Glu Glu Ile Gln Asp Val Ser Gly Thr Trp Tyr Leu Lys
   1               5                   10                  15
   Ala Met Thr Val Asp Thr Gln Thr Pro Leu Ser Leu Tyr Val Ser Val
               20                  25                  30
   Thr Pro Ile Thr Leu Thr Thr Leu Glu Gly Gly Asn Leu Glu Ala Glu
           35                  40                  45
   Val Thr Leu Asn Gln Val Gly Arg Ser Gln Glu Val Arg Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Ile Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly His His His His His His
   145                 150                 155
<210> 45
   <211> 158
   <212> PRT
   <213> Artificial
<220>
   <223> Mutein of human tear lipocalin with binding affinity for c-Met
<400> 45
   Ala Ser Asp Glu Glu Ile Gln Asp Val Ser Gly Thr Trp Tyr Leu Lys
   1               5                   10                  15
   Ala Met Thr Val Asp Thr Gln Met Pro Leu Ser Leu Tyr Val Ser Val
   20 25 30
   Thr Pro Ile Thr Leu Thr Thr Leu Glu Gly Gly Ser Leu Glu Ala Thr
   35 40 45
   Val Thr Leu Asn Gln Ile Gly Arg Ser Gln Glu Val Arg Ala Val Leu
   50 55 60
   Glu Lys Thr Asp Glu Pro Gly Lys Tyr Thr Ala Tyr Gly Gly Ala His
   65 70 75 80
   Val Ala Tyr Ile Ser Arg Ser His Val Lys Asp His Tyr Ile Phe Tyr
   85 90 95
   Ser Glu Gly Asp Thr Trp Gly Gly Pro Val Pro Gly Val Trp Leu Val
   100 105 110
   Gly Arg Asp Pro Lys Asn Asn Leu Glu Ala Leu Glu Asp Phe Glu Lys
   115 120 125
   Ala Ala Gly Ala Arg Gly Leu Ser Thr Glu Ser Ile Leu Ile Pro Arg
   130 135 140
   Gln Ser Glu Thr Ser Ser Pro Gly His His His His His His
   145 150 155
<210> 46
   <211> 158
   <212> PRT
   <213> Artificial
<220>
   <223> Mutein of human tear lipocalin with binding affinity for c-Met
<400> 46
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Thr Pro Ile Thr Leu Thr Thr Leu Glu Gly Gly Asn Leu Glu Ala Met
           35                  40                  45
   Val Thr Leu Asn Gln Val Gly Arg Ser Gln Glu Val Leu Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly His His His His His His
   145                 150                 155
<210> 47
   <211> 158
   <212> PRT
   <213> Artificial
<220>
   <223> Mutein of human tear lipocalin with binding affinity for c-Met
<400> 47
   Ala Ser Asp Glu Glu Ile Gln Asp Val Ser Gly Thr Trp Tyr Leu Lys
   1 5 10 15
   Ala Met Thr Val Asp Thr Gln Asp Pro Leu Ser Leu Tyr Val Ser Val
   20 25 30
   Thr Pro Leu Thr Leu Thr Thr Leu Glu Gly Gly Asn Leu Glu Ala Met
   35 40 45
   Val Thr Leu Asn Gln Val Gly Arg Ser Gln Glu Val Leu Ala Val Leu
   50 55 60
   Glu Lys Thr Asp Glu Pro Gly Lys Tyr Thr Ala Tyr Gly Gly Ala His
   65 70 75 80
   Val Ala Tyr Ile Thr Arg Ser His Val Lys Asp His Tyr Ile Phe Tyr
   85 90 95
   Ser Glu Gly Asp Thr Trp Gly Gly Pro Val Pro Gly Val Trp Leu Val
   100 105 110
   Gly Arg Asp Pro Lys Asn Asn Leu Glu Ala Leu Glu Asp Phe Glu Lys
   115 120 125
   Ala Ala Gly Ala Arg Gly Leu Ser Thr Glu Ser Ile Leu Ile Pro Arg
   130 135 140
   Gln Ser Glu Thr Ser Ser Pro Gly His His His His His His
   145 150 155
<210> 48
   <211> 158
   <212> PRT
   <213> Artificial
<220>
   <223> Mutein of human tear lipocalin with binding affinity for c-Met
<400> 48
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Ser Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Ala Tyr Gly Gly Ala His
   65                  70                  75                  80
   Val Ala Tyr Ile His Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly His His His His His His
   145                 150                 155
<210> 49
   <211> 152
   <212> PRT
   <213> Artificial
<220>
   <223> Mutein of human tear lipocalin with binding affinity for c-Met
<400> 49
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Ser Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Leu Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 50
   <211> 152
   <212> PRT
   <213> Artificial
<220>
   <223> Mutein of human tear lipocalin with binding affinity for c-Met
<400> 50
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Ser Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Leu Tyr Gly Gly Ala His
   65                  70                  75                  80
   Val Thr Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 51
   <211> 152
   <212> PRT
   <213> Artificial
<220>
   <223> Mutein of human tear lipocalin with binding affinity for c-Met
<400> 51
   Ala Ser Asp Glu Glu Ile Gln Asp Val Ser Gly Thr Trp Tyr Leu Lys
   1               5                   10                  15
   Ala Met Thr Val Asp Thr Gln Asp Pro Leu Ser Leu Tyr Val Ser Val
               20                  25                  30
   Ser Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Ser Tyr Gly Gly Ala His
   65                  70                  75                  80
   Val Thr Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 52
   <211> 152
   <212> PRT
   <213> Artificial
<220>
   <223> Mutein of human tear lipocalin with binding affinity for c-Met
<400> 52
   Ala Ser Asp Glu Glu Ile Gln Asp Val Ser Gly Thr Trp Tyr Leu Lys
   1               5                   10                  15
   Ala Met Thr Val Asp Val Gly Asp Leu Gly Ser Arg Val Val Ser Val
               20                  25                  30
   Thr Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Leu Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 53
   <211> 152
   <212> PRT
   <213> Artificial
<220>
   <223> Mutein of human tear lipocalin with binding affinity for c-Met
<400> 53
   Ala Ser Asp Glu Glu Ile Gln Asp Val Ser Gly Thr Trp Tyr Leu Lys
   1               5                   10                  15
   Ala Met Thr Val Asp Pro Gly Asp Ile Arg Ser Leu Ile Val Ser Val
               20                  25                  30
   Thr Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Leu Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 54
   <211> 152
   <212> PRT
   <213> Artificial
<220>
   <223> Mutein of human tear lipocalin with binding affinity for c-Met
<400> 54
   Ala Ser Asp Glu Glu Ile Gln Asp Val Ser Gly Thr Trp Tyr Leu Lys
   1               5                   10                  15
   Ala Met Thr Val Asp Ser Glu Asp Ala Phe Ser Val Thr Val Ser Val
               20                  25                  30
   Thr Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Leu Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 55
   <211> 152
   <212> PRT
   <213> Artificial
<220>
   <223> Mutein of human tear lipocalin with binding affinity for c-Met
<400> 55
   Ala Ser Asp Glu Glu Ile Gln Asp Val Ser Gly Thr Trp Tyr Leu Lys
   1               5                   10                  15
   Ala Met Thr Val Asp Val Val Asp Trp Arg Ser Gln Val Arg Ser Val
               20                  25                  30
   Thr Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Ala Tyr Gly Gly Ala His
   65                  70                  75                  80
   Val Thr Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
   145                 150
<210> 56
   <211> 152
   <212> PRT
   <213> Artificial
<220>
   <223> Mutein of human tear lipocalin with binding affinity for c-Met
<400> 56
   Ala Ser Asp Glu Glu Ile Gln Asp Val Ser Gly Thr Trp Tyr Leu Lys
   1               5                   10                  15
   Ala Met Thr Val Asp Gly Ser Asp Trp Glu Ser Val Phe Ala Ser Val
               20                  25                  30
   Thr Pro Ile Thr Leu Thr Thr Leu Glu Gly Gly Asn Leu Glu Ala Thr
           35                  40                  45
   Val Thr Leu Asn Gln Ile Gly Arg Ser Gln Glu Val Leu Ala Val Leu
       50                  55                  60
   Glu Lys Thr Asp Glu Pro Gly Lys Tyr Thr Leu Tyr Gly Gly Ala His
   65                  70                  75                  80
   Val Ala Tyr Ile Gln Arg Ser His Val Lys Asp His Tyr Ile Phe Tyr
                   85                  90                  95
   Ser Glu Gly Asp Thr Trp Gly Gly Pro Val Pro Gly Val Trp Leu Val
               100                 105                 110
   Gly Arg Asp Pro Lys Asn Asn Leu Glu Ala Leu Glu Asp Phe Glu Lys
           115                 120                 125
   Ala Ala Gly Ala Arg Gly Leu Ser Thr Glu Ser Ile Leu Ile Pro Arg
       130                 135                 140
   Gln Ser Glu Thr Ser Ser Pro Gly
                       145                 150
<210> 57
   <211> 186
   <212> PRT
   <213> Artificial
<220>
   <223> Mutein of hNGAL with binding affinity for CTLA-4
<400> 57
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Met Val Phe Phe Lys Leu Val Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 58
   <211> 186
   <212> PRT
   <213> Artificial
<220>
   <223> Mutein of hNGAL with binding affinity for CTLA-4
<400> 58
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Tyr Ile Trp Arg Asn Asp Arg Tyr Pro
           35                  40                  45
   Met Gln Met Tyr Ala Thr Ile Tyr Glu Leu Lys Glu Asp Lys Ser Tyr
       50                  55                  60
   Asn Val Thr Ser Val Ile Phe Asp Thr Lys Lys Cys Glu Tyr Pro Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Arg Met Asp Thr Ser Tyr Leu Val Arg Val Val Ser
               100                 105                 110
   Thr Asn Tyr Asn Gln His Ala Met Val Phe Phe Lys Gln Val Asn His
           115                 120                 125
   Asn Thr Glu His Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 59
   <211> 186
   <212> PRT
   <213> Artificial
<220>
   <223> Mutein of hNGAL with binding affinity for CTLA-4
<400> 59
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 60
   <211> 186
   <212> PRT
   <213> Artificial
<220>
   <223> Mutein of hNGAL with binding affinity for CTLA-4
<400> 60
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Met Val Phe Phe Lys Leu Ala Glu Asp
           115                 120                 125
   Asn Ala Glu Phe Phe Ala Val Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 61
   <211> 186
   <212> PRT
   <213> Artificial
<220>
   <223> Mutein of hNGAL with binding affinity for CTLA-4
<400> 61
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                      80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Thr Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 62
   <211> 186
   <212> PRT
   <213> Artificial
<220>
   <223> Mutein of hNGAL with binding affinity for CTLA-4
<400> 62
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu His Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln His Ala Met Val Phe Phe Glu Leu Val Glu Asp
           115                 120                 125
   Asn Ala Gly Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 63
   <211> 186
   <212> PRT
   <213> Artificial
<220>
   <223> Mutein of hNGAL with binding affinity for CTLA-4
<400> 63
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Met Val Phe Phe Glu Leu Val Glu Asp
           115                 120                 125
   Asn Ala Gly Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 64
   <211> 186
   <212> PRT
   <213> Artificial
<220>
   <223> Mutein of hNGAL with binding affinity for CTLA-4
<400> 64
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Val Val Phe Phe Gln Leu Val Glu Asp
           115                 120                 125
   Asn Ala Gly Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 65
   <211> 186
   <212> PRT
   <213> Artificial
<220>
   <223> Mutein of hNGAL with binding affinity for CTLA-4
<400> 65
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Leu Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Met Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 66
   <211> 186
   <212> PRT
   <213> Artificial
<220>
   <223> Mutein of hNGAL with binding affinity for CTLA-4
<400> 66
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Phe
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Thr Val Phe Phe Lys Leu Ala Glu Asp
           115                 120                 125
   Asn Ala Glu Phe Phe Ala Val Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 67
   <211> 186
   <212> PRT
   <213> Artificial
<220>
   <223> Mutein of hNGAL with binding affinity for CTLA-4
<400> 67
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asp Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Thr Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 68
   <211> 186
   <212> PRT
   <213> Artificial
<220>
   <223> Mutein of hNGAL with binding affinity for CTLA-4
<400> 68
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Arg His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Thr Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 69
   <211> 186
   <212> PRT
   <213> Artificial
<220>
   <223> Mutein of hNGAL with binding affinity for CTLA-4
<400> 69
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asp Val Thr Ser Val Ile Phe Pro His Lys Lys Cys Glu Tyr Thr Thr
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Gly Lys Thr Ser Tyr Leu Val Arg Val Val Ser
               100                 105                 110
   Thr Asn Tyr Asn Gln His Ala Thr Val Phe Phe Lys Leu Ala Gly Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly Ser Ala Trp Ser His Pro Gln Phe
               180                 185
<210> 70
   <211> 178
   <212> PRT
   <213> Artificial
<220>
   <223> Mutein of hNGAL with binding affinity for CTLA-4
<400> 70
<210> 71
   <211> 178
   <212> PRT
   <213> Artificial
<220>
   <223> Mutein of hNGAL with binding affinity for CTLA-4
<400> 71
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Ser Asp
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 72
   <211> 178
   <212> PRT
   <213> Artificial
<220>
   <223> Mutein of hNGAL with binding affinity for CTLA-4
<400> 72
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Ser Asp
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 73
   <211> 178
   <212> PRT
   <213> Artificial
<220>
   <223> Mutein of hNGAL with binding affinity for CTLA-4
<400> 73
   Gln Asp Ser Thr Ser Asp Leu Ile Pro Ala Pro Pro Leu Ser Lys Val
   1 5 10 15
   Pro Leu Gln Gln Asn Phe Gln Asp Asn Gln Phe His Gly Lys Trp Tyr
   20 25 30
   Val Val Gly Leu Ala Gly Asn Arg Ile Leu Arg Asp Asp Gln His Pro
   35 40 45
   Met Asn Met Tyr Ala Thr Ile Tyr Glu Leu Lys Glu Asp Lys Ser Tyr
   50 55 60
   Asn Val Thr Ser Val Ile Leu Ser His Lys Lys Cys Glu Tyr Thr Ile
   65 70 75 80
   Ala Thr Phe Val Pro Gly Ser Arg Pro Gly Glu Phe Thr Leu Gly Asn
   85 90 95
   Ile Lys Ser Tyr Gly Asp Lys Thr Ser Tyr Leu Val Arg Val Val Ser
   100 105 110
   Thr Asn Tyr Asn Gln Tyr Ala Val Val Phe Phe Lys Leu Ala Glu Asp
   115 120 125
   Asn Ala Glu Phe Phe Ala Ile Thr Ile Tyr Gly Arg Thr Lys Glu Leu
   130 135 140
   Ala Ser Glu Leu Lys Glu Asn Phe Ile Arg Phe Ser Lys Ser Leu Gly
   145 150 155 160
   Leu Pro Glu Asn His Ile Val Phe Pro Val Pro Ile Asp Gln Cys Ile
   165 170 175
   Asp Gly
<210> 74
   <211> 178
   <212> PRT
   <213> Artificial
<220>
   <223> Mutein of hNGAL with binding affinity for CTLA-4
<400> 74
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asp Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Ser Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                     165                 170                 175
   Asp Gly
<210> 75
   <211> 178
   <212> PRT
   <213> Artificial
<220>
   <223> Mutein of hNGAL with binding affinity for CTLA-4
<400> 75
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Leu Ser His Lys Lys Cys Glu Tyr Thr Val
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 76
   <211> 178
   <212> PRT
   <213> Artificial
<220>
   <223> Mutein of hNGAL with binding affinity for CTLA-4
<400> 76
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Val Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 77
   <211> 178
   <212> PRT
   <213> Artificial
<220>
   <223> Mutein of hNGAL with binding affinity for CTLA-4
<400> 77
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asp Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 78
   <211> 178
   <212> PRT
   <213> Artificial
<220>
   <223> Mutein of hNGAL with binding affinity for CTLA-4
<400> 78
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Met Val Phe Phe Lys Leu Val Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 79
   <211> 180
   <212> PRT
   <213> Artificial
<220>
   <223> Mutein of hNGAL with binding affinity for CTLA-4
<400> 79
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Tyr Ile Trp Arg Asn Asp Arg Tyr Pro
           35                  40                  45
   Met Glu Asn Asp Met Tyr Ala Thr Ile Tyr Glu Leu Lys Glu Asp Lys
       50                  55                  60
   Ser Tyr Asn Val Thr Ser Val Ile Phe Asp Thr Lys Lys Cys Glu Tyr
   65                  70                  75                  80
   Pro Ile Ala Thr Phe Val Pro Gly Ser Gln Pro Gly Glu Phe Thr Leu
                   85                  90                  95
   Gly Asn Ile Lys Ser Tyr Arg Met Asp Thr Ser Tyr Leu Val Arg Val
               100                 105                 110
   Val Ser Thr Asn Tyr Asn Gln His Ala Met Val Phe Phe Lys Gln Val
           115                 120                 125
   Asn His Asn Thr Glu His Phe Ala Ile Thr Ile Tyr Gly Arg Thr Lys
       130                 135                 140
   Glu Leu Ala Ser Glu Leu Lys Glu Asn Phe Ile Arg Phe Ser Lys Ser
   145                 150                 155                 160
   Leu Gly Leu Pro Glu Asn His Ile Val Phe Pro Val Pro Ile Asp Gln
                   165                 170                 175
   Cys Ile Asp Gly
               180
<210> 80
   <211> 178
   <212> PRT
   <213> Artificial
<220>
   <223> Mutein of hNGAL with binding affinity for CTLA-4
<400> 80
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Met Val Phe Phe Lys Leu Ala Glu Asp
           115                 120                 125
   Asn Ala Glu Phe Phe Ala Val Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 81
   <211> 178
   <212> PRT
   <213> Artificial
<220>
   <223> Mutein of hNGAL with binding affinity for CTLA-4
<400> 81
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 82
   <211> 178
   <212> PRT
   <213> Artificial
<220>
   <223> Mutein of hNGAL with binding affinity for CTLA-4
<400> 82
   Gln Asp Ser Thr Ser Asp Leu Ile Pro Ala Pro Pro Leu Ser Lys Val
   1 5 10 15
   Pro Leu Gln Gln Asn Phe Gln Asp Asn Gln Phe His Gly Lys Trp Tyr
   20 25 30
   Val Val Gly Leu Ala Gly Asn Arg Ile Leu Arg Asp Asp Gln His Pro
   35 40 45
   Met Asn Met Tyr Ala Thr Ile Tyr Glu Leu Lys Glu Asp Lys Ser Tyr
   50 55 60
   Asn Val Thr Ser Val Ile Phe Pro His Lys Lys Cys Glu Tyr Thr Ile
   65 70 75 80
   Ala Thr Phe Val Pro Gly Ser Gln Pro Gly Glu Phe Thr Leu Gly Asn
   85 90 95
   Ile Lys Ser Tyr Gly Asp Lys Thr Ser Tyr Leu Val Arg Val Val Ser
   100 105 110
   Thr Asn Tyr Asn Gln His Ala Thr Val Phe Phe Lys Leu Ala Glu Asp
   115 120 125
   Asn Ala Glu Phe Phe Ala Ile Thr Ile Tyr Gly Arg Thr Lys Glu Leu
   130 135 140
   Ala Ser Glu Leu Lys Glu Asn Phe Ile Arg Phe Ser Lys Ser Leu Gly
   145 150 155 160
   Leu Pro Glu Asn His Ile Val Phe Pro Val Pro Ile Asp Gln Cys Ile
   165 170 175
   Asp Gly
<210> 83
   <211> 178
   <212> PRT
   <213> Artificial
<220>
   <223> Mutein of hNGAL with binding affinity for CTLA-4
<400> 83
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu His Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln His Ala Met Val Phe Phe Glu Leu Val Glu Asp
           115                 120                 125
   Asn Ala Gly Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 84
   <211> 177
   <212> PRT
   <213> Artificial
<220>
   <223> Mutein of hNGAL with binding affinity for CTLA-4
<400> 84
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Met Val Phe Phe Glu Leu Val Glu Asp
           115                 120                 125
   Asn Ala Gly Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp
<210> 85
   <211> 178
   <212> PRT
   <213> Artificial
<220>
   <223> Mutein of hNGAL with binding affinity for CTLA-4
<400> 85
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Val Val Phe Phe Gln Leu Val Glu Asp
           115                 120                 125
   Asn Ala Gly Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 86
   <211> 178
   <212> PRT
   <213> Artificial
<220>
   <223> Mutein of hNGAL with binding affinity for CTLA-4
<400> 86
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Leu Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Met Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 87
   <211> 178
   <212> PRT
   <213> Artificial
<220>
   <223> Mutein of hNGAL with binding affinity for CTLA-4
<400> 87
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
   20 25 30
   Val Val Gly Leu Ala Gly Asn Arg Ile Leu Arg Asp Asp Gln His Pro
   35 40 45
   Met Asn Met Tyr Ala Thr Ile Tyr Glu Leu Lys Glu Asp Lys Ser Tyr
   50 55 60
   Asn Val Thr Ser Val Ile Phe Pro His Lys Lys Cys Glu Tyr Thr Phe
   65 70 75 80
   Ala Thr Phe Val Pro Gly Ser Gln Pro Gly Glu Phe Thr Leu Gly Asn
   85 90 95
   Ile Lys Ser Tyr Gly Asp Lys Thr Ser Tyr Leu Val Arg Val Val Ser
   100 105 110
   Thr Asn Tyr Asn Gln His Ala Thr Val Phe Phe Lys Leu Ala Glu Asp
   115 120 125
   Asn Ala Glu Phe Phe Ala Val Thr Ile Tyr Gly Arg Thr Lys Glu Leu
   130 135 140
   Ala Ser Glu Leu Lys Glu Asn Phe Ile Arg Phe Ser Lys Ser Leu Gly
   145 150 155 160
   Leu Pro Glu Asn His Ile Val Phe Pro Val Pro Ile Asp Gln Cys Ile
   165 170 175
   Asp Gly
<210> 88
   <211> 178
   <212> PRT
   <213> Artificial
<220>
   <223> Mutein of hNGAL with binding affinity for CTLA-4
<400> 88
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asp Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Thr Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 89
   <211> 178
   <212> PRT
   <213> Artificial
<220>
   <223> Mutein of hNGAL with binding affinity for CTLA-4
<400> 89
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Arg His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln His Ala Thr Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 90
   <211> 178
   <212> PRT
   <213> Artificial
<220>
   <223> Mutein of hNGAL with binding affinity for CTLA-4
<400> 90
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asp Val Thr Ser Val Ile Phe Pro His Lys Lys Cys Glu Tyr Thr Thr
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Gly Lys Thr Ser Tyr Leu Val Arg Val Val Ser
               100                 105                 110
   Thr Asn Tyr Asn Gln His Ala Thr Val Phe Phe Lys Leu Ala Gly Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 91
   <211> 178
   <212> PRT
   <213> Artificial
<220>
   <223> Mutein of hNGAL with binding affinity for CTLA-4
<400> 91
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Leu Ser His Lys Lys Cys Glu Tyr Thr Val
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 92
   <211> 178
   <212> PRT
   <213> Artificial
<220>
   <223> Mutein of hNGAL with binding affinity for CTLA-4
<400> 92
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asp Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Ser Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 93
   <211> 178
   <212> PRT
   <213> Artificial
<220>
   <223> Mutein of hNGAL with binding affinity for CTLA-4
<400> 93
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Phe Pro His Lys Lys Cys Glu Tyr Thr Ile
   65                  70                  75                  80
   Ala Thr Phe Val Pro Gly Ser Gln Pro Gly Glu Phe Thr Leu Ser Asp
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 94
   <211> 178
   <212> PRT
   <213> Artificial
<220>
   <223> Mutein of hNGAL with binding affinity for CTLA-4
<400> 94
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Val Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 95
   <211> 178
   <212> PRT
   <213> Artificial
<220>
   <223> Mutein of hNGAL with binding affinity for CTLA-4
<400> 95
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Leu Ser His Lys Lys Cys Glu Tyr Thr Ile
   65                  70                  75                  80
   Ala Thr Phe Val Pro Gly Ser Arg Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 96
   <211> 178
   <212> PRT
   <213> Artificial
<220>
   <223> Mutein of hNGAL with binding affinity for CTLA-4
<400> 96
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asn Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 97
   <211> 178
   <212> PRT
   <213> Artificial
<220>
   <223> Mutein of hNGAL with binding affinity for CTLA-4
<400> 97
   Gln Asp Ser Thr Ser Asp Leu Ile Pro Ala Pro Pro Leu Ser Lys Val
   1 5 10 15
   Pro Leu Gln Gln Asn Phe Gln Asp Asn Gln Phe His Gly Lys Trp Tyr
   20 25 30
   Val Val Gly Leu Ala Gly Asn Arg Ile Leu Arg Asp Asp Gln His Pro
   35 40 45
   Met Asn Met Tyr Ala Thr Ile Tyr Glu Leu Lys Glu Asp Lys Ser Tyr
   50 55 60
   Asn Val Thr Ser Val Ile Phe Pro His Lys Lys Cys Glu Tyr Thr Ile
   65 70 75 80
   Ala Thr Phe Val Pro Gly Ser Gln Pro Gly Glu Phe Thr Leu Gly Asn
   85 90 95
   Ile Lys Ser Tyr Gly Asp Lys Thr Ser Tyr Leu Val Arg Val Val Ser
   100 105 110
   Thr Asp Tyr Asn Gln Tyr Ala Val Val Phe Phe Lys Leu Ala Glu Asp
   115 120 125
   Asp Ala Glu Phe Phe Ala Ile Thr Ile Tyr Gly Arg Thr Lys Glu Leu
   130 135 140
   Ala Ser Glu Leu Lys Glu Asn Phe Ile Arg Phe Ser Lys Ser Leu Gly
   145 150 155 160
   Leu Pro Glu Asn His Ile Val Phe Pro Val Pro Ile Asp Gln Cys Ile
   165 170 175
   Asp Gly
<210> 98
   <211> 178
   <212> PRT
   <213> Artificial
<220>
   <223> Mutein of hNGAL with binding affinity for CTLA-4
<400> 98
<210> 99
   <211> 178
   <212> PRT
   <213> Artificial
<220>
   <223> Mutein of hNGAL with binding affinity for CTLA-4
<400> 99
   Gln Asp Ser Thr Ser Asp Leu Ile Pro Ala Pro Pro Leu Ser Lys Val
   1 5 10 15
   Pro Leu Gln Gln Asn Phe Gln Asp Asn Gln Phe His Gly Lys Trp Tyr
   20 25 30
   Val Val Gly Leu Ala Gly Asn Arg Ile Leu Arg Asp Asp Gln His Pro
   35 40 45
   Met Asn Met Tyr Ala Thr Ile Tyr Glu Leu Lys Glu Asp Lys Ser Tyr
   50 55 60
   Asn Val Thr Ser Val Ile Ser Ser His Lys Lys Cys Glu Tyr Thr Ile
   65 70 75 80
   Ala Thr Phe Val Pro Gly Ser Gln Pro Gly Glu Phe Thr Leu Gly Asn
   85 90 95
   Ile Lys Ser Tyr Gly Asp Lys Thr Ser Tyr Leu Val Arg Val Val Ser
   100 105 110
   Thr Asp Tyr Asn Gln Tyr Ala Val Val Phe Phe Lys Leu Ala Glu Asp
   115 120 125
   Asn Ala Glu Phe Phe Ala Ile Thr Ile Tyr Gly Arg Thr Lys Glu Leu
   130 135 140
   Ala Cys Glu Leu Lys Glu Asn Phe Ile Arg Phe Ser Lys Ser Leu Gly
   145 150 155 160
   Leu Pro Glu Asn His Ile Val Phe Pro Val Pro Ile Asp Gln Cys Ile
   165 170 175
   Asp Gly
<210> 100
   <211> 178
   <212> PRT
   <213> Artificial
<220>
   <223> Mutein of hNGAL with binding affinity for CTLA-4
<400> 100
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Cys Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 101
   <211> 178
   <212> PRT
   <213> Artificial
<220>
   <223> Mutein of hNGAL with binding affinity for CTLA-4
<400> 101
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Cys Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 102
   <211> 178
   <212> PRT
   <213> Artificial
<220>
   <223> Mutein of hNGAL with binding affinity for CTLA-4
<400> 102
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Cys Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 103
   <211> 178
   <212> PRT
   <213> Artificial
<220>
   <223> Mutein of hNGAL with binding affinity for CTLA-4
<400> 103
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Cys Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 104
   <211> 178
   <212> PRT
   <213> Artificial
<220>
   <223> Mutein of hNGAL with binding affinity for CTLA-4
<400> 104
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Cys Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 105
   <211> 178
   <212> PRT
   <213> Artificial
<220>
   <223> Mutein of hNGAL with binding affinity for CTLA-4
<400> 105
   Gln Asp Ser Thr Ser Asp Leu Ile Pro Ala Pro Pro Leu Cys Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 106
   <211> 178
   <212> PRT
   <213> Artificial
<220>
   <223> Mutein of hNGAL with binding affinity for CTLA-4
<400> 106
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Cys Leu Gly
   145                 150                 155                 160
   Leu Pro Glu Asn His Ile Val Phe Pro Val Pro Ile Asp Gln Cys Ile
                   165                 170                 175
   Asp Gly
<210> 107
   <211> 178
   <212> PRT
   <213> Artificial
<220>
   <223> Mutein of hNGAL with binding affinity for CTLA-4
<400> 107
   Gln Asp Ser Thr Ser Asp Leu Ile Pro Ala Pro Pro Leu Ser Lys Val
   1 5 10 15
   Pro Leu Gln Gln Asn Phe Gln Asp Asn Gln Phe His Gly Lys Trp Tyr
   20 25 30
   Val Val Gly Leu Ala Gly Asn Arg Ile Leu Arg Asp Asp Gln His Pro
   35 40 45
   Met Asn Met Tyr Ala Thr Ile Tyr Glu Leu Lys Glu Asp Lys Ser Tyr
   50 55 60
   Asn Val Thr Ser Val Ile Ser Ser His Lys Lys Cys Glu Tyr Thr Ile
   65 70 75 80
   Ala Thr Phe Val Pro Gly Ser Cys Pro Gly Glu Phe Thr Leu Gly Asn
   85 90 95
   Ile Lys Ser Tyr Gly Asp Lys Thr Ser Tyr Leu Val Arg Val Val Ser
   100 105 110
   Thr Asp Tyr Asn Gln Tyr Ala Val Val Phe Phe Lys Leu Ala Glu Asp
   115 120 125
   Asn Ala Glu Phe Phe Ala Ile Thr Ile Tyr Gly Arg Thr Lys Glu Leu
   130 135 140
   Ala Ser Glu Leu Lys Glu Asn Phe Ile Arg Phe Ser Lys Ser Leu Gly
   145 150 155 160
   Leu Pro Glu Asn His Ile Val Phe Pro Val Pro Ile Asp Gln Cys Ile
   165 170 175
   Asp Gly
<210> 108
   <211> 178
   <212> PRT
   <213> Artificial
<220>
   <223> Mutein of hNGAL with binding affinity for CTLA-4
<400> 108
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Cys Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 109
   <211> 178
   <212> PRT
   <213> Artificial
<220>
   <223> Mutein of hNGAL with binding affinity for CTLA-4
<400> 109
   Gln Asp Ser Thr Ser Asp Leu Ile Pro Ala Pro Pro Leu Ser Lys Val
   1               5                   10                  15
   Pro Leu Gln Gln Cys Phe Gln Asp Asn Gln Phe His Gly Lys Trp Tyr
               20                  25                  30
   Val Val Gly Leu Ala Gly Asn Arg Ile Leu Arg Asp Asp Gln His Pro
           35                  40                  45
   Met Asn Met Tyr Ala Thr Ile Tyr Glu Leu Lys Glu Asp Lys Ser Tyr
       50                  55                  60
   Asn Val Thr Ser Val Ile Ser Ser His Lys Lys Cys Glu Tyr Thr Ile
   65                  70                  75                  80
   Ala Thr Phe Val Pro Gly Ser Gln Pro Gly Glu Phe Thr Leu Gly Asn
                   85                  90                  95
   Ile Lys Ser Tyr Gly Asp Lys Thr Ser Tyr Leu Val Arg Val Val Ser
               100                 105                 110
   Thr Asp Tyr Asn Gln Tyr Ala Val Val Phe Phe Lys Leu Ala Glu Asp
           115                 120                 125
   Asn Ala Glu Phe Phe Ala Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
<210> 110
   <211> 178
   <212> PRT
   <213> Artificial
<220>
   <223> Mutein of hNGAL with binding affinity for CTLA-4
<400> 110
   Gln Asp Ser Thr Ser Asp Leu Ile Pro Ala Pro Pro Leu Ser Lys Val
   1               5                   10                  15
   Pro Leu Gln Gln Asn Phe Gln Asp Asn Gln Phe His Gly Lys Trp Tyr
               20                  25                  30
   Val Val Gly Leu Ala Gly Asn Ala Ile Leu Arg Glu Asp Lys Asp Pro
           35                  40                  45
   Gln Lys Met Tyr Ala Thr Ile Tyr Glu Leu Lys Glu Asp Lys Ser Tyr
       50                  55                  60
   Asn Val Thr Ser Val Leu Phe Arg Lys Lys Lys Cys Asp Tyr Trp Ile
   65                  70                  75                  80
   Arg Thr Phe Val Pro Gly Ser Gln Pro Gly Glu Phe Thr Leu Gly Asn
                   85                  90                  95
   Ile Lys Ser Tyr Pro Gly Leu Thr Ser Tyr Leu Val Arg Val Val Ser
               100                 105                 110
   Thr Asn Tyr Asn Gln His Ala Met Val Phe Phe Lys Lys Val Ser Gln
           115                 120                 125
   Asn Arg Glu Tyr Phe Lys Ile Thr Ile Tyr Gly Arg Thr Lys Glu Leu
       130                 135                 140
   Ala Ser Glu Leu Lys Glu Asn Phe Ile Arg Phe Ser Lys Ser Leu Gly
   145                 150                 155                 160
   Leu Pro Glu Asn His Ile Val Phe Pro Val Pro Ile Asp Gln Cys Ile
                   165                 170                 175
   Asp Gly
   1

## Description

### FIELD OF THE INVENTION

The present invention relates to pharmaceutical compositions for the controlled release of lipocalin muteins or conjugates thereof in combination with a biodegradable polymer. The invention is directed to a mutein of human tear lipocalin (hTLc) having detectable binding affinity to the human Met receptor tyrosine kinase (c-Met) or a fragment thereof, wherein said mutein has an amino acid sequence as set forth in SEQ ID NO: 49 or wherein said mutein of human tear lipocalin (hTLc) has 70 to 98 % sequence identity with a lipocalin mutein of SEQ ID NO: 49, and wherein said mutein is conjugated to a maleimide-derivatized polyethylenglycol (PEG), which is reacted with free thiol groups of cysteine residues in the mutein, wherein these cysteine side chains may naturally occur in the protein or may be artificially introduced by mutagenesis. Further, the invention is directed to a respective mutein for use in binding human met receptor tyrosine kinase (c-Met) or a fragment thereof.

### BACKGROUND OF THE INVENTION

The members of the lipocalin protein family (Pervaiz, S., and Brew, K. (1987) FASEB J. 1, 209-214) are typically small, secreted proteins, which are characterized by a range of different molecular-recognition properties: their ability to bind various, principally hydrophobic molecules (such as retinoids, fatty acids, cholesterols, prostaglandins, biliverdins, pheromones, tastants, and odorants), their binding to specific cell-surface receptors and their formation of macromolecular complexes. Although they have, in the past, been classified primarily as transport proteins, it is now clear that the lipocalins fulfill a variety of physio logical functions. These include roles in retinol transport, olfaction, pheromone signaling, and the synthesis of prostaglandins. The lipocalins have also been implicated in the regulation of the immune response and the mediation of cell homoeostasis (reviewed, for example, in Flower, D.R. (1996) Biochem. J. 318, 1-14 and Flower, D.R. et al. (2000) Biochim. Biophys. Acta 1482, 9-24).
The lipocalins share unusually low levels of overall sequence conservation, often with sequence identities of less than 20%. In strong contrast, their overall folding pattern is highly conserved. The central part of the lipocalin structure consists of a single eight-stranded anti-parallel β-sheet closed back on itself to form a continuously hydrogen-bonded β-barrel. One end of the barrel is sterically blocked by the N-terminal peptide segment that runs across its bottom as well as three peptide loops connecting the β-strands. The other end of the β-barrel is open to the solvent and encompasses a target-binding site, which is formed by four peptide loops. It is this diversity of the loops in the otherwise rigid lipocalin scaffold that gives rise to a variety of different binding modes each capable of accommodating targets of different size, shape, and chemical character (reviewed, e.g., in Flower, D.R. (1996), *supra;* Flower, D.R. et al. (2000), *supra,* or Skerra, A. (2000) Biochim. Biophys. Acta 1482, 337-350).

Members of the lipocalin family have become subject of research concerning proteins having defined ligand-binding properties. The PCT publication WO 99/16873 discloses polypeptides of the lipocalin family with mutated amino acid positions in the region of the four peptide loops, which are arranged at the end of the cylindrical β-barrel structure encompassing the binding pocket, and which correspond to those segments in the linear polypeptide sequence comprising the amino acid positions 28 to 45, 58 to 69, 86 to 99, and 114 to 129 of the bilin-binding protein of *Pieris brassicae.*

The PCT publication WO 00/75308 discloses muteins of the bilin-binding protein, which specifically bind digoxigenin, whereas the International Patent Applications WO 03/029463 and WO 03/029471 relate to muteins of the human neutrophil gelatinase-associated lipocalin (hNGAL) and apolipoprotein D, respectively. In order to further improve and fine tune ligand affinity, specificity as well as folding stability of a lipocalin variant various approaches using different members of the lipocalin family have been proposed (Skerra, A. (2001) Rev. Mol. Biotechnol. 74, 257-275; Schlehuber, S., and Skerra, A. (2002) Biophys. Chem. 96, 213-228), such as the replacement of additional amino acid residues. The PCT publication WO 2006/56464 discloses muteins of human neutrophil gelatinase-associated lipocalin with binding affinity for CTLA-4 in the low nanomolar range.

The PCT publication WO 2005/19256 discloses muteins of tear lipocalin with at least one binding site for different or the same target ligand and provides a method for the generation of such muteins of human tear lipocalin. According to this PCT application, certain amino acid stretches within the primary sequence of tear lipocalin, in particular the loop regions comprising amino acids 7-14, 24-36, 41-49, 53-66, 69-77, 79-84, 87-98, and 103-110 of mature human tear lipocalin, are subjected to mutagenesis in order to generate muteins with high binding affinities for a given ligand. The resulting muteins have binding affinities for the selected ligand (K_{D}) in the nanomolar range, in most cases < 100 nM.

The PCT publication WO 2008/015239 discloses muteins of human tear lipocalin with a least one binding site for a non-natural ligand, including IL-4 receptor, VEGF and VEGF receptor, and provides a method for the production of such lipocalin muteins. This application discloses that certain amino acid sequence stretches in the primary sequence of human tear lipocalin can be subjected to mutagenesis to generate muteins with high binding affinities for a given target. The reported binding affinities are in the nanomolar range. The mutated positions are in the loop regions and comprise at least one mutation at any of the amino acid sequence positions 26-34, 56-58, 80, 83, 104-106 and 108 of the linear polypeptide sequence of native mature human tear lipocalin and at least one mutation at any of the amino acid sequence positions 61 and 153 of the linear polypeptide sequence of native mature human tear lipocalin. Alternatively, the disclosed muteins have at least one mutation at any of the amino acid sequence positions 34, 80, and 104 of the linear polypeptide sequence of native mature human tear lipocalin.

The PCT publication WO 2007/107563 discloses a mutein of human tear lipocalin, wherein the mutein comprises at least one mutated amino acid residue at any two or more of the sequence positions 24-36, 53-66, 79-84 and 102-110 (or 103-110) of the linear polypeptide sequence of the mature human tear lipocalin, and wherein the mutein binds to the extracellular region of the T-cell coreceptor CD4 with detectable affinity and discloses a method of generating such a mutein and pharmaceutical uses of such a mutein.

The PCT publication 2009/095447 discloses a mutein of human tear lipocalin (hTLc) having detectable binding affinity to a given non-natural ligand of human tear lipocalin, wherein said non-natural ligand is the human Met receptor tyrosin kinase (c-Met) or a domain or fragment thereof and wherein said mutein comprises amino acid replacements at at least one of the sequence position corresponding to sequence positions 26-34, 56-58, 80, 83, 104-106 and 108 of hTLC. Further, it discloses a corresponding nucleic acid molecule encoding such a mutein and a method for its generation, a method for producing such a mutein and a pharmaceutical composition comprising such a lipocalin mutein as well as to various uses of the respective mutein.

It would be desirable to have compositions and formulations that allow the controlled delivery of these lipocalin muteins, increase the *in vivo* half-life, reduce the immunogenicity of the mutein and/or increase the bioavailability of the mutein to further improve the suitability of muteins of lipocalins in therapeutic applications.

Accordingly, it is an object of the invention to provide lipocalin mutein conjugates and formulations that meet this need.

### SUMMARY OF THE INVENTION

This object is accomplished by themutein and the pharmaceutical composition having the features of the independent claims.

In a first aspect, the present invention provides a mutein of human tear lipocalin (hTLc) having detectable binding affinity to the human Met receptor tyrosine kinase (c-Met) or a fragment thereof, wherein said mutein has an amino acid sequence as set forth in SEQ ID NO: 49 or wherein said mutein of human tear lipocalin (hTLc) has 70 to 98 % sequence identity with a lipocalin mutein of SEQ ID NO: 49, and wherein said mutein is conjugated to a maleimide-derivatized polyethylenglycol (PEG), which is reacted with free thiol groups of cysteine residues in the mutein, wherein these cysteine side chains may naturally occur in the protein or may be artificially introduced by mutagenesis. Further, the present invention provides a pharmaceutical composition comprising said mutein and a pharmaceutically acceptable excipient. The present invention also provides said mutein for use in binding human Met receptor tyrosine kinase (c-Met) or a fragment thereof.

Further is described a conjugate comprising a lipocalin mutein and a moiety selected from the group consisting of a protein, protein domain, peptide, fatty acid, lipid, polysaccharide and/or organic polymer.

As is further described, the conjugate comprises, consists essentially of or consists of the lipocalin mutein and the protein, protein domain, peptide, fatty acid, lipid, polysaccharide and/or organic polymer moiety. The moiety may also comprise combinations of the afore-mentioned compounds, for example glycopeptides, lipid-modified peptides and proteins, glycolipids, polysaccharides modified with organic groups, such as alkylated polysaccharides, etc.

As it is further described: The lipocalin mutein and a moiety selected from the group consisting of a protein, protein domain, peptide, fatty acid, lipid, polysaccharide and/or organic polymer and combinations thereof are covalently linked to each other. This linkage may be via the N- or C-terminus of the lipocalin mutein or an amino acid side chain. In one particular embodiment, the linkage is made via a mutated amino acid residue, such as for example a cysteine or lysine residue.

### DETAILED DESCRIPTION OF THE INVENTION

It is described herein: In conjugates, a moiety may facilitate controlled delivery of the lipocalin mutein, extend the *in vivo* half-life of the lipocalin mutein, increase the bioavailability of the lipocalin mutein and/or decrease the immunogenicity of the lipocalin mutein.

The moiety that extends the serum half-life may be a hydrophilic polymer, a fatty acid molecule, such as palmitic acid (Vajo & Duckworth 2000, Pharmacol. Rev. 52, 1-9), an Fc part of an immunoglobulin, a CH3 domain of an immunoglobulin, a CH4 domain of an immunoglobulin, albumin or a fragment thereof, an albumin binding peptide, or an albumin binding protein, ubiquitin, an ubiquitin-derived peptide, transferrin to name only a few.

The albumin binding protein may be a bacterial albumin binding protein, an antibody, an antibody fragment including domain antibodies (see US patent 6,696,245, for example), or a lipocalin mutein with binding activity for albumin. Accordingly, suitable conjugation partners for extending the half-life of a lipocalin mutein include albumin (Osborn, B.L. et al. (2002) Pharmacokinetic and pharmacodynamic studies of a human serum albumin-interferon-alpha fusion protein in cynomolgus monkeys J. Pharmacol. Exp. Ther. 303, 540-548), or an albumin binding protein, for example, a bacterial albumin binding domain, such as the one of streptococcal protein G (Konig, T. and Skerra, A. (1998) Use of an albumin-binding domain for the selective immobilisation of recombinant capture antibody fragments on ELISA plates. J. Immunol. Methods 218, 73-83). Other examples of albumin binding peptides that can be used as conjugation partner are, for instance, those having a Cys-Xaa₁-Xaa₂-Xaa₃-Xaa₄-Cys consensus sequence, wherein Xaa₁ is Asp, Asn, Ser, Thr, or Trp; Xaa₂ is Asn, Gln, His, Ile, Leu, or Lys; Xaa₃ is Ala, Asp, Phe, Trp, or Tyr; and Xaa₄ is Asp, Gly, Leu, Phe, Ser, or Thr as described in US patent application 2003/0069395 or Dennis et al. (Dennis, M. S., Zhang, M., Meng, Y. G., Kadkhodayan, M., Kirchhofer, D., Combs, D. & Damico, L. A. (2002). "Albumin binding as a general strategy for improving the pharmacokinetics of proteins." J Biol Chem 277, 35035-35043).

It is further described that albumin itself or a biological active fragment of albumin can be used as conjugation partner of a lipocalin mutein. The term "albumin" comprises all mammal albumins such as human serum albumin or bovine serum albumin or rat albumin. The albumin or fragment thereof can be recombinantly produced as described in US patent 5,728,553 or European patent applications EP 0 330 451 and EP 0 361 991. Recombinant human albumin (Recombumin®) Novozymes Delta Ltd. (Nottingham, UK) can be conjugated or fused to a lipocalin mutein in order to extend the half-life of the mutein.

If the albumin-binding protein is an antibody fragment it may be a domain antibody. Domain Antibodies (dAbs) are engineered to allow precise control over biophysical properties and *in vivo* half-life to create the optimal safety and efficacy product profile. Domain Antibodies are for example commercially available from Domantis Ltd. (Cambridge, UK and MA, USA).

Using transferrin as a moiety to extend the serum half-life of muteins, the muteins can be genetically fused to the N or C terminus, or both, of non-glycosylated transferrin. Non-glycosylated transferrin has a half-life of 14-17 days, and a transferrin fusion protein will similarly have an extended half-life. The transferrin carrier also provides high bioavailability, biodistribution and circulating stability. This technology is commercially available from BioRexis (BioRexis Pharmaceutical Corporation, PA, USA). Recombinant human transferrin (DeltaFerrin™) for use as a protein stabilizer/half-life extension partner is also commercially available from Novozymes Delta Ltd. (Nottingham, UK).
If an Fc part of an immunoglobulin is used for the purpose to prolong the serum half-life of muteins, the *SynFusion*™technology, commercially available from Syntonix Pharmaceuticals, Inc (MA, USA), may be used. The use of this Fc-fusion technology allows the creation of longer-acting biopharmaceuticals and may for example consist of two copies of the mutein linked to the Fc region of an antibody to improve pharmacokinetics, solubility, and production efficiency.

Yet another alternative to prolong the half-life of a mutein is to fuse to the N-or C-terminus of a mutein long, unstructured, flexible glycine-rich sequences (for example poly-glycine with about 20 to 80 consecutive glycine residues). This approach disclosed in WO2007/038619, for example, has also been termed "rPEG" (recombinant PEG).

The term "hydrophilic polymer", as used herein, refers to any water-soluble linear, branched, forked, branched-forked, dendrimeric, multi-armed, or star-shaped polymer including, but not limited to, polyethylene glycol and polyethylene glycol/polypropylene glycol copolymers, polyoxyethylated glycerol, and similar polymers. Preferably, the molecular weight of the polymer ranges from about 300 daltons to about 70,000 daltons, more preferably from about 500 daltons to about 50,000 daltons, still more preferably from about 5,000 daltons to about 30,000 daltons.

Hydrophilic polymers for use typically have at least one reactive group incorporated for attachment to the bioactive molecule of interest through amino, carboxyl, sulfhydryl, phosphate or hydroxyl functions. Hydrophilic polymers, such as polyethylene glycol, can be prepared according to standard protocols with one end capped as with a methoxy group and the other end activated for facile conjugation to active groups on bioactive molecules. For example, US Patent 6,113,906 describes the use of succinimidyl succinate or succinimidyl carbonate reactive groups on a "U-shaped" (i.e., branched) form of polyethylene glycol for reaction with the amino groups of proteins. US Patent 5,650,234 describes the use of N-hydroxybenzotriazole carbonate, 2-hydroxypyrimidine carbonate, and N-hydroxy-2-pyrrolidinone carbonate derivatives of polyethylene glycol for reaction with the amino groups of proteins to form a stable urethane bond. US Patent 5,672,662 describes the use of succinimidyl esters of propionic and butanoic acid substituted polyethylene glycols for reaction with the amino groups of proteins to form a stable amide bond. US Patent 5,446,090 describes the use of vinyl-sulfone derivatives of polyethylene glycol to form stable thioether bonds with the sulfhydryl groups of proteins. US Patent 5,880,255 describes the use of tresyl (2,2,2-trifluoroethane-sulfonyl) derivatives of polyethylene glycol for reaction with the amino groups of proteins to form a simple, stable secondary amine linkage. US Patent 5,252,714 describes the use of propionaldehyde derivatives of polyethylene glycol for reaction with the amino groups of proteins resulting in a stable secondary amine bond. The bonds resulting from the attachment of such hydrophilic polymers to bioactive molecules can be intentionally designed to be stable or unstable (i e., reversible). In addition, hydrophilic polymers can be prepared according to standard protocols with two similar (e. g., homobifunctional) or dissimilar (e. g., heterobifunctional) functional groups available to facilitate conjugation to active groups on bioactive molecules. For example, WO 01/26692 A1 describes the use of heterobifunctional polyethylene glycol derivatives for protein modification.

Exemplary hydrophilic polymers for the described conjugates are, but are not limited to, polyalkylene glycols, polyoxyethylated polyols, hydroxyethyl starch, polyhydroxy acids, polylactic acids, polyglycolic acids, and copolymers thereof as well as linear, branched and activated derivatives thereof.

The polyalkylene glycols can be substituted, unsubstituted, linear or branched. They can also be activated polyalkylene derivatives. The polyalkylene glycols include, but are not limited to, polyethylene glycol, polypropylene, and polyethylene glycol/polypropylene glycol copolymers.

In the present invention, the hydrophilic polymer is a maleimide-derivatized polyethylene glycol (PEG).

PEG refers to a linear or branched neutral polyether with the chemical formula HO-(CH₂CH₂O)ₙ-H. PEG is highly soluble in water and many organic solvents (e.g., methylene chloride, ethanol, toluene, acetone, and chloroform), and is readily available in various sizes (molecular weights) and functionalized architectures (e.g., amino-, carboxyl-, and sulfhydryl-terminated). PEG has been found to be non-toxic and is approved by the FDA for use in drugs (parenterals, topicals, suppositories, nasal sprays), foods, and cosmetics. In solution PEG is a highly hydrated polymer, where each monomer (ethylene oxide unit) can bind up to three molecules of water. In addition, it is thought that PEG has the ability to influence the structure of several molecular layers of more loosely associated hydrating water molecules. Molecular simulations of the behavior of single surface-bound chains in water show the polymer exhibits a large degree of segmental flexibility. Thus, the polymer is assumed to occupy a large hydrodynamic volume in aqueous environments. These findings serve to explain why PEG is remarkably effective in excluding other polymers (natural and synthetic) from its presence. The exclusion of other polymers is the primary driving force behind PEG's ability to reject proteins, form two-phase systems with other synthetic polymers, and makes this polymer both nonimmunogenic and nonantigenic. When PEG is covalently attached to a protein, it typically transfers many of the polymer's favorable characteristics to the resultant conjugate. Because of the many beneficial properties mentioned above, PEG is well suited for protein modification.

As used herein, the term "PEG" includes any PEG polymer, including amino-reactive derivatives of PEG ("PEG reagents"). A variety of PEG reagents for protein conjugation are known. A typical PEG reagent is a linear PEG polymer with one end terminated in an ether linkage (e. g., O-methyl) and the other end functionalized with a reactive group. Other PEG reagents are branched or dendrimeric, again with a combination of non-reactive termini and reactive functional groups for linking to proteins. Alternatively, homo- or hetero-bifunctional PEG reagents with a combination of similar or dissimilar reactive functional groups may be used for linking to proteins.

Examples of PEG reagents described herein can be an aldehyde, an N-hydroxy succinimidyl carbonate, an N-hydroxy succinimidyl propionate, a p-nitrophenyl-carbonate, an N-maleimidyl, an N-succinimidyl, a thiol, or a benzotriazole-carbonate terminated species or other amino-reactive activated species of PEG.

The maleimide-derivatized PEG polymer may have a molecular weight in the range of, for example, 300 to 70,000 Da. The reactive functional groups may be separated from the PEG chain by linker groups. Optionally, the polymers have degradable internal bonds between the PEG and linkers. Accordingly, reactive groups on the PEG polymer may be electrophilically activated for reaction with protein nucleophiles. Examples of electrophilic groups are n-hydroxy succinimidyl carbonate, tresyl and aldehyde functionalities. PEG reagents with these functionalities will react to form covalent linkages to amino groups of proteins. A preferred PEG reagent for PEG conjugation to protein amino groups is the mPEG succinimidyl active ester of a propionic acid linker mPEG-SPA. Another preferred PEG reagent is monomethoxy PEG-aldehyde (mPEG-Ald).
polyethylene glycol (PEG) molecules have been already mentioned above. Further examples are described in WO 99/64016, in US Patent 6,177,074 or in US Patent 6,403,564 in relation to interferon, or have been described for other proteins such as PEG-modified asparaginase, PEG-adenosine deaminase (PEG-ADA) or PEG-superoxide dismutase (see for example, Fuertges et al. (1990) The Clinical Efficacy of Poly(Ethylene Glycol)-Modified Proteins J. Control. Release 11, 139-148). Other examples can, for example, be found in US Patent 4,179,337, US Patent 5,446,090, and US Patent 5,880,255. It is known in the art that such attachment may lead to an apparent increase in molecular mass and decreased blood clearance rate for the modified therapeutic protein (see e. g., US Patent 5,320,840), as well as to diminished immunogenicity, the extension of the duration of release from biodegradable polymer drug delivery systems, an increase in the drug loading achievable in a biodegradable drug delivery system relative to the unpegylated drug, and a reduced burst of drug relative to the unpegylated drug (see e.g. PCT publication WO 02/036169).

The polyoxyethylated polyols described herein include polyoxyethylated glycerol, polyoxyethylated sorbitol, polyoxyethylated glucose and derivatives, such as esters, thereof.

The activated derivatives described herein may comprise an amino-reactive derivative selected from the group consisting of an aldehyde, a thiol, an N-hydroxy succinimide, a succinimide, a maleimide, a PNP-carbonate, and a benzotrizole terminated hydrophilic polymer. According to the present invention, the activated derivative of a hydrophilic polymer is a maleimide-derivatized PEG, which is then reacted with free thiol groups of cysteine residues in the mutein of the invention, wherein these cysteine side chains may naturally occur in the protein or may be artificially introduced by mutagenesis. To render these thiol groups available for coupling chemistry, the protein may be subjected to a reduction step to reduce cystin bridge-forming cysteine residues. In one embodiment, this step uses a reducing agent, such as Tris(2-carboxyethyl)phosphine hydrochloride (TCEP) or β-mercaptoethanol.

The molecular weight of the maleimide-derivatized PEG polymers may range from about 300 to about 70000 Dalton.

It is described in US patents 6,500,930 or 6,620,413, carbohydrate oligo- and polymers such as starch or hydroxyethyl starch (HES) can be conjugated to a mutein for the purpose of serum half-life extension.

Further is described herein that in the conjugates, the lipocalin mutein may be selected from any mutein of a protein belonging to the lipocalin family of proteins. Exemplary lipocalins are retinol-binding protein (RBP), bilin-binding protein (BBP), apolipoprotein D (APO D), neutrophil gelatinase associated lipocalin (NGAL), tear lipocalin (TLPC), α₂-microglobulin-related protein (A2m), 24p3/uterocalin (24p3), von Ebners gland protein 1 (VEGP 1), von Ebners gland protein 2 (VEGP 2), and Major allergen Can f1 precursor (ALL-1). Lipocalin muteins described herein include muteins of human neutrophil gelatinase associated lipocalin (hNGAL), human tear lipocalin (hTLPC), human apolipoprotein D (APO D) and the bilin-binding protein of *Pieris brassicae.* The mutein of the invention is a mutein of human tear lipocalin (hTLc) as already defined above.

Human tear pre-albumin, now called tear lipocalin (TLPC or Tlc; SWISS-PROT Data Bank Accession No. P31025), was originally described as a major protein of human tear fluid (approximately one third of the total protein content) but has recently also been identified in several other secretory tissues including prostate, nasal mucosa and tracheal mucosa. Homologous proteins have been found in rat, pig, dog and horse.

Human neutrophil gelatinase-associated lipocalin (hNGAL, also termed Lcn2, SWISS-PROT Data Bank Accession Number P80188) is a 178 amino acid glycoprotein abundant in human plasma. Animal homologs to human Lcn2 are rat α₂-microglobulin-related protein (A2m; SWISS-PROT Data Bank Accession Number P31052) and mouse 24p3/uterocalin (24p3; SWISS-PROT Data Bank Accession Number P11672).

Human apolipoprotein D (Apo-D; SWISS-PROT Data Bank Accession Number P05090) is a component of high density lipoprotein that has no marked similarity to other apolipoprotein sequences. It has a high degree of homology to plasma retinol-binding protein and other members of the alpha 2 microglobulin protein superfamily of carrier proteins, also known as lipocalins.

The bilin-binding protein (BBP; SWISS-PROT Data Bank Accession Number P09464) is a blue pigment protein which is abundant in the butterfly *Pieris brassicae.*

In one embodiment of the invention, said lipocalin mutein as defined above has 70% to 98% sequence homology with the amino acid sequence of a human tear lipocalin mutein of SEQ ID NO: 49.

Further is described herein, lipocalin muteins having at least 50%, 60%, 70%, 75%, 80%, 85%, 90% or 95% sequence identity with the amino acid sequence of human tear lipocalin, human neutrophil gelatinase-associated lipocalin, rat α₂-microglobulin-related protein, mouse 24p3/uterocalin, bilin-binding protein, human apolipoprotein D, retinol-binding protein, von Ebners gland protein 1 (VEGP 1), von Ebners gland protein 2 (VEGP 2), or Major allergen Can f1 precursor (ALL-1).

The term "homology" as used herein in its usual meaning and includes identical amino acids as well as amino acids which are regarded to be conservative substitutions (for example, exchange of a glutamate residue by a aspartate residue) at equivalent positions in the linear amino acid sequence of two proteins that are compared with each other. By "identity" or "sequence identity" is meant a property of sequences that measures their similarity or relationship. The term "sequence identity" or "identity" as used in the present invention means the percentage of pair-wise identical residues - following (homology) alignment of a sequence of a polypeptide of the invention with a sequence in question - with respect to the number of residues in the longer of these two sequences. Identity is measured by dividing the number of identical residues by the total number of residues and multiplying the product by 100.

The percentage of sequence homology or sequence identity can, for example, be determined herein using the program BLASTP, version blastp 2.2.5 (November 16, 2002; cf. Altschul, S. F. et al. (1997) Nucl. Acids Res. 25, 3389-3402). The percentage of homology is based on the alignment of the entire polypeptide sequences (matrix: BLOSUM 62; gap costs: 11.1; cutoff value set to 10⁻³) including the propeptide sequences, using the human Lipocalin 2 as reference in a pairwise comparison. It is calculated as the percentage of numbers of "positives" (homologous amino acids) indicated as result in the BLASTP program output divided by the total number of amino acids selected by the program for the alignment. It is noted in this connection that this total number of selected amino acids can differ from the length of the human Lipocalin 2.

Further are described herein: Lipocalin muteins binding a given non-natural target with detectable affinity. The target may be a protein, protein domain, protein fragment or peptide.

As is described herein, the target that is bound by a tear lipocalin mutein is a protein or fragment thereof selected from the group of vascular endothelial growth factor (VEGF), vascular endothelial growth factor receptor 2 (VEGF-R2), interleukin 4 receptor alpha chain (IL-4R alpha), cytotoxic T-lymphocyte antigen-4 (CTLA-4), the receptor tyrosine kinase c-Met, or fragments thereof. Further are mentioned ligands that are an extracellular region or a domain of VEGF-R2, IL-4R alpha, CTLA-4 or c-Met. The ligands are typically of mammalian origin. These ligands are of human origin, but they may also be of mouse, rat, porcine, equine, canine, feline, bovine, marmoset or cynomolgus origin, to name only a few illustrative examples.

Further is described herein: The lipocalin mutein may comprise at least one mutated amino acid residues at any sequence position in the four peptide loops AB, CD, EF, and GH encompassing the natural lipocalin binding pocket. These loops form the known binding site of the lipocalins (which was therefore called the open end). In human tear lipocalin (hTLPC; SWISS-PROT Data Bank Accession No. P31025) the AB loop comprises positions 24-36, the CD loop comprises positions 53-66, the EF loop comprises positions 69-77 and the GH loop comprises positions 103-110 of the linear polypeptide sequence of native mature human tear lipocalin. The definition of these four loops is used herein in accordance with Flower (Flower, D.R. (1996), *supra* and Flower, D.R. et al. (2000), *supra*). In the bilin-binding protein of *Pieris brassicae* these four peptide loops, which are arranged at the end of the cylindrical β-barrel structure encompassing the binding pocket, correspond to segments in the linear polypeptide sequence comprising the amino acid positions 28-45, 58-69, 86-99, and 114-129 of the linear polypeptide sequence of the bilin-binding protein of *Pieris brassicae.*
Further are described herein lipocalin muteins comprising at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 mutated amino acid residues at any sequence position in the four peptide loops AB, CD, EF, and GH encompassing the natural lipocalin binding pocket.

Further are described herein lipocalin muteins comprising at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 mutated amino acid residues at any sequence positions corresponding to the sequence positions 24-36, 53-66, 79-84, and 103-110 of the linear polypeptide sequence of native mature human tear lipocalin (SWISS-PROT Data Bank Accession Number P31025). As is described herein, a lipocalin mutein comprising at least 2, 3, 4, 5, 6, 7, 8, 9, 10, 11, 12, 13, 14, 15, 16, 17, or 18 mutated amino acid residues at any sequence positions corresponding to sequence positions 26-34, 56-58, 80, 83, 104-106 and 108 of the linear polypeptide sequence of native mature human tear lipocalin. The respective sequence positions of other members of the lipocalin family of proteins can be readily determined by the skilled person using techniques and programs well-known in the art.

Lipocalin muteins may comprise the wild type (natural) amino acid sequence outside the mutated amino acid sequence positions. On the other hand, the lipocalin muteins discribed herein may also contain amino acid mutations outside the sequence positions in the loop regions subjected to mutagenesis as long as those mutations do not interfere with the binding activity and the folding of the mutein. Such mutations can be accomplished very easily on DNA level using established standard methods (Sambrook, J. et al. (1989) Molecular Cloning: A Laboratory Manual, 2nd Ed., Cold Spring Harbor Laboratory Press, Cold Spring Harbor, NY). Possible alterations of the amino acid sequence are insertions or deletions as well as amino acid substitutions. Such substitutions may be conservative, i.e. an amino acid residue is replaced with a chemically similar amino acid residue. Examples of conservative substitutions are the replacements among the members of the following groups: 1) alanine, serine, and threonine; 2) aspartic acid and glutamic acid; 3) asparagine and glutamine; 4) arginine and lysine; 5) isoleucine, leucine, methionine, and valine; and 6) phenylalanine, tyrosine, and tryptophan. One the other hand, it is also possible to introduce non-conservative alterations in the amino acid sequence. In addition, instead of replacing single amino acid residues, it is also possible to either insert or delete one or more continuous amino acids of the primary structure of tear lipocalin as long as these deletions or insertion result in a stable folded/functional mutein (see for example, the experimental section in which muteins with truncated N- and C-terminus are generated).

Such modifications of the amino acid sequence include directed mutagenesis of single amino acid positions in order to simplify sub-cloning of the mutated lipocalin gene or its parts by incorporating cleavage sites for certain restriction enzymes. In addition, these mutations can also be incorporated to further improve the affinity of a lipocalin mutein for a given target. Furthermore, mutations can be introduced in order to modulate certain characteristics of the mutein such as to improve folding stability, serum stability, protein resistance or water solubility or to reduce aggregation tendency, if necessary. For example, naturally occurring cysteine residues may be mutated to other amino acids to prevent disulphide bridge formation. However, it is also possible to deliberately mutate other amino acid sequence position to cysteine in order to introduce new reactive groups, for example for the conjugation to the moieties described herein or for the formation of non-naturally occurring disulphide linkages.

Further are described herein truncated lipocalin muteins as defined above, in which one or more of the N-terminal and/or C-terminal amino acids, such as the first four N-terminal amino acid residues and/or the last two C-terminal amino acid residues of the sequence of the mature lipocalin have been deleted.

Described herein are conjugates as mentioned above, wherein the lipocalin mutein comprises, consist essentially of or consists of the amino acid sequence set forth in any one of SEQ ID Nos. 1 - 48 and 50 - 110. Further are described lipocalin muteins having 70, 75, 80, 85, 88, 90, 92, 94, 96 or 98 % sequence identity with a lipocalin mutein of any of SEQ ID Nos. 1 - 48 and 50 - 110.

Further is described herein: Conjugation of one of the above moieties to the lipocalin muteins may occur via the N-terminus, the C-terminus or an amino acid side chain of the lipocalin mutein. To facilitate conjugation, the moiety may comprise a reactive group, such as those described above. Suitable amino acid side chains may occur naturally in the amino acid sequence of the respective lipocalin or may be introduced by mutagenesis. In case a suitable binding site is introduced via mutagenesis, one possibility is the replacement of an amino acid at the appropriate position by a cysteine residue. In one embodiment, such mutation includes at least one of Thr 40→ Cys, Glu 73→ Cys, Arg 90→ Cys, Asp 95→ Cys or Glu 131→ Cys substitution with respect to the linear polypeptide sequence of human tear lipocalin (SWISS PROT Databank entry P31025) or the corresponding substitutions in other members of the lipocalin family of proteins. The newly created cysteine residue at any of these positions can in the following be utilized to conjugate the mutein to a moiety prolonging the serum half-life of the mutein, such as PEG or an activated derivative thereof.

Described herein is a mutein of human tear lipocalin into which a cysteine is introduced at any of these sequence positions is the VEGF binding human tear lipocalin mutein S236.1-A22 (SEQ ID NO:22). Other examples for such mutations are known in the art and include those previously disclosed in PCT publications WO 99/16873, WO 00/75308, WO 03/029471, WO 03/029462, WO 03/029463, WO 2005/019254, WO 2005/019255, WO 2005/019256, WO 2006/56464, and WO 2008/015239.

In order to provide suitable amino acid side chains for conjugating one of the above moieties to the muteins described herein , artificial amino acids may be introduced by mutagenesis. Generally, such artificial amino acids are designed to be more reactive and thus to facilitate the conjugation to the desired moiety. One example of such an artificial amino acid that may be introduced via an artificial tRNA is para-acetyl-phenylalanine.

As is described herein, the lipocalin muteins and the moiety conjugated thereto may be used in the form of fusion proteins. Therein, the lipocalin mutein is fused at its N-terminus or its C-terminus to a protein, a protein domain or a peptide moiety.

As is described herein, the lipocalin mutein may be fused to another protein, protein domain or peptide, for example a second lipocalin mutein of the same or different binding specificity (which results in the formation of "Duocalins", cf. Schlehuber, S., and Skerra, A. (2001), Duocalins, engineered ligand-binding proteins with dual specificity derived from the lipocalin fold. Biol. Chem. 382, 1335-1342), and the resulting fusion protein comprising the lipocalin mutein may then be conjugated to the above moieties.

Techniques for the generation of lipocalin muteins are known in the art (See, for example Flower, D.R. (1996), *supra*; Flower, D.R. et al. (2000), *supra,* Skerra, A. (2000), *supra;* Skerra, A. (2001), *supra*; Schlehuber, S., and Skerra, A. (2002), *supra*; and PCT publications WO 99/16873, WO 00/75308, WO 03/029471, WO 03/029462, WO 03/029463, WO 2005/019254, WO 2005/019255, WO 2005/019256, WO 2006/56464, and WO 2008/015239).

The term "non-natural ligand", "non-natural binding partner" or "non-natural target", as used interchangeably herein, refers to a compound, which does not bind to the respective native lipocalin under physiological conditions. The target (ligand) may be any chemical compound in free or conjugated form, which exhibits features of an immunological hapten, a hormone such as steroid hormones or any biopolymer or fragment thereof, for example, a protein or protein domain, a peptide, an oligodeoxynucleotide, a nucleic acid, an oligo- or polysaccharide or conjugates thereof, a lipid or another macromolecule.

The lipocalin muteins comprised in the conjugates of the invention retain their ability to bind the desired target with detectable affinity, wherein the muteins have a binding affinity to c-Met with a dissociation constant of 20 nM or even less. The binding affinity of a mutein to c-Met can be measured by a multitude of methods such as fluorescence titration, competition ELISA or surface plasmon resonance (BIAcore).

The invention also relates to a pharmaceutical formulation for controlled release of a lipocalin mutein of the invention, the formulation comprising the lipocalin mutein of the invention conjugated to a maleimide-derivatized polyethylenglycol (PEG) and a pharmaceutically acceptable excipient.

The terms "controlled release" or "sustained release" refer to control of the rate and/or quantity of lipocalin mutein molecules delivered according to the drug delivery formulations of the invention. The controlled release can be continuous or discontinuous, and/or linear or nonlinear. This can be accomplished using one or more types of polymer compositions, drug loadings, inclusion of excipients or degradation enhancers, administered alone, in combination or sequentially to produce the desired effect. Zero order or linear release is generally construed to mean that the amount of the bioactive molecule released over time remains relatively constant as a function of amount/unit time during the desired time frame. Multi-phasic is generally construed to mean that release occurs in more than one "burst".
It is described herein that the liposome encapsulates the lipocalin mutein or conjugate thereof and that the liposomes are dispersed or emulgated in an aqueous base medium.

It is described herein that the polymer is a biodegradable polymer. The biodegradable polymer is selected from the group consisting of polylactides, polyglycolides, poly(lactide-co-glycolide)s, polylactic acids, polyglycolic acids, poly(lactic acid-co-glycolic acid)s, polycaprolactones, polycarbonates, polyesteramides, polyanhydrides, poly(amino acids), polyorthoesters, polyacetyls, polycyanoacrylates, polyetheresters, polydioxanones, polyalkylen alkylates, copolymers of polyethylene glycol and polylactides or poly(lactide-co-glycolide)s, biodegradable polyurethanes, and certain types of protein and polysaccharide polymers, as well as blends and copolymers thereof. The biodegradable polymer is selected from the group consisting of polyhydroxy acids, polylactic acids, polylactides, polyglycolides, polyglycolic acids, and copolymers thereof as well as derivatives thereof. The biodegradable polymers may also include or consist of polyanhydrides, polyorthoesters, and polysaccharide polymers. One suitable polyanhydride copolymer is a copolymer of bis(*p*-carboxyphenoxy) propane and sebacic acid. Further suitable biodegradable hydrophilic polymers include biopolymers, such as the above-mentioned protein and polysaccharide polymers, including, but not limited to hyaluronan, chitosan, gelatin, collagen, starch, dextrin, and cellulose. These biomacromolecules may be cross-linked to form a three-dimensional hydrogel.

Further is described that the biodegradable polymer is poly-(D,L-lactide-co-glycolide). If poly-(D, L-lactide-co-glycolide) is chosen as the polymer for forming a pharmaceutical formulation, the formulation the poly-(D,L-lactide-co-glycolide) may contain about 55 to about 80 mole % lactide monomer and about 45 to about 20 mole % glycolide. The poly-(D, L-lactide-co-glycolide) may also contain about 65 to about 75 mole % lactide monomer and about 35 to about 25 mole % glycolide. The poly-(D, L-lactide-co-glycolide) used can contain terminal acid groups.

Further is described herein that the biodegradable polymer is a polylactic acid polymer or copolymer comprising lactide units substituted with alkyl moieties. The biodegradable polymer may, for example, comprise, consist essentially of or consist of poly(hexyl-substituted lactide) or poly(dihexyl-substituted lactide).

Further is described herein that in the pharmaceutical formulation, the biodegradable polymer is formulated into microparticles or nanoparticles encapsulating the conjugate. Therein, the formulations may be based on microparticles or nanoparticles formed of the combination of biodegradable polymers such as polylactic acid (PLA), polyglycolic acid (PGA), and copolymers thereof and the lipocalin muteins or conjugates mentioned herein, such as conjugates of the lipocalin muteins with hydrophilic polymers, for example polyethylene glycol or polypropylene glycol, to provide controlled release.

It is further described herein that the pharmaceutical formulation may be in form of an implant or implantable device. The implant or implantable device may have any shape and includes implantable rods, plugs, discs, pellets, membranes or sheets. Such implantable devices may be produced by known methods such as molding, extrusion and film-preparation from suitable polymers. If the pharmaceutical formulation is an implant or implantable device, the implant material may be biodegradable or nonbiodegradable, such as biodegradable or nonbiodegradable polymers. Suitable biodegradable polymers include those detailed above.

Nonbiodegradable polymers can remain in a living organism for prolonged period of time, such as years, without substantial breakdown. Such materials include polymers of polyvinyl alcohols (PVA) and ethylene vinyl acetate (EVA), to name only a few.

Further is described: The pharmaceutical formulations, in particular the implantable devices may also contain antimetabolites, for example to prevent fibrosis at the site of the implant.

Further are described herein biodegradable microparticles for controlled release of lipocalin muteins or polymer conjugated lipocalin muteins.

As used herein, "microparticles" refers to particles having a diameter of preferably less than 1.0 mm, typically between 1.0 and 200.0 microns and more preferably between 1.0 and 100.0 microns. A microparticle may have any suitable particle size as long as the same provides for a satisfying sustained release of the lipocalin mutein of interest. A microparticle may have a mean particle size (diameter) smaller than 100 micrometer, for example, in the range from about 20 to about 80 micrometer or a mean partice size (diameter) in the range from about 40 to about 50 micrometer. The term "microparticles" include microspheres, which are typically solid spherical microparticles. The term "microparticles" also include microcapsules, which are spherical microparticles typically having a core of a composition distinct from the surrounding shell. For the purpose of this disclosure, the terms microsphere, microparticle and microcapsule are used interchangeably.

The mass (ratio) of the lipocalin mutein to the mass of the polymer that is used for the preparation of a pharmaceutical composition/formulation of the invention should be chosen such that a satisfying controlled sustained release is achieve. It is described herein that the mass of the lipcalin mutein with respect to the mass of the polymer that is used for the prepation of a formulation such as a microparticle or nanoparticle is 15 % or less or that the mass of the mutein in the pharmaceutical composition with respect to the mass of the polymer is 10 % or less.

Microparticles for use can be made using a variety of biodegradable polymers used for controlled release formulations, as are well known in the art. As mentioned above, suitable polymers for example include, but are not limited to polylactides, polyglycolides, poly(lactide-co-glycolide)s, polylactic acids, polyglycolic acids, poly (lactic acid-co-glycolic acid)s, polycaprolactones, polycarbonates, polyesteramides, polyanhydrides, polyamino acids, polyorthoesters, polyacetyls, polycyanoacrylates, polyetheresters, polydioxanones, polyalkylen alkylates, copolymers of polyethylene glycol and polylactides or poly(lactide-co-glycolide)s, biodegradable polyurethanes, and certain types of protein and polysaccharide polymers, as well as blends and copolymers thereof. Examples of protein and polysaccharide polymers have been disclosed above and include, but are not limited to hyaluronan, chitosan, gelatin, collagen, starch, dextrin, and cellulose.

For the purposes of the present invention, the term "biodegradable" refers to polymers that dissolve or degrade *in vivo* within a period of time that is acceptable in a particular therapeutic situation. Such dissolved or degraded product may include a smaller chemical species. Degradation can result, for example, by enzymatic, chemical and/or physical processes. Biodegradation takes typically less than five years and usually less than one year after exposure to a physiological pH and temperature, such as a pH ranging from 6 to 9 and a temperature ranging from 22°C to 38°C.

Polymers described herein include poly (hydroxy acids), especially poly(lactic acid-co-glycolic acid) (poly(D,L-lactide-co-glycolide);"PLGA"), polyglycolides (PGA) and polylactides (PLA) or derivatives thereof that degrade by hydrolysis following exposure to physiological pH, e.g. the aqueous environment of the body. The polymer is then hydrolyzed to yield lactic and glycolic acid monomers, which are normal byproducts of cellular metabolism. The rate of polymer disintegration can vary from several weeks to periods of greater than one year, depending on several factors including polymer molecular weight, ratio of lactide to glycolide monomers in the polymer chain, and stereoregularity of the monomer subunits (mixtures of L and D stereoisomers disrupt the polymer crystallinity enhancing polymer breakdown). In illustrative embodiments the poly-(D,L-lactide-co-glycolide) that is used to prepare a formulation (for example a microparticle) may contain about 55 to 80 mole % lactide monomer and about 45 to 20 mole % glycolide. The poly-(D, L-lactide-co-glycolide) may alternatively contain about 65 to 75 mole % lactide monomer and about 35 to 25 mole % glycolide. The poly-(D, L-lactide-co-glycolide) used can contain terminal acid groups. Microspheres may also contain blends of two and more biodegradable polymers, of different molecular weight and/or monomer ratio.

Derivatized biodegradable polymers described herein are also suitable for use, including hydrophilic polymers attached to PLGA and the like. The hydrophilic polymer described herein is selected from the group consisting of polyethylene glycol, polypropylene glycol, copolymers of polyethylene glycol and polypropylene glycol, and polyvinyl pyrrolidone.

To form microspheres, in particular, a variety of techniques known in the art can be used. These include, for example, single or double emulsion steps followed by solvent removal. Solvent removal may be accomplished by extraction, evaporation or spray drying among other methods.

In the solvent extraction method, the polymer is dissolved in an organic solvent that is at least partially soluble in the extraction solvent such as water. The lipocalin mutein conjugate, either in soluble form or dispersed as fine particles, is then added to the polymer solution, and the mixture is dispersed into an aqueous phase that contains a surface-active agent such as polyvinyl alcohol. The resulting emulsion is added to a larger volume of water where the organic solvent is removed from the polymer/bioactive agent to form hardened microparticles.

In the solvent evaporation method, the polymer is dissolved in a volatile organic solvent. The bioactive molecule, i.e. the lipocalin mutein conjugate, either in soluble form or dispersed as fine particles, is then added to the polymer solution, and the mixture is suspended in an aqueous phase that contains a surface-active agent such as polyvinyl alcohol. The resulting emulsion is stirred until most of the organic solvent evaporates, leaving solid microspheres.

In the spray drying method, the polymer is dissolved in a suitable solvent, such as methylene chloride (e.g., 0.04 g/ml). A known amount of the lipocalin mutein conjugate is then suspended (if insoluble) or co-dissolved (if soluble) in the polymer solution. The solution or the dispersion is then spray-dried. Microspheres ranging in diameter between one and ten microns can be obtained with a morphology, which depends on the selection of polymer.

Other known methods, such as phase separation and coacervation, and variations of the above, are known in the art and also may be employed in the present invention.

Further are described herein biodegradable nanoparticles for controlled release of lipocalin muteins and polymer conjugated lipocalin muteins. As used herein, the term "nanoparticles" refers to particles having a diameter of preferably between about 20.0 nanometers and about 2.0 microns, typically between about 100 nanometers and 1.0 micron.

Formulation of nanoparticles can be achieved essentially as described above for microparticles, except that high speed mixing or homogenization is used to reduce the size of the polymer/bioactive agent emulsions to below about 2.0 microns, preferably below about 1.0 micron. For example, suitable techniques for making nanoparticles are described in WO 97/04747.

These controlled release formulations can be administered by injection, by inhalation, nasally, or orally.

Generally, the encapsulation of pharmaceuticals in biodegradable polymer microspheres and nanospheres can prolong the maintenance of therapeutic drug levels relative to administration of the drug itself. Sustained release may be extended up to several months depending on the formulation and the active molecule encapsulated. However as therapeutic proteins are prone to damage caused by the procedures required to encapsulate them in the polymeric carriers, and the charged, polar nature of many proteins may limit the extent of encapsulation in polymer drug carriers and may lead to rapid loss of a fraction of the encapsulated bioactive molecule when first administered ("burst"), it is advantageous to attach hydrophilic polymers to these therapeutic proteins to protect them from degradation and denaturation under the conditions of encapsulation in drug carriers. Additionally this increases the amount of modified protein that can be encapsulated relative to the unmodified protein. Moreover, the immunogenicity ofpegylated therapeutic proteins encapsulated in biodegradable polymer drug delivery carriers is decreased relative to non-pegylated proteins in the carriers, particularly when administered by subcutaneous or intramuscular injection or inhalation or mucosal delivery (e.g., oral or nasal delivery). Such diminished immunogenicity is particularly advantageous when biodegradable polymers are used for oral delivery. Furthermore, it has been reported that pegylated proteins, peptides, oligosaccharides and oligonucleotides, which normally are not absorbed from the gastro-intestinal tract, are made bioavailable by administration in biodegradable polymer systems, particularly nanospheres. These advantages are described in more detail in PCT publication WO 02/36169.

The pharmaceutical compositions of the present invention can be used to improve *in vivo* delivery of lipocalin muteins or conjugates thereof in several respects, such as reduced immunogenicity, increased bioavailability, increased duration, increased stability, decreased burst and/or controlled, sustained release of the lipocalin muteins *in vivo.*

In the present invention, the lipocalin mutein is conjugated to a maleimide-derivatized polyethylene glycol moiety. The maleimide-derivatized polyethylene glycol may be linear or branched and may have a molecular mass of about 5, 7, 10, 12, 15, 20, 25, 30, 35, 40, 45, 50, 55, 60, 65, or 70 kDa, preferably of about 5 to about 40 kDa, more preferably of about 15 to 25 kDa, for example 20 kDa. Further is described herein that a lipocalin mutein conjugated is formulated with a polylactide, polyglycolide or a copolymer such as poly(D,L-lactide-co-glycolide) thereof, including alkylated derivatives of lactides and glycolides. The poly(D,L-lactide-co-glycolide) may contain about 55 to 80 mole % lactide monomer and about 45 to 20 mole % glycolide. The poly-(D,L-lactide-co-glycolide) may also contain about 65 to 75 mole % lactide monomer and about 35 to 25 mole % glycolide. It is described herein that such a formulation is in the form of microparticles or nanoparticles.In the present invention, the mutein is a human tear lipocalin.

Furthr is described herein: The lipocalin mutein in unconjugated form is formulated with a polylactide, polyglycolide or a copolymer thereof, including alkylated derivatives of lactides and glycolides.

In the formulations of the invention, about 30 to 90 %, preferably 40 to 70 % of the lipocalin mutein are released in active form, i.e. retain their ability to bind a given ligand. The release can be over a period of time depending on the actual composition of the formulation. The period of time may comprise one or more days, for example 2 to about 70 days, preferably 20 to 60 days.

Further is described herein a method for controlled systemic or local delivery of a lipocalin mutein to a subject comprising administering to the subject the conjugates or pharmaceutical formulations of the invention.

In such methods, the controlled local delivery of a lipocalin mutein may be facilitated by administration to a confined body compartment or organ of the subject. It has been found that the administration of lipocalin mutein formulations to a confined body compartment or organ of a subject, such as a mammal, preferably a human being, can facilitate a sustained release effect, since the half-life of the lipocalin mutein in the respective body compartment or organ may differ from the systemic half-life. Thus, by local administration of the lipocalin mutein conjugates or formulations of the invention, local sustained release may be achieved while at the same time avoiding prolonged systemic exposure. This may be attributed to the fact that the metabolic degradation or excretion of the lipocalin muteins differs between distinct body compartments or organs. For example, the half-life of the lipocalin muteins in the intraocular cavity is, compared to the serum half-life, prolonged by a factor of about 150. Thus, the released mutein may have a comparably longer half-life in the respective body compartment or organ than in the serum so that lipocalin muteins leaving the confined body compartment or organ are rapidly degraded or cleared by renal filtration, whereas their half-life in the body compartment or organ is long enough to achieve the desired pharmacological effect.

Described herein is that the confined body compartment is an eye, a joint, the brain, the spine, a tumor or a bodily lumen or cavity.

In these methods described herein, the conjugate or formulation is administered orally, by inhalation, mucosal delivery, intracutaneous injection, subcutaneous injection, intramuscular injection, intravenous injection, intravitreal injection, intraarticular injection, intracranial injection, intraspinal injection, intratumoral injection or implantation.

In these methods, the subject may be a mammal, preferably a human.

Additionally is described herein a method for preparing a lipocalin mutein-polymer conjugate, the method comprising contacting a lipocalin mutein with a hydrophilic polymer in the presence of at least one organic solvent and at least one metal chelator, under conditions that promote the formation of a conjugate of the mutein and the polymer, and isolating the conjugate. The conjugate can be isolated using a variety of standard techniques such as column chromatography.

The hydrophilic polymer described herein is selected from the group consisting of polyethylene glycol, polyethylene glycol/polypropylene glycol copolymers, polyoxyethylated glycerol, and linear, branched and amino-reactive derivatives thereof. Suitable amino-reactive derivatives include, for example, aldehydes, N-hydroxy succinimide esters of PEG-carboxylic acids, PNP-carbonates, and a benzotrizole terminated hydrophilic polymer derivatives. Other activated derivatives of PEG include, but are not limited to and N-maleimide PEG derivatives. Typically, the hydrophilic polymer and lipocalin mutein are contacted at a molar ratio of about 10:1-1:1, preferably 3:1 to 1.2:1, more preferably 1.7:1 to 1.5:1.

Suitable organic solvents for use in the invention include a wide variety of known solvents including, but not limited to, water-miscible organic solvents, such as dichloromethane. ethanol, methanol, DMSO, dioxane, DMF, and NMP. Typically, the organic solvent, preferably dioxane, is present at a concentration (v/v) of about 0 to 25%, preferably from 2-20%, more preferably from 5- 15% or from 0.1 - 10%.

Suitable metal chelators for use in the invention also include a wide variety of known compounds including, but not limited to, aminopolycarboxylic acids, such as, ethylenediaminetetraacetic acid (EDTA), diethylenetriamine pentaacetic acid (DTPA), nitrilotriacetic acid (NTA), N-2-acetamido-2-iminodiacetic acid (ADA), bis(aminoethyl) glycolether-N,N,N',N'-tetraacetic acid (EGTA), trans-diaminocyclohexane tetraacetic acid (DCTA), glutamic acid, and aspartic acid; and hydroxyaminocarboxylic acids, such as, for example, N-hydroxyethyliminodiacetic acid (HIMDA), N,N-bis-hydroxyethylglycine (bicine) and N-(trishydroxymethylmethyl)glycine (tricine); and N-substituted glycines, such as glycylglycine. Other suitable chelators include 2-(2-amino-2-oxoethyl) aminoethane sulfonic acid (BES) and deferoxamine (DEF). Suitable chelators used in the methods described herein include, for example, those that bind to metal ions in solution to render them unable to react with available oxygen, thereby minimizing or preventing generation of-OH radicals which are free to react with and degrade the protein. Such chelators can reduce or prevent degradation of a protein that is formulated without the protection of a chelating agent.

Chelating agents used in the invention can be present in their salt form, e. g., carboxyl or other acidic functionalities of the foregoing chelators. Examples of such salts include salts formed with sodium, potassium, calcium, and other weakly bound metal ions. As is known in the art, the nature of the salt and the number of charges to be neutralized will depend on the number of carboxyl groups present and the pH at which the stabilizing chelator is supplied. As is also known in the art, chelating agents have varying strengths with which particular target ions are bound. In general, heavy metal ions are bound more strongly than their similarly charged lower molecular weight counterparts.

The chelator used in the methods described herein may also be selected from EDTA, EGTA, and other multivalent cation chelators known in the art.

Herein, the chelator is selected from the group consisting of EDTA, deferoxamine (DEF), diethylenetriamine pentaacetic acid (DTPA), and bis(aminoethyl)glycolether-N,N,N',N'-tetraacetic acid (EGTA).

Generally, the chelator, preferably EDTA, is present at a concentration from about 0.1-10 mM, preferably from 1-5 mM, more preferably from 1-3 mM.

Further is described herein: The lipocalin mutein and hydrophilic polymer (e. g., PEG) are contacted (i. e., reacted or conjugated) in an aqueous solution at a protein concentration of about 0.1-5% by weight, preferably from 0.5-1.5%. As is described herein, the lipocalin mutein and hydrophilic polymer are contacted in an aqueous solution at a pH of about 5.0-7.5, preferably pH 6.5 to 7.2, more preferably about 7.0. The pH can be controlled by inclusion of buffer salts, addition of organic acids/bases, or addition of common inorganic acids/bases. As is described herein, the hydrophilic polymer and lipocalin mutein are contacted at a temperature of about 4°C to 50°C, preferably about 15°C to 25°C.

Once formed, the protein-polymer conjugate is then separated from unwanted side reaction products and unreacted lipocalin mutein. This can be achieved using a variety of known techniques, such as chromatography. For example, ion exchange chromatography (e.g., cation exchange) is employed, and the desired conjugate may be collected, concentrated, desalted and dried.

It is described herein that the method further comprises the step of quenching the reaction (i. e., conjugation) of the lipocalin mutein and the hydrophilic polymer, prior to isolating the conjugation product. For example, this is achieved by reducing the pH of the reaction to about 1-4, preferably about 2-3, more preferably about 2.4 to 2.6.

Further is described herein that the conjugates obtained according to the afore-mentioned methods for preparing a lipocalin mutein polymer-conjugate.

Particular protein-polymer conjugates produced by the methods described herein include, for example, lipocalin mutein-polymer conjugates, preferably lipocalin mutein-PEG conjugates (PEGylated lipocalin mutein). This can include any ofthe above-described lipocalin muteins. It is described herein that the lipocalin mutein is specifically reacted (PEGylated) at the N-terminus, the C-terminus or an amino acid side chain of the lipocalin mutein. To facilitate conjugation, the PEG moiety may comprise a reactive group. As described above, suitable amino acid side chains may occur naturally in the amino acid sequence of the respective lipocalin or may be introduced by mutagenesis. In case a suitable binding site is introduced via mutagenesis, one possibility is the replacement of an amino acid at the appropriate position by a cysteine residue. Such mutation includes at least one of Thr 40→ Cys, Glu 73→ Cys, Arg 90→ Cys, Asp 95→ Cys or Glu 131→ Cys substitution with respect to the linear polypeptide sequence of human tear lipocalin (SWISS PROT Databank entry P31025) or the corresponding substitutions in other members of the lipocalin family of proteins. The resulting PEGylated lipocalin mutein can be administered therapeutically in any suitable formulation as is well known in the art.It is described herein that the conjugate is administered in a sustained release formulation by, for example, encapsulating the conjugate in a biodegradable polymer prior to administration.

This single-step method allows rapidly and efficiently preparing lipocalin mutein-polymer conjugates. The preparation method is disclosed in more detail in connection with insulin in PCT publication WO 2004/091494.

Further is described a method of making a controlled release composition comprising: combining an organic phase comprising a lipocalin mutein and a polymer with an aqueous phase; and recovering said composition. The aqueous phase may comprise an organic ion, wherein said organic ion is present to reduce possible degradation of the lipocalin mutein.

For the purposes of the present invention, the terms "organic phase" and "discontinuous phase" are interchangeable and refer to the solution of solvent, polymer and lipocalin mutein created in the methods described herein that will then be contacted with an aqueous phase through an emulsion process in order to create the controlled release compositionsdescribed herein.

For the purposes of the present invention, the term "degradation" refers to any unwanted modification to the lipocalin mutein, such as acylation, or to the polymer, such as lysis.

For the purposes of the present invention, the terms "aqueous phase" and "continuous phase" are interchangeable and refer to the solution of water and organic ion agent created in the methods described herein that will then be contacted with an organic phase through an emulsion process in order to create the controlled release compositions described herein.

For the purposes of the present invention, the term "combining" refers to any method described herein of putting two or more materials together. Such methods include, but are not limited to, mixing, blending, commingling, concocting, homogenizing, incorporating, intermingling, fusing, joining, shuffling, stirring, coalescing, integrating, confounding, joining, uniting, and the like.

For the purposes of the present invention, ranges may be expressed herein as from "about" or "approximately" one particular value, and/or to "about" or "approximately" another particular value. When such a range is expressed, another embodiment includes from the one particular value and/or to the other particular value. It will be further understood that the endpoints of each of the ranges are significant both in relation to the other endpoint, and independently of the other endpoint. Usually, the term "about" includes ± 10% of the given value.

For the purposes of the present invention, the term "organic ion" refers to cationic and anionic materials. Exemplary organic ions include pamoate, naphthoate, cholate and the like. Organic ions may be present in their salt or acid forms. Organic ions of use in the present invention include anionic and cationic materials. Anionic materials include, but are not limited to, the following organic acids and their salts: dodecylsulfuric, cholic, trifluoromethyl-p-toluic, 2-naphthalene sulfonic, 2, 3-naphthalene dicarboxylic, 1-hydroxy-2-naphthoic, 3-hydroxy-2-naphthoic, 2-naphthoic, and salicylsalicylic. In addition, organic forms of sulfates, sulfonates, phosphates, and phosphonates are suitable organic ions. Salt forms of the anionic materials may include sodium, ammonium, magnesium, calcium and the like. Cationic molecules include, but are not limited to, those having an ammonium or guanidinium group or a substituted ammonium group. Organic anionic agents are used with bioactive agents that have one or more functional groups having, or capable of adopting, a positive charge, such as an ammonium or guanidinium group. Organic cationic agents can be used with bioactive agents that have one or more functional groups having or capable of adopting a negative charge such as a carboxyl, sulfate, sulfonate, phosphate, or phosphonate group. Organic ion agents of use in the present invention may be soluble in water and in the organic phase to the extent required to enhance encapsulation efficiency and drug loading. In a particular embodiment, enhanced encapsulation efficiency and drug loading are achieved via decreased degradation of the bioactive agent. In a particular embodiment, the concentration of the organic ion agent in the aqueous phase ranges from about 0.5 to 100 mM. In another particular embodiment, the organic ion ranges from about 5 to 40 mM.

For the purposes of the present invention, a "controlled release composition" shall refer to any formulation with a different release profile than native bioactive agent. Typically release profiles will include physiologically detectable concentrations of a bioactive agent over a period of at least one week, at least one month, at least 45 days, or for longer than 45 days.

In these methods described herein, the methods further include the steps of combining a lipocalin mutein and a polymer in an organic phase and/or combining an organic ion in an aqueous phase.

In the methods described herein, the organic and aqueous phases are combined in an emulsion process to produce a controlled release composition. The emulsion may comprise droplets of the organic phase dispersed in the aqueous phase. Solvent may subsequently be removed from the emulsion droplets to form hardened microparticles. In a particular embodiment, the solvent is removed by evaporation. Further, the solvent can be removed by extraction into an extraction liquid; for example, the extraction liquid may be water. Further is described that the solvent is removed by filtration.

The hardened microparticles may then be recovered from the aqueous phase and dried.

Further is described herein that the emulsion is produced by stirring the organic and aqueous phases.Further, the emulsion is produced by use of a mixer. Further, the mixer is a static mixer. Further is described that the emulsion is produced by use of turbulent mixing or without turbulent mixing.

The emulsion process may be carried out at any temperature between the boiling point and freezing point of the components. The temperature may range from about 0°C to about 100°C and is typically between 5°C and 75°C. In a particular embodiment, the emulsion process is carried out between about 15°C to about 60°C.

As si further described herein, the method includes contacting an organic phase comprising a polymer and a bioactive agent with a water phase comprising an organic ion wherein an effective quantity of an organic ion leaves the aqueous phase and enters the organic phase.

As is described herein, the organic phase comprises a solvent selected from the group consisting of, but not limited to, dichloromethane, ethyl acetate, benzyl alcohol, acetone, acetic acid and propylene carbonate. The organic phase may also comprise other solvents in which the biodegradable polymer is soluble.For example, the solvent is ethyl acetate or dichloromethane.

It si further described herein that the organic phase further includes a cosolvent. They are optionally used to promote solubility of the bioactive agent in the organic phase.

The co-solvent may be selected from the group consisting of, but not limited to, dimethyl sulfoxide, dimethyl formamide, N-methylpyrrolidinone, PEG200, PEG400, methyl alcohol, ethyl alcohol, isopropyl alcohol, benzyl alcohol and water.

The co-solvent may be present between 0 and 90% w/w of the solvent of the organic phase. In another particular embodiment, the cosolvent is present between 0 and 50% w/w ofthe solvent of the organic phase.

The lipocalin mutein may be dissolved first in an appropriate volume ofthe cosolvent which is then added to the solvent of the organic phase, optionally having the biodegradable polymer dissolved therein, so as to form a solution of all the components ofthe organic phase. A person of ordinary skill can adjust the volumes and order of addition to achieve the desired solution of lipocalin mutein and biodegradable polymer. The biodegradable polymer may be present in the organic phase at a concentration of 2-40% w/w. The biodegradable polymer may be present in the organic phase at a concentration of 5-20% w/w.

Further is described herein: The aqueous phase further includes an emulsifying agent. The emulsifying agent may be selected from the group consisting of, but not limited to, poly (vinyl alcohol), albumin, lecithin, vitamin E-D-alpha-tocopheryl polyethylene glycol (TPGS) and polysorbates. The emulsifying agent may be present at a final concentration ranging from about 0.1 to 10% (w/w), at a final concentration of about 1.0% to about 8 % (w/w), of about 1.0% to about 6 % (w/w), of about 1.5% to about 5% (w/w), or at a concentration between 0.5 to 5% (w/w).

In the emulsion process to make a controlled release composition the organic phase that contains the lipocalin mutein comprises a solvent selected from the group consisting of dichloromethane, ethyl acetate, benzyl alcohol, acetone, acetic acid and propylene carbonate.

The organic solvent comprises dichloromethane as solvent and methanol, acetic acid and H₂O as cosolvent (cf., Example 6). The organic solvent may consist of dichloromethane (only) and comprises methanol, acetic acid and H₂O as cosolvent (cf., Example 6).

In embodiments in which the organic solvent comprises or consists of dichloromethane as solvent and methanol, acetic acid and H₂O as cosolvent, the organic phase comprises with respect to the total volume of the organic phase about 70 % to about 85 % (v/v) dichloromethane, about 10 % to about 15 % (v/v) methanol, about 5 to about 10% acetic acid (v/v) and about 0.1 % to about 1.0 % (v/v) water. In other such embodiments, the organic phase comprises with respect to the total volume of the organic phase about 75% to 82% (v/v) dichloromethane, about 11% to about 14% (v/v) methanol, about 6% to about 8.5 % methanol and about 0.1 % to about 0.8 % (v/v) water. In this respect, it is noted that in cases where the organic phase contains only dichloromethane, methanol, acetic acid and H₂O as solvent/cosolvent, their respective volume given in the ranges above is of course chosen such that total volume adds up to 100 % (see also Example 6).

In a certain embodiment, the organic ion is at a final concentration ranging from about 0.1 to 1000 mM. In a particular embodiment, they are dissolved at a concentration of between 1 to 100 mM. The concentration may be adjusted for each particular organic ion agent and bioactive agent to achieve the desired drug loading and encapsulation efficiency.

It is described herein: The controlled release composition is selected from the group consisting of, but not limited to, microparticles and nanoparticles. The microparticles and nanoparticles are biodegradable.

It is also described herein that the polymer may be selected from the group consisting of, but not limited to, polylactides, polyglycolides, poly(lactide-co-glycolide)s, poly(lactic acid), poly (glycolic acid), poly (lactic acid-co-glycolic acid), polycaprolactone, polycarbonates, polyesteramides, polyanhydrides, poly(amino acids), polyorthoesters, polyacetyls, polycyanoacrylates, polyetheresters, polydioxanones, polyalkylene alkylates, copolymers of polyethylene glycol and polyorthoester, biodegradable polyurethanes, and blends and copolymers thereof.

As is described herein, the emulsion process is selected from the group consisting of oil-in-water and water-oil-water.

The methods described herein may be practiced with any known emulsion process.

Further is described: The organic ion is selected from the group consisting of anionic and cationic materials. The organic ion may be a salt of an organic acid. The organic ion may be selected from trifluoromethyl-p-toluate, cholate, 2-naphthalene sulfonate, 2,3-naphthalene dicarboxylate, 1-hydroxy-2-naphthoate, 3-hydroxy-2- naphthoate, 2-naphthoate and salicylsalicylate or organic derivatives of sulfates, sulfonates, phosphates, and phosphonates.

It is described herein: Degradation includes acylation. The acylation reaction involves nucleophilic attack of an amino group of the lipocalin mutein, such as the N-terminal amino group or an amino group of an amino acid side chain, directed to a carbonyl carbon of a polyester such as poly(D,L-lactide-co-glycolide). In the prepared composition, the degradation of the lipocalin mutein is prevented or reduced by facilitated protonation of potential nucleophiles (e. g., amino groups), thus rendering the nucleophiles less apt to participate in acylation reactions with the PLGA polymer backbone or fragments thereof.

Degradation includes lysis of the polymer. Excessive lysis may lead to rapid loss of polymer molecular weight and premature release of bioactive agent.

It is described herein that the lipocalin mutein content may be increased relative to the bioactive agent content of compositions prepared by the methods described herein in the absence of an organic ion.

Further is described a controlled release composition made by the above outlined methods.

The use of the conjugates, pharmaceutical formulations or controlled release compositions according to the invention for the controlled delivery of the lipocalin mutein, for extending the *in vivo* half-life of the lipocalin mutein, for increasing the bioavailability of the lipocalin mutein, or for decreasing the immunogenicity ofthe lipocalin mutein upon administration to a subject is also described.

It is further described: The lipocalin mutein conjugates, pharmaceutical formulations or controlled release compositions can be administered via any parenteral or non-parenteral (enteral) route that is therapeutically effective for proteinaceous drugs. Parenteral application methods comprise, for example, intracutaneous, subcutaneous, intramuscular, intratracheal, intranasal, intravitreal, intraarticular, intracranial, intraspinal, intratumoral or intravenous injection and infusion techniques, e.g. in the form of injection solutions, infusion solutions or tinctures, as well as aerosol installation and inhalation, e.g. in the form of aerosol mixtures, sprays or powders. An overview about pulmonary drug delivery, i.e. either via inhalation of aerosols (which can also be used in intranasal administration) or intratracheal instillation is given by J.S. Patton et al. The lungs as a portal of entry for systemic drug delivery. Proc. Amer. Thoracic Soc. 2004, Vol. 1, pages 338-344, for example). Non-parenteral delivery modes are, for instance, orally, e.g. in the form of pills, tablets, capsules, solutions or suspensions, or rectally, e.g. in the form of suppositories. The lipocalin mutein conjugates and pharmaceutical compositions of the invention can be administered systemically or topically in formulations containing conventional non-toxic pharmaceutically acceptable excipients or carriers, additives and vehicles as desired.

The lipocalin mutein conjugates, pharmaceutical formulations or controlled release compositions are administered parenterally to a mammal, and in particular to humans. Corresponding administration methods include, but are not limited to, for example, intracutaneous, subcutaneous, intramuscular, intratracheal or intravenous injection and infusion techniques, e.g. in the form of injection solutions, infusion solutions or tinctures as well as aerosol installation and inhalation, e.g. in the form of aerosol mixtures, sprays or powders. A combination of intravenous and subcutaneous infusion and /or injection might be most convenient in case of compounds with a relatively short serum half life. The pharmaceutical composition may be an aqueous solution, an oil-in water emulsion or a water-in-oil emulsion.

In this regard it is noted that transdermal delivery technologies, e.g. iontophoresis, sonophoresis or microneedle-enhanced delivery, as described in Meidan VM and Michniak BB 2004 Am. J. Ther. 11(4): 312-316, can also be used for transdermal delivery of the muteins described herein. Non-parenteral delivery modes are, for instance, oral, e.g. in the form of pills, tablets, capsules, solutions or suspensions, or rectal administration, e.g. in the form of suppositories. The lipocalin mutein conjugates and pharmaceutical compositions of the invention can be administered systemically or topically in formulations containing a variety of conventional non-toxic pharmaceutically acceptable excipients or carriers, additives, and vehicles.

The dosage of the mutein applied may vary within wide limits to achieve the desired preventive effect or therapeutic response. It will, for instance, depend on the affinity of the compound for a chosen ligand as well as on the half-life of the complex between the mutein and the ligand *in vivo.* Further, the optimal dosage will depend on the biodistribution of the mutein or its fusion protein or its conjugate, the mode of administration, the severity of the disease/disorder being treated as well as the medical condition of the patient. For example, when used in an ointment for topical applications, a high concentration of the lipocalin mutein can be used. However, if wanted, the lipocalin mutein conjugate of the invention may also be given in a sustained release formulation, for example liposomal dispersions or hydrogel-based polymer microspheres, like PolyActive™ or OctoDEX™ (cf. Bos et al., Business Briefing: Pharmatech 2003: 1-6). Other sustained release formulations available are, for example, PLA-PEG based hydrogels (Medincell) and PEA based polymers (Medivas).

Accordingly, the muteins of the present invention can be formulated into compositions using pharmaceutically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA). To prepare the formulations or compositions of the invention, pharmaceutically inert inorganic or organic excipients can be used. To prepare e.g. pills, powders, gelatin capsules or suppositories, for example, lactose, talc, stearic acid and its salts, fats, waxes, solid or liquid polyols, natural and hardened oils can be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils.

The formulations or composition may also contain additives, such as, for example, fillers, binders, wetting agents, glidants, stabilizers, preservatives, emulsifiers, and furthermore solvents or solubilizers.

The formulations can be sterilized by numerous means, including filtration through a bacteria-retaining filter, or by incorporating sterilizing agents in the form of sterile solid compositions which can be dissolved or dispersed in sterile water or other sterile medium just prior to use.

Further is described herein a method of treating a disease or disorder, comprising administering a lipocalin mutein conjugate, pharmaceutical formulation or controlled release composition of the invention as defined above to a subject in need thereof.

The subject in need of such a treatment may be a mammal, such as a human, a dog, a mouse, a rat, a pig, an ape such as cymologous to name only a few illustrative examples.

The precise nature of the diseases and disorders that are to be treated according to the method described above depends on the ligand that the utilized mutein is intended to bind. It is described herein that the muteins of the present invention can be used to treat any disease as long as a target molecule that is known to be involved in the development of the disease or disorder can be displayed to the expression product of a nucleic acid library or displayed to otherwise obtain muteins of a lipocalin.

Further is described herein: The lipocalin mutein used in conjugates, and compositions is a human tear lipocalin mutein binding VEGF with high affinity. The lipocalin mutein has the amino acid sequence set forth in SEQ ID NO:22. It is described herein that this lipocalin mutein is conjugated to a hydrophilic polymer, for example, polyethylene glycol, preferably of a molecular weight of approximately 20 kDa, and, optionally, formulated with a biodegradable polymer, the biodegradable polymer being a poly(D,L-lactide-co-glycolide) polymer.As is further described, the formulation is in form of a microparticle or nanoparticle.

This conjugate, formulation or composition may be used in a method for the treatment of a disease or disorder as described above, for example a VEGF-related disease or disorder. The disease or disorder, such as a VEGF-related disease or disorder, is connected to an increased vascularization such as cancer, asthma, arthritis, including osteoarthritis and rheumatoid arthritis, inflammation, chronic obstructive pulmonary disease (COPD), pulmonary hypertension, neovascular wet age-related macular degeneration (AMD), diabetic retinopathy or macular edema, retinopathy of prematurity or retinal vein occlusion. The cancer may be selected from the group consisting of carcinomas of the gastrointestinal tract, rectum, colon, prostate, ovaries, pancreas, breast, bladder, kidney, endometrium, and lung, leukaemia, and melanoma, to name only a few illustrative examples.

### BRIEF DESCRIPTION OF THE DRAWINGS

**Figure 1** shows an RP-HPLC chromatogram of a solution of a human tear lipocalin mutein with binding affinity for VEGF (SEQ ID NO:22).
**Figure 2** shows an RP-HPLC chromatogram of a solution of a human tear lipocalin mutein with binding affinity for VEGF conjugated to a linear PEG with a molecular weight of 20 kDa (SEQ ID NO:22#2 PEG20).
**Figure 3** shows an RP-HPLC chromatogram of a solution of a human tear lipocalin mutein with binding affinity for VEGF conjugated to a branched PEG with a molecular weight of 40 kDa (SEQ ID NO:22#2 PEG40).
**Figure 4** shows an RP-HPLC chromatogram of a solution of a human tear lipocalin mutein with binding affinity for VEGF conjugated to a linear PEG with a molecular weight of 5 kDa (SEQ ID NO:22#2 PEG05).
**Figure 5** shows an RP-HPLC chromatogram of a solution of a human tear lipocalin mutein with binding affinity for VEGF conjugated to a linear PEG with a molecular weight of 10 kDa (SEQ ID NO:22#2 PEG10).
**Figure 6** shows an RP-HPLC chromatogram of a human tear lipocalin mutein with binding affinity for VEGF conjugated to a linear PEG with a molecular weight of 5 kDa (SEQ ID NO:22#2 PEG05) extracted from a microsphere particle.
**Figure 7** shows an RP-HPLC chromatogram of a human tear lipocalin mutein with binding affinity for VEGF conjugated to a linear PEG with a molecular weight of 10 kDa (SEQ ID NO:22#2 PEG10) extracted from a microsphere particle.
**Figure 8** shows an RP-HPLC chromatogram of a human tear lipocalin mutein with binding affinity for VEGF conjugated to a linear PEG with a molecular weight of 20 kDa (SEQ ID NO:22#2 PEG20) extracted from a microsphere particle.
**Figure 9** shows an RP-HPLC chromatogram of a human tear lipocalin mutein with binding affinity for VEGF conjugated to a branched PEG with a molecular weight of 40 kDa (SEQ ID NO:22#2 PEG40) extracted from a microsphere particle.
**Figure 10** shows an RP-HPLC chromatogram of a human tear lipocalin mutein with binding affinity for VEGF conjugated to a linear PEG with a molecular weight of 20 kDa (SEQ ID NO:22#2 PEG20) released from a microsphere particle over 24 hours.
**Figure 11** shows an RP-HPLC chromatogram of a human tear lipocalin mutein with binding affinity for VEGF conjugated to a branched PEG with a molecular weight of 40 kDa (SEQ ID NO:22#2 PEG40) released from a microsphere particle over 24 hours.
**Figure 12** shows the absolute amounts of lipocalin mutein released over a period of three weeks for a microsphere formulation in vitro.
**Figure 13** shows the daily release of lipocalin mutein over a period of three weeks for a microsphere formulation in vitro.

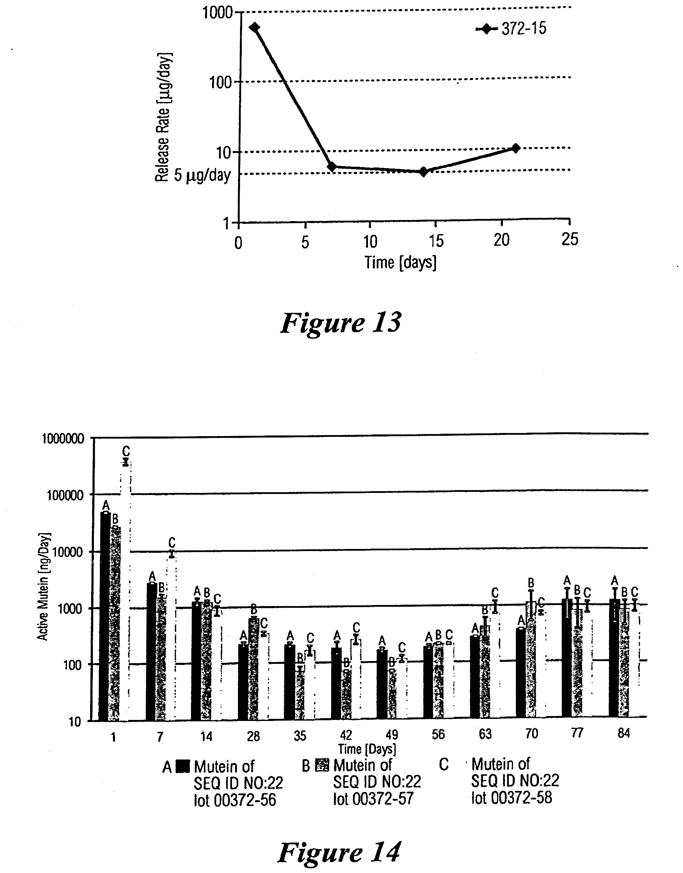

**Figure 14** shows the calculated daily in vitro release of active, VEGF-binding human tear lipocalin mutein with binding affinity for VEGF conjugated to a linear PEG with a molecular weight of 20 kDa (SEQ ID NO:22#2 PEG20) from three controlled release formulations, lots 372-56, 372-57 and 372-58. The amount of active lipocalin mutein of the mutein SEQ ID NO:22#2 PEG20 (in ng/day) released from the formulation of lot 372-56 is depicted in dark grey bars, the amount of active lipocalin mutein released from the formulation of lot 372-57 is depicted in medium grey bars and the amount of active lipocalin mutein released from formulation of lot 372-58 is depicted in light gray bars.

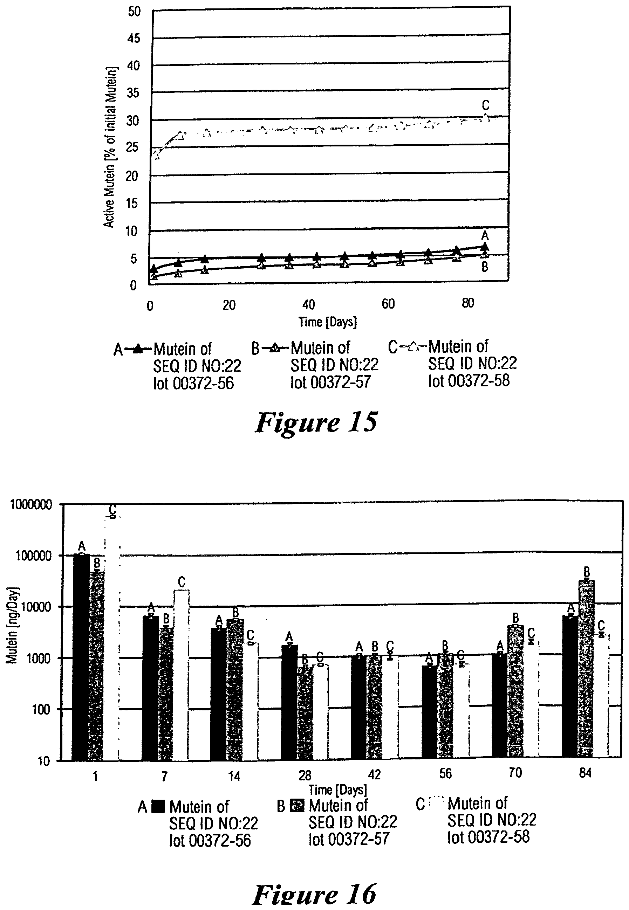

**Figure 15** shows the cumulative in vitro release of active, VEGF-binding human tear lipocalin mutein with binding affinity for VEGF conjugated to a linear PEG with a molecular weight of 20 kDa (SEQ ID NO:22#2 PEG20) from the three controlled release formulations of lots 372-56, 372-57 and 372-58. The amount of active lipocalin mutein (with respect to the initial amount of the mutein) of the mutein SEQ ID NO:22#2 PEG20 (in percentage) released from the formulation of lot 372-56 is depicted by dark grey triangles, the amount of active lipocalin mutein (in percentage) released from the formulation of lot 372-57 is depicted by medium grey triangles and the amount of active lipocalin mutein (in percentage) released from the formulation of lot 372-58 is depicted by light gray triangles.
**Figure 16** shows the calculated daily in vitro release of total human tear lipocalin mutein with binding affinity for VEGF conjugated to a linear PEG with a molecular weight of 20 kDa (SEQ ID NO:22#2 PEG20) from the three controlled release formulations lots 372-56, 372-57 and 372-58. As in Fig. 14, the amount of active lipocalin mutein of the mutein SEQ ID NO:22#2 PEG20 (in ng/day) released from the formulation of lot 372-56 is depicted in dark grey bars, the amount of active lipocalin mutein released from the formulation of lot 372-57 is depicted in medium grey bars and the amount of active lipocalin mutein released from formulation of lot 372-58 is depicted in light gray bars.

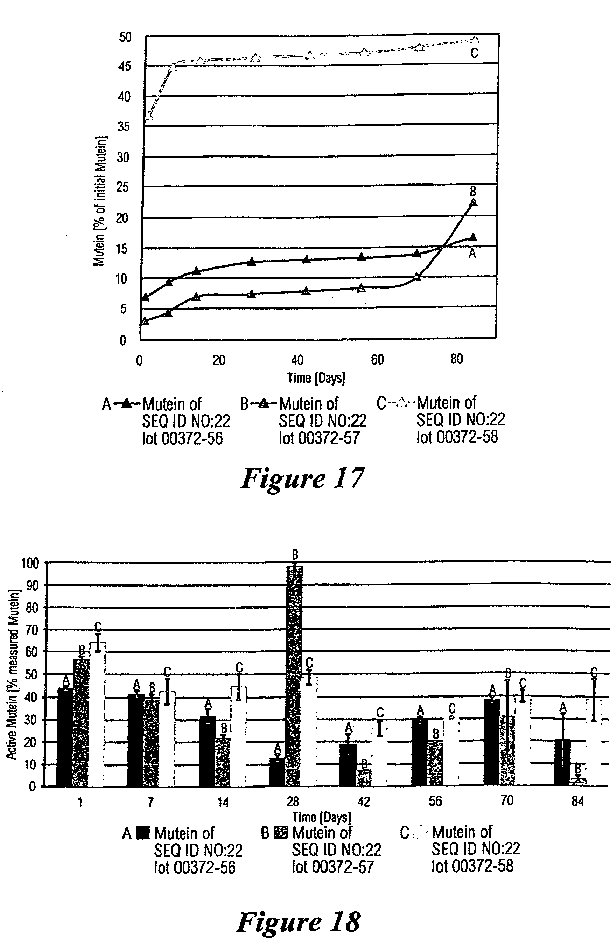

**Figure 17** shows the cumulative *in vitro* release of total human tear lipocalin mutein with binding affinity for VEGF conjugated to a linear PEG with a molecular weight of 20 kDa (SEQ ID NO:22#2 PEG20) from the three controlled release formulations lots 372-56, 372-57 and 372-58. As in Fig. 15, the amount of active lipocalin mutein (with respect to the initial amount of the mutein) of the mutein SEQ ID NO:22#2 PEG20 (in percentage) released from the formulation of lot 372-56 is depicted by dark grey triangles, the amount of active lipocalin mutein (in percentage) released from the formulation of lot 372-57 is depicted by medium grey triangles and the amount of active lipocalin mutein (in percentage) released from the formulation of lot 372-58 is depicted by light gray triangles.
**Figure 18** shows the percentage of released active, VEGF-binding human tear lipocalin mutein with binding affinity for VEGF conjugated to a linear PEG with a molecular weight of 20 kDa (SEQ ID NO:22#2 PEG20) relative to total released lipocalin mutein over 84 days from the controlled release formulations lots 372-56, 372-57 and 372-58. The same symbols as in Fig. 14 and 16 are used to depict the amount of lipocalin mutein released from the controlled release formulations of lots 372-56, 372-57 and 372-58.
**Figure 19** shows plasma levels of total human tear lipocalin mutein with binding affinity for VEGF conjugated to a linear PEG with a molecular weight of 20 kDa (SEQ ID NO:22#2 PEG20) released from the three controlled release formulations controlled release formulations lots 372-56, 372-57 and 372-58. The same symbols as in Fig. 14 and 16 are used to depict the plasma level (ng/ml) of lipocalin mutein released from the controlled release formulations of lots 372-56, 372-57 and 372-58.

### EXAMPLES

Unless otherwise indicated, established methods of recombinant gene technology were used, for example, as described in Sambrook et al. (*supra*).

### Example 1: Preparation of a PEGylated mutein of human tear lipocalin (not according to the invention)

The human tear lipocalin mutein of SEQ ID NO:22 with nanomolar binding affinity for human VEGF was conjugated to polyethylene glycol polymers (PEGs).

An unpaired cysteine residue was introduced instead of the amino acid Asp at position 91 of the mutein of SEQ ID NO:22 by point mutation in order to provide a reactive group for coupling with activated PEG. The recombinant mutein carrying the free Cys residue was subsequently produced in E. coli as previously described (see, for example, PCT publication WO 2008/015239).

Tris (2-carboxyethyl) phosphine hydrochloride (TCEP) was used as reducing agent to obtain free Cysteine 91 residues of SEQ ID NO:22 for modification with PEG. The TCEP concentration is a critical factor, because an excess of TCEP can lead to the reduction of the intramolecular disulfide bond of PRS-055. The presence of this reduced mutein of SEQ ID NO:22 species can be detected by RP HPLC and by ion exchange HPLC. All sulfhydryl groups of the Cysteine can be PEGylated if the disulfid bond is opened, leading to a mixture of the wanted PEG-SEQ ID NO:22 species with misPEGylated and oligoPEGylated mutein of SEQ ID NO:22. If the TCEP concentration is too low, the activation of the mutein of SEQ ID NO:22 is incomplete, leading to a reduction of the PEGylation yield. Activation is carried out by mixing the solution containing the mutein of SEQ ID NO:22 with TCEP solution. The activation batches are incubated at room temperature with gentle mixing for about 2 to 3 h.

PEGylation is carried out by mixing the activation batch with maleimide coupled PEG (average molecular weight 2, 5, 10, 20 kDa, linear carbon chain; or average molecular weight 40 kDa, branched carbon chain (e.g. PEG40 Sunbright GL2-400MA01; NOF, Singapore)). The PEG is added promptly after the end of the activation period. The amount of PEG is calculated on basis of the amount of the mutein of SEQ ID NO:22. The target molar ratio of the mutein of SEQ ID NO:22 to PEG is 1:2.

After PEGylation one further ion exchange chromatography step (Macro cap) is performed to improve the purity of the PEGylated protein, and to remove free PEG and non-reacted mutein of SEQ ID NO:22.

The products thus obtained were termed SEQ ID NO:22#1 (SEQ ID NO:22; unconjugated mutein), SEQ ID NO:22#002 PEG05 (mutein conjugated to linear PEG with a molecular weight of 5 kDa), SEQ ID NO:22#002 PEG 10 (mutein conjugated to linear PEG with a molecular weight of 10 kDa), SEQ ID NO:22#002 PEG20 (mutein conjugated to linear PEG with a molecular weight of 20 kDa), and SEQ ID NO:22#002 PEG40 (mutein conjugated to branched PEG with a molecular weight of 40 kDa).

### Example 2: Lyophilization of the mutein of SEQ ID NO:22 and PEG conjugates thereof (not according to the invention)

Before attempting to dissolve the mutein of SEQ ID NO:22 in organic solvents, it was necessary to remove the salts from the protein solution and lyophilize the material. Dialysis was selected as the method to remove the salts. The protein was dialyzed in volatile buffer containing ammonium bicarbonate to produce a relatively salt free powder after lyophilization.

A known volume of drug stock (300 µL ofPRS-055, 2 mL SEQ ID NO:22#002 PEG20, 3 mL of SEQ ID NO:22 #002 PEG40) was diluted with 0.02% ammonium bicarbonate to a volume of 10 mL. The diluted solution was transferred into dialysis tubing (SpectraPor 3500 MWCO) and dialyzed against a volume of 3 L of 0.02% ammonium bicarbonate at 4 °C while stirring at 105 rpm. The volume of 0.02% ammonium bicarbonate was emptied and replaced with fresh buffer twice at three-hour intervals. After the third replacement the dialysis was allowed to continue overnight. The solutions were then transferred to 50 mL Falcon tubes and placed in a -80 °C freezer. Once the solutions were frozen the tubes were lyophilized for approximately 72 hours.

After confirmation of activity, the remaining drug solutions of SEQ ID NO:22#002 PEG20 and SEQ ID NO:22#002 PEG40 were dialyzed. Approximately 5 mL of SEQ ID NO:22#002 PEG20 and 6.5 mL of SEQ ID NO:22#002 PEG40 were diluted with 0.02% ammonium bicarbonate to a volume of 20 mL and 25 mL respectively. The diluted solution was transferred into dialysis tubing and dialyzed against a volume of 4 L of 0.02% ammonium bicarbonate at 4 °C while stirring at 105 rpm. The volume of 0.02% ammonium bicarbonate was emptied and replaced twice with fresh buffer twice at two-hour intervals. The solutions were then transferred to 50 mL Falcon tubes and placed in a -80 °C freezer. Once the solutions were frozen the tubes were lyophilized for approximately 45 hours.

Approximately 25 mL of SEQ ID NO:22#002 PEG05 and 40 mL of SEQ ID NO:22#002 PEG10 were diluted with 0.02% ammonium bicarbonate to a volume of 50 mL and 40 mL respectively. The diluted solution was transferred into dialysis tubing and dialyzed against a volume of 4 L of 0.02% ammonium bicarbonate at 4 °C stirring at 105 rpm. The volume of 0.02% ammonium bicarbonate was emptied and replaced twice with fresh buffer twice at two-hour intervals. The solutions were then transferred to 50 mL Falcon tubes and placed in a -80 °C freezer. Once the solutions were frozen the tubes were lyophilized for approximately 45 hours. A portion of SEQ ID NO:22#002 PEG05 and SEQ ID NO:22#002 PEG10 was re-dialyzed, 32.5 mg of SEQ ID NO:22-PEG05 and 31.6 mg SEQ ID NO:22-PEG10 were dissolved in approximately 37.5 mL and 35 mL of 0.02% ammonium bicarbonate respectively. The solutions were put in dialysis tubing and 4 L of 0.02% ammonium bicarbonate and left at 4°C for 16 hours then the ammonium bicarbonate was replaced with fresh. After two hours the samples were collected in multiple 50 mL falcon tubes, frozen, and lyophilized for approximately 48 hours.

### Example 3: Solubility of the mutein of SEQ ID NO:22 and PEG conjugates thereof (not according to the invention)

The solubility of SEQ ID NO:22 and PEG conjugates thereof in organic solvents was determined. A small amount of either SEQ ID NO:22 or the respective conjugate was accurately weighed into a 4 or 6 mL glass vial. Selected organic solvents were added in small increments by volume (micropipetter). The vial was rotated, and then was observed for visual clarity. Subsequent volumes of solvent were added until a clear solution was obtained.

### Example 4: RP-HPLC Analysis of Conjugates (not according to the invention)

Solutions of three different compounds: SEQ ID NO:22, SEQ ID NO:22#002-PEG20, and SEQ ID NO:22#002-PEG40 were diluted in PBS so that the concentration of the solution was ∼0.1 mg/mL. These solutions were analyzed by reverse phase HPLC (RP-HPLC). The reverse phase method was implemented on an HP/Agilent system, using a Waters xBridge BEH300 C18 3.5µm, (4.6x250mm) column. A gradient method was used, advancing at 1 ml/min at 30°C (Table 1). A 15 µl injection volume was used.

**Table 1: Reverse Phase Gradient Method**

| Time (minutes) | Mobile Phase A (Water w/0.06% TFA) | Mobile Phase B (ACN w/0.06% TFA) |
|---|---|---|
| 0 | 63% | 37% |
| 25 | 60% | 40% |
| 50 | 53% | 47% |
| 60 | 63% | 37% |

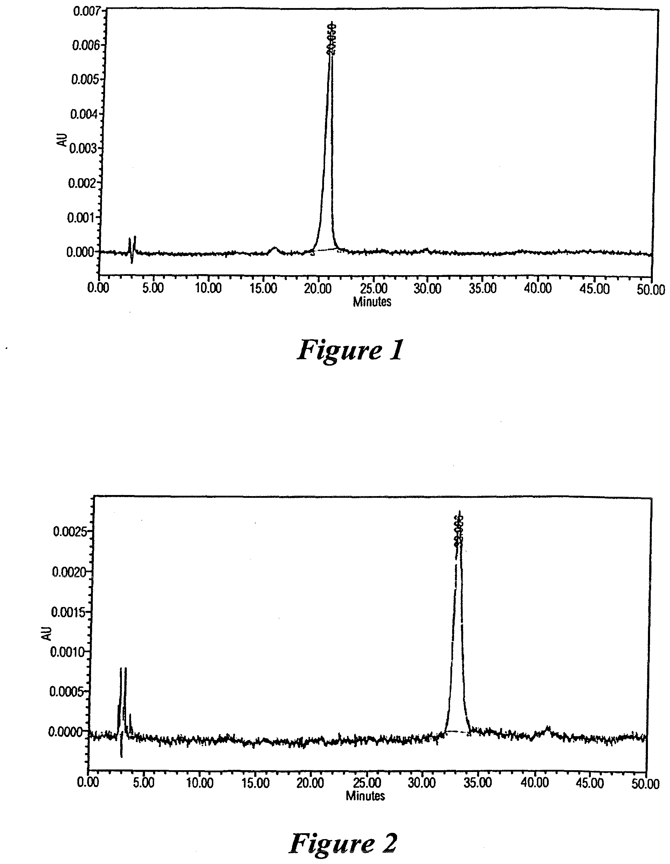

Table 2 contains the results obtained. The RP-HPLC concentrations are based on the mutein SEQ ID NO:22 as a standard to determine lipocalin mutein content and molecular weights were used to determine PEGylated analog concentrations. Chromatograms for injected solutions of each compound are shown in Figures 1, 2 and 3.

**Table 2: Concentration of samples by RP-HPLC**

| | **Concentration (mg/mL) ^{@ 280 nm}** | |
|---|---|---|
| **Compound** | **RP-HPLC** | **Label (diluted)** |
| SEQ ID NO:22 | 0.094 | 0.0995 |
| SEQ ID NO:22#002 PEG20 | 0.098 | 0.0950 |
| SEQ ID NO:22#002 PEG40 | 0.094 | 0.1014 |

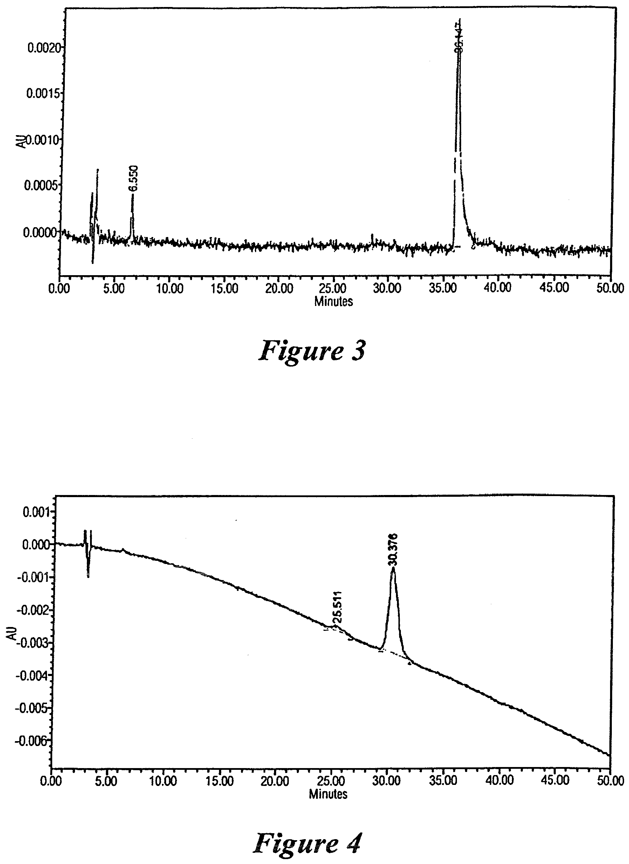

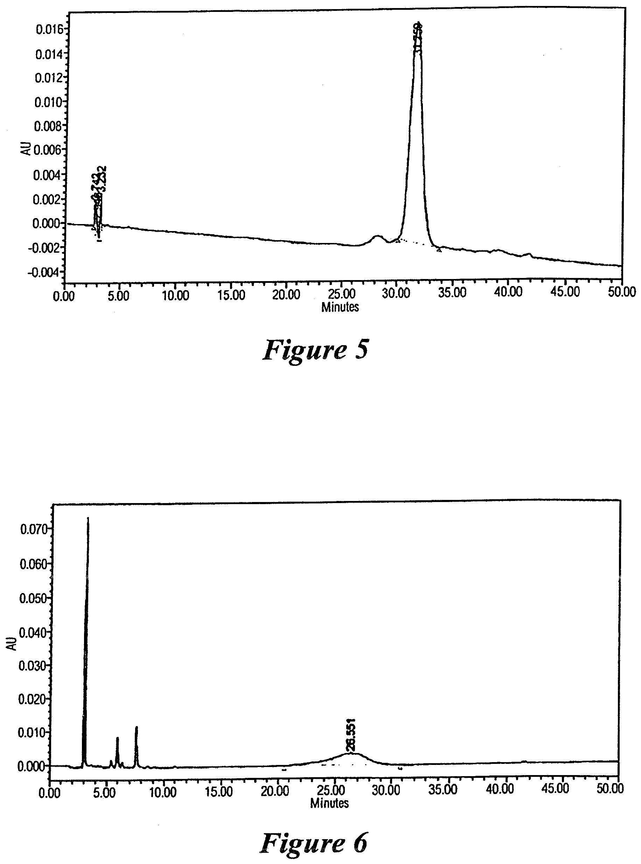

Chromatograms of SEQ ID NO:22#002 PEG05 and SEQ ID NO:22#002 PEG10 were obtained after lyophilization and resuspension in PBS (Figures 4 and 5).

### Example 5: Solubility of SEQ ID NO:22 and PEG conjugates thereof in organic solvents (not according to the invention)

Preparation of microsphere formulations requires that the drug and the polymer (PLGA) be co-dissolved in an organic solvent. Initial testing was done with SEQ ID NO:22 conjugates in dichloromethane (DCM). Since the solubility of SEQ ID NO:22 and conjugates in this solvent is poor, additional solubility testing was performed with SEQ ID NO:22 and conjugates in dimethyl sulfoxide (DMSO), DCM with benzyl alcohol (BnOH), DCM/acetic acid (AcOH), DCM/methanol (MeOH), ethyl acetate (EtOAc), EtOAc/AcOH, and a solvent system of DCM, MeOH, AcOH, and H₂O.

PLGA polymer was added to some of these solutions to determine if solubility of the drug changed in the presence of polymer. Polymer was added such that the resulting weight/weight ratio of the PEGylated drug/polymer was 15/85. In some cases the addition of polymer resulted in a cloudy solution, so more solvent was added until the mixture became clear.

The solubility of the SEQ ID NO:22#001 was poor (<3 mg/mL) in all solvents tested (Table 3).

**Table 3: SEQ ID NO:22#001 Solubility**

| **Solvent** | **Solubility (mg/mL)** |
|---|---|
| DCM | <3 |
| DCM/Polymer | <3 |
| 1:22.5 DCM: DMSO | <1 |
| 1:18 DCM: BnOH | <1 |
| DMSO | <1 |
| 79% DCM, 12.8% MeOH, 7.9% AcOH, 0.3% diH₂O | <2 |

Both SEQ ID NO:22#002 PEG20 and SEQ ID NO:22#002 PEG40 had the highest solubility in a solvent system consisting of DCM/MeOH/AcOH/H₂O (Tables 4 and 5).

**Table 4: SEQ ID NO:22#002 PEG20 Solubility**

| **Solvent** | **Solubility (mg/mL)** |
|---|---|
| DCM | <5 |
| DCM/Polymer | <5 |
| DMSO | <2 |
| 1:1 DCM: DMSO | 2 |
| 1:1 DCM: DMSO, Polymer | 2 |
| 1:1.5 DCM: BnOH | 2 |
| 1:1.5 DCM: BnOH, Polymer | 2 |
| 80% DCM, 20% AcOH | <10 |
| MeOH | <6 |
| 78% MeOH, 22% AcOH | 5 |
| 79% DCM, 12.8% MeOH, 7.9% AcOH, 0.3% diH2O | 8 |
| 79% DCM, 12.8% MeOH, 7.9% AcOH, 0.3% diH2O; Polymer | 7 |
| 56.6% DCM, 28.9% MeOH, 14.5% AcOH | 7 |
| 56.6% DCM, 28.9% MeOH, 14.5% AcOH, Polymer | 7 |

**Table 5: SEQ ID NO:22#002 PEG40 Solubility**

| **Solvent** | **Solubility (mg/mL)** |
|---|---|
| DCM | <5 |
| DCM/Polymer | <5 |
| 1:0.5 DCM: DMSO | 3 |
| 1:0.6 DCM: BnOH | 3 |
| 1:0.6 DCM: BnOH, polymer | 3 |
| DMSO | <3 |
| 1:0.3 DCM: DMSO | 3 |
| 77% DCM, 23% AcOH | <12 |
| MeOH | 17 |
| 79% DCM, 12.8% MeOH, 7.9% AcOH, 0.3% diH2O | 18 |
| 79% DCM, 12.8% MeOH, 7.9% AcOH, 0.3% diH2O; Polymer | 7 |
| 51.8% DCM, 32.5% MeOH, 15.7% AcOH | 8 |
| 49.4% DCM, 29.4% MeOH. 21.2% AcOH, Polymer | 7 |
| Ethyl Acetate (EtOAc) | <4 |
| 68.7% EtOAc, 31.3% AcOH | 2.5 |
| 68.7% EtOAc, 31.3% AcOH, Polymer | 2.5 |

When polymer was added to the SEQ ID NO:22#002 PEG20 solution the solubility remained about the same. When polymer was added to the SEQ ID NO:22#002 PEG40 solution, the solution clouded and had to be diluted by almost two times for the drug to become soluble. The solubility of the conjugates in simpler solvent systems of DCM/AcOH or DCM/MeOH was not any better than that of DCM/MeOH/AcOH/H₂O.

SEQ ID NO:22#002 PEG05 and SEQ ID NO:22#002 PEG10 were also tested in the solvent system containing DCM, MeOH, AcOH, and H₂O. The solubility of these conjugates is compared to the higher molecular weight conjugates in Table 6.

**Table 6: Solubility comparison of SEQ ID NO:22 conjugates in 79% DCM, 12.8% MeOH, 7.9% AcOH, 0.3% diH₂O and PLGA polymers**

| **SEQ ID NO:22 -conjugate** | **Solubility (mg/mL)** | **Calculated Concentration of SEQ ID NO:22 (mg/mL)** |
|---|---|---|
| SEQ ID NO:22#002-PEG05 | 4 | 3.1 |
| SEQ ID NO:22#002-PEG10 | 0.8 | 0.5 |
| SEQ ID NO:22#002-PEG20 | 7 | 3.3 |
| SEQ ID NO:22#002-PEG40 | 7 | 2.1 |

The SEQ ID NO:22#002-PEG05 and SEQ ID NO:22#002 PEG20 conjugates have the highest solubility (protein basis) in this solvent mixture with the PLGA polymer.

### Example 6: Compatibiliy of conjugates of the mutein of SEQ ID NO:22 with microsphere production technology (not according to the invention)

To determine if the conjugates of the mutein of SEQ ID NO:22 are compatible with microsphere-forming technology samples were prepared which were put through a microsphere process. Samples for activity analysis were then prepared by either extracting the conjugates out of the microspheres, or by collecting conjugate released from the microspheres into PBS.

Each of the conjugates of the mutein of SEQ ID NO:22 were dissolved in a solvent system consisting of 79% DCM, 12.8% MeOH, 7.9% AcOH, 0.3% H₂O and polymer. A clear homogenous oil phase solution was obtained, which was used to make microspheres. An emulsion was made by combining the oil phase with a water phase consisting of 1 % polyvinyl alcohol (PVA) and 1% DCM. The emulsion was collected in 0.3% PVA and the DCM was allowed to evaporate in order to harden the microparticles. The hardened particles were then collected by filtration, washed with water and lyophilized.

Once the particles were dry, the drug content was determined by dissolving a sample of the microspheres in acetonitrile (ACN) and precipitating the polymer with 0.1% TFA in water. The solution containing the extracted drug was centrifuged and the supernatant was analyzed by RP-HPLC (Figures 6-9).

The chromatograms of SEQ ID NO:22#002 PEG05 and SEQ ID NO:22#002 PEG10 are broad with shifted retention times, suggesting that they have been altered in some way. The chromatograms of SEQ ID NO:22#002 PEG20 and SEQ ID NO:22#002 PEG40 extracted from microspheres contain extraneous peaks that are tentatively ascribed to partial loss of one branch of the PEG structure (see Guiotto, A. et al. (2004) Bioorg. Med. Chem. 12, 5031-5037). The elution time of the new peak for the 40kDa PEG conjugate (31.8 min) is similar to that of the intact 20kDa PEG SEQ ID NO:22 (e.g. 32.8 min) as would be expected if the extraneous peak has 20kDa of PEG still attached.

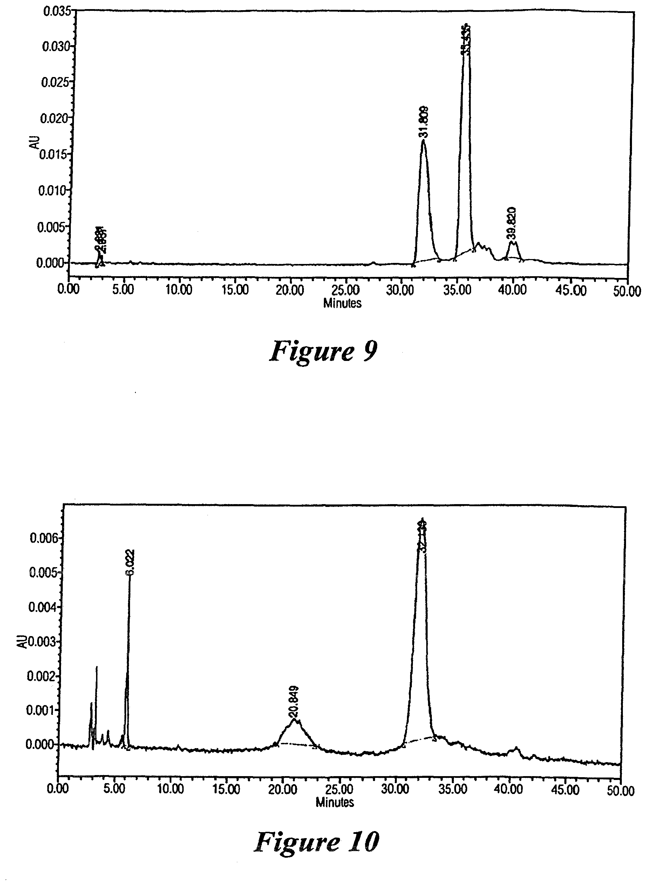

The samples of the conjugates that were extracted from the microspheres were exposed not only to the conditions used in the manufacturing of microspheres, but also to the harsh conditions used to extract the protein from the microsphere. To obtain more representative samples that had been exposed to the microsphere process, a sample either the microspheres were suspended in 1.5 mL of PBS in a microcentrifuge tube and placed on a rotisserie in an incubation cabinet at 37°C. After 24 hours the microcentrifuge tube was centrifuged at 3000 rpm for 3 minutes. The resulting supernatant containing the released conjugate was analyzed by RP-HPLC (Figures 10-11). Only chromatograms for SEQ ID NO:22#002 PEG20 and SEQ ID NO:22#002 PEG40 are shown here, because the SEQ ID NO:22#002 PEG05 and SEQ ID NO:22#002 PEG10 did not have detectable levels of drug released at 24 hours.

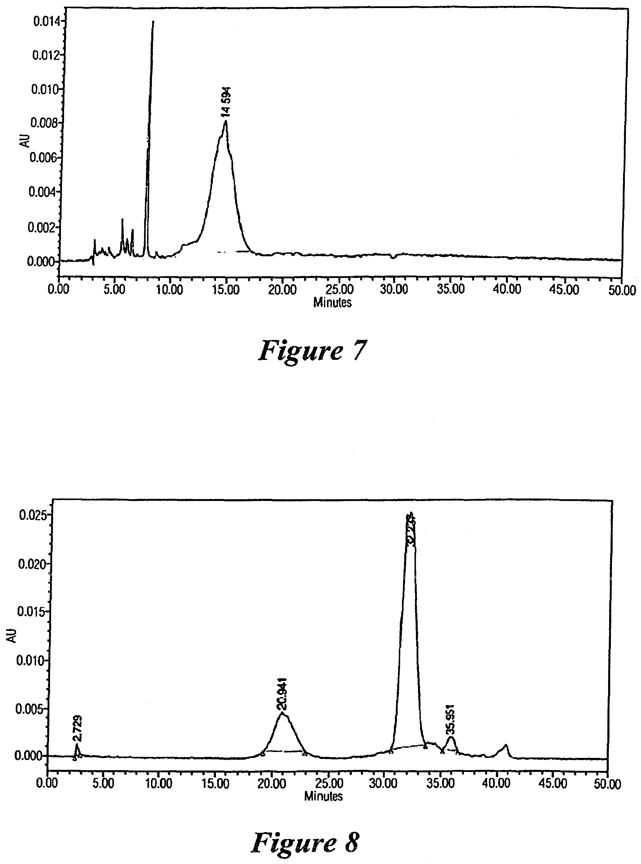

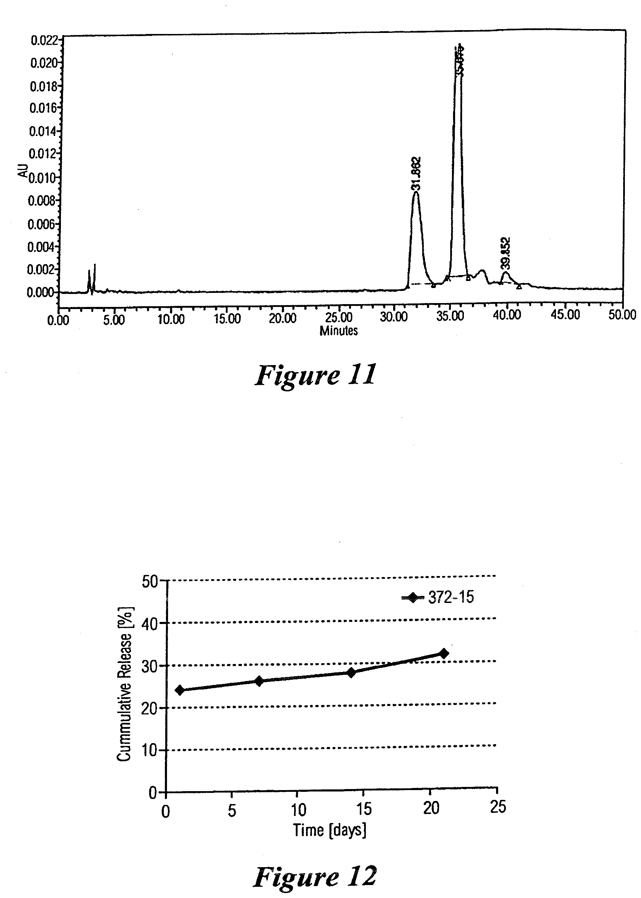

The chromatograms for the PEGylated lipocalin muteins released from microspheres (Figures 10 and 11) are similar to those for the extracted protein samples (Figures 8 and 9).

The drug content and initial 24 hr release of the drug from the microspheres was determined. The results are shown in Table 7 and were determined by using the area of all protein related peaks.

**Table 7: Drug content and initial release of SEQ ID NO:22 microspheres**

| | % Target Drug Content (mass lipocalin/mass polymer) (w/w) | Drug Content (mass lipocalin/mass polymer) (w/w) | % SEQ ID NO:22 (mass lipocalin/mass polymer) Content (w/w) | % Initial Release (mass lipocalin/mass polymer) (w/w) |
|---|---|---|---|---|
| SEQ ID NO:22#002 PEG05 | 4.8% | 2.7% | 2.1% | ND |
| SEQ ID NO:22#002 PEG10 | 6.8% | 4.6% | 2.9% | ND |
| SEQ ID NO:22#002 PEG20 | 10.0% | 9.3% | 4.3% | 21.4% |
| SEQ ID NO:22#002 PEG40 | 15.0% | 14.4% | 4.3% | 56.8% |

| | | | | |
|---|---|---|---|---|
| ND= none detected | | | | |

### Example 7: Encapsulation of conjugates of the mutein of SEQ ID NO:22 in PLG microspheres (not according to the invention)

The desired product profile was a biodegradable PLG microsphere suspension delivered through no larger than a 25 gauge needle with a volume not to exceed 100 µL providing SEQ ID NO:22 sustained release in the range of 1-5µg/day for at least 3 months.

Microspheres having a mean diameter less than 50 µm are injectable through 25 gauge needles.

The percentage of microspheres suspended in injection vehicle that has been shown injectable through a 25 gauge needle ranges from 10% to 30% depending on design of injection vehicle. Using an average of 20% microspheres in injection vehicle, for a 100 µL injection, 20 mg of microspheres would be delivered.

SEQ ID NO:22-PEG20 was encapsulated up to 13.5% by weight of PLG (poly(lactide-co-glycolide)) polymer in microspheres. Assuming 20 mg of microspheres are injected, 2.7 mg of active drug would be administered per injection. Dividing the total mass by the number of days in 3 months (90 days), a maximal delivery rate of 30µg/day can be sustained. This delivery rate can be reduced by changing injected mass of microspheres. With current parameters, duration at the specified delivery rate can be achieved.

### Example 8: In vitro release studies (not according to the invention)

Using 300mg of PEGylated SEQ ID NO:22 (SEQ ID NO:22#002 PEG20) a lot of microspheres was prepared to perform *in vitro* release studies.

SEQ ID NO:22 PEG20 content, particle size analysis and residual solvent (by TGA) were assessed (Table 8).

**Table 8: Summary data for the lot of microspheres**

| Formulation | Content % (mass lipocalin/mass polymer) | Residual Solvent | Mean Particle Diameter |
|---|---|---|---|
| 372-15 | 7% | 0.15% | 36.07 µm |

Content was assessed by both SEC and RP-HPLC. These measured values were consistent between analytical techniques, and measured values were lower than previously observed.

*In vitro* release dissolution was performed on SEQ ID NO:22-PEG20 loaded microspheres by monitoring amounts of active drug in sink condition buffer at 37°C. Briefly, approximately 10 mg of microspheres, exact mass recorded, was weighed into 14 mL polypropylene test tubes. A volume of 4 mL phosphate buffered saline (PBS) (1x PBS, sterile filtered, HyClone) was added to each tube. In place of centrifugation, serum separating filters were used.

*In vitro* release assemblies, tightly capped, were placed horizontally in a Fisher water filled shaker bath at 37°C operating at 120 rpm. At recorded intervals, supernatant was separated from microspheres using a 16 x 100 mm serum separator filter (Fisher, cat. no. 02-681-52). Nearly all 4 mL of supernatant was removed using a transfer pipette, with a portion of the volume transferred directly to HPLC glass vials. Excess supernatant was discarded. Collected samples in HPLC vials were refrigerated until analyzed by HPLC, within 48 hours. A 4 mL volume of new PBS was added directly to the polypropylene tube, and tubes were tightly recapped. Tubes were returned to the shaker bath. The results from these experiments are depicted in Figures 12 and 13.

Initial burst was significantly greater than previously observed. After 7 days the formulation continued to release from days 7 to 14 with the approximate mass release rate being nearly 5 µg/day. The range of percent release was from 0.28% per day to 0.42% per day.

Release increased from week 2 to 3 with a rate increase to 10 µg/day. The potential that as the polymer degrades, the rate could rapidly increase can be addressed by formulation approaches such as including a mixture of an ester endgroup polymer with the acid endgroup.

### Example 9: In vitro release of three different microparticle formulations at 37°C in 50% rat plasma (not according to the invention)

Using 300 mg of PEGylated SEQ ID NO:22 (SEQ ID NO:22#002 PEG20) each, three lots of microspheres was prepared to perform *in vitro* and *in vivo* release studies after having optimized the protein lyphylization cycle for high solubility in the designed solvent system described in Example 6 (organic phase consisting of 79 % DCM, 12.8% MeOH, 7.9% AcOH, 0.3% H₂O and polymer, aqueous phase water phase consisting of 1% polyvinyl alcohol (PVA) and 1% DCM).

SEQ ID NO:22 PEG20 content, 24 hour burst release, residual solvent (by TGA) particle size and particle size distribution were assessed (Table 9).

**Table 9: Summary data for three lots of microspheres**

| Formulation | Content % (mass lipocalin/mass polymer) | 24h burst | Residual solvent | Mean particle size [µm] | Particle size distribution, 10% smaller/90% smaller [µm] |
|---|---|---|---|---|---|
| 372-56 | 8.5% | Low | 0.42% | 46.8 | 33.7/61.5 |
| 372-57 | 8.5% | Low | 0.51% | 39.4 | 29.2/50.4 |
| 372-58 | 7.8% | Low | 0.22% | 49.1 | 32.9/58.9 |

Microparticle from the three different particle lots 372-56, 372-57 and 372-58 containing approximately 1.6 mg human tear lipocalin mutein with binding affinity for VEGF conjugated to a linear PEG with a molecular weight of 20 kDa (SEQ ID NO:22#2 PEG20) (400µl, 5% solution, ∼8% loading) were incubated at 37°C in 50% rat Li-heparin plasma using an overhead stirrer at 40 rpm. The amounts of binding active lipocalin mutein released into the sink over the first 84 days were analyzed by quantitative ECL ELISA. For the lots 372-56 and 372-58 a poly(DL-lactide-glycolide) polymer with 75 mole % DL lactide monomer and 25 mole % glycolide with terminal acid groups and an inherent viscosity of 0.45 to 0.55 Dl/g was used. In addition, palmitic acid was used as pore forming agent for lot #371-58. For the lot 372-57 a poly(DL-lactide-glycolide) polymer with 65 mole % DL lactide monomer and 35 mole % glycolide with terminal acid groups and an inherent viscosity of 0.4 to 0.5 Dl/g was used (the viscosity were measured at 0.5% w/v in CHCl₃ at 30 °C, with a size 25 Cannon-Fenske glass capillary viscometer).

Extended release of functionally active lipocalin mutein could be observed from all three formulations. An initial release is followed by a low plateau (day 28-56) and a higher release from day 63 onward (Figures 14 and 15). The diffusion controlled initial release is relatively slow and extends over several days. Inclusion of phthalic acid did increase the initial release of lot 372-58 significantly to ∼25% presumably due to an increased porosity of the microparticles. The plateau release rate of all three formulations is fairly similar.

For comparison, the release of total lipocalin mutein was also determined (Figures 16 and 17). Comparing the results for levels of release active lipocalin mutein and total released lipocalin mutein, it was determined that 40-65% of the initially released lipocalin mutein appears to be active throughout the release period (Figure 17). The ratio of active and total lipocalin mutein was most stable for one of the lots of microspheres (lot 00372-58).

### Example 10: Pharmacokinetics of three different microparticle formulations after single subcutaneous administration in female RNU rats (not according to the invention)

The aim of this experiment was to determine an extended *in vivo* release profile and correlate this with *in vitro* release by measuring the plasma levels of released lipocalin mutein over 4 months.

Day 2-78 plasma samples from nude rats dosed s.c. with 150 mg/kg microparticles solution (5%, 3 ml/kg) were analyzed by quantitative ECL ELISA measuring binding active lipocalin mutein. Active SEQ ID NO:22-PEG20 released from microspheres s.c. can be detected in the plasma and an initial release is followed by a constant low plateau phase. The initial *in vivo* release pattern up to day 43 corresponds very well with the pattern observed *in vitro.*

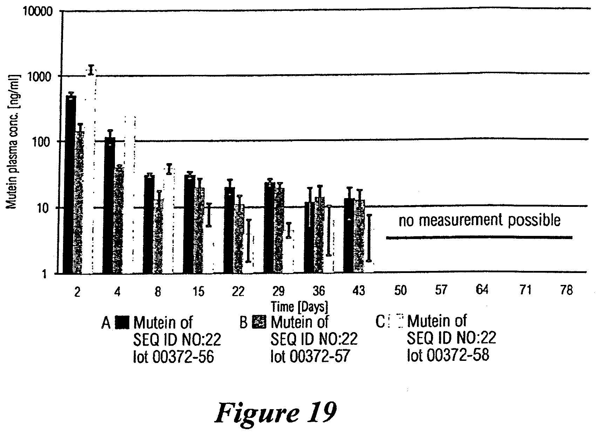

The results of this study depicted in Figure 19 show that plasma levels fall below 1 ng/ml after day 43 which indicated that the *in vivo* release might occur at a faster rate that *in vitro.* A s.c. bioavailability of 30% and clearance rate of 18 ml/h/kg has been experimentally determined for SEQ ID NO:22-PEG20 in a separate study. The in vivo release rates could be calculated as the rate of elimination equals the release rate (infusion) at steady state (Css = k0 / Cl, k0 is the rate constant for release/infusion and Cl is the clearance rate (Cl = 18 ml h-1 kg-1) and Css is the steady state plasma concentration (Css = 0.015 µg/ml at plateau)). The release rate from the SEQ ID NO:22-PEG20 microparticle depot is therefore k0 = Css * Cl = 0.015 µg/ml * 18 ml h⁻¹ kg⁻¹ = 270 ng h⁻¹ kg⁻¹ or k0 = 6.48 µg/day*kg = 1.3 µg/day*rat. A constant release rate of 26 µg/day would be expected from a 3 month depot of ∼2,4 mg (150 mg/kg, 8% loading) in a 200 g rat which is 10 fold higher than the currently observed release rate (taking also into account that the total release of the lipocalin mutein compared to the active concentration could be ∼2 fold higher).

Further is described herein:
1. A pharmaceutical formulation for controlled release of a lipocalin mutein, the formulation comprising the lipocalin mutein or a conjugate thereof in combination with a polymer, lipid or liposome.
2. The pharmaceutical formulation of item 1, wherein the liposome encapsulates the lipocalin mutein or conjugate thereof.
3. The pharmaceutical composition of item 2, wherein the liposomes are dispersed or emulgated in an aqueous base medium.
4. The pharmaceutical formulation of item 1, wherein the polymer is a biodegradable polymer.
5. The pharmaceutical formulation of item 4, wherein the biodegradable polymer is selected from the group consisting of polyhydroxy acids, polylactides, polyglycolides, poly(lactide-co-glycolide)s, polylactic acids, polyglycolic acids, poly(lactic acid-co-glycolic acid)s, polycaprolactones, polycarbonates, polyesteramides, polyanhydrides, poly(amino acids), polyorthoesters, polyacetyls, polycyanoacrylates, polyetheresters, polydioxanones, polyalkylene alkylates, copolymers of polyethylene glycol and polylactides or poly(lactide-co-glycolide)s, biodegradable polyurethanes, and certain types of protein and polysaccharide polymers, as well as blends, copolymers and derivatives thereof.
6. The pharmaceutical formulation of item 5, wherein the biodegradable polymer is selected from the group consisting of polyhydroxy acids, polylactic acids, polylactides, polyglycolides, polyglycolic acids, and copolymers thereof as well as derivatives thereof.
7. The pharmaceutical formulation of item 5 or 6, wherein the biodegradable polymer is selected from the group consisting of polyanhydrides, polyorthoesters, and polysaccharide polymers.
8. The pharmaceutical formulation of any one of items 5 to 7, wherein the biodegradable polymer is poly-(D,L-lactide-co-glycolide).
9. The pharmaceutical formulation of any one of items 5 to 8, wherein the biodegradable polymer is a polylactic acid polymer or copolymer comprising lactide units substituted with alkyl moieties.
10. The pharmaceutical formulation of item 9, wherein the biodegradable polymer comprises poly(hexyl-substituted lactide) or poly(dihexyl-substituted lactide).
11. The pharmaceutical formulation of any one of items 4 to 10, wherein the biodegradable polymer is formulated into microparticles or nanoparticles encapsulating the lipocalin mutein or conjugate thereof.
12. The pharmaceutical composition of any one of items 1 to 11, wherein the formulation comprises a conjugate of a lipocalin mutein and a moiety selected from the group consisting of a protein, protein domain, peptide, fatty acid, lipid, polysaccharide and/or organic polymer, or combinations thereof.
13. The pharmaceutical composition of item 12, wherein the lipocalin mutein and the moiety are covalently conjugated.
14. The pharmaceutical composition of item 12 or 13, wherein the moiety facilitates controlled delivery of the lipocalin mutein, extends the *in vivo* half-life of the lipocalin mutein, increases the bioavailability of the lipocalin mutein and/or decreases the immunogenicity of the lipocalin mutein.
15. The pharmaceutical composition of any one of items 12 to 14, wherein the moiety is selected from the group consisting of hydrophilic polymers, palmitic acid or other fatty acid molecules, an Fc part of an immunoglobulin, a CH₃ domain of an immunoglobulin, a CH₄ domain of an immunoglobulin, albumin or an albumin fragment, an albumin binding peptide, an albumin binding protein, ubiquitin, an ubiquitin-derived peptide, and transferrin.
16. The pharmaceutical composition of item 15, wherein the albumin binding protein is a bacterial albumin binding protein, an antibody or antibody fragment directed against albumin or a lipocalin mutein with binding activity for albumin.
17. The pharmaceutical composition of item 16, wherein the bacterial albumin domain is an albumin binding domain of streptococcal protein G.
18. The pharmaceutical composition of item 17, wherein the albumin binding peptide has the formula Cys-Xaa₁-Xaa₂-Xaa₃-Xaa₄-Cys, wherein Xaa₁ is Asp, Asn, Ser, Thr, or Trp; Xaa₂ is Asn, Gln, His, Ile, Leu, or Lys; Xaa₃ is Ala, Asp, Phe, Trp, or Tyr; and Xaa₄ is Asp, Gly, Leu, Phe, Ser, or Thr.
19. The pharmaceutical composition of item 14, wherein the moiety that extends the serum half-life is a hydrophilic polymer.
20. The pharmaceutical composition of item 19, wherein the hydrophilic polymer is selected from the group consisting ofpolyalkylene glycols, polyoxyethylated polyols, hydroxyethyl starch, polyhydroxy acids, polylactic acids, polyglycolic acids, and copolymers thereof as well as linear, branched and activated derivatives thereof.
21. The pharmaceutical composition of item 20, wherein the hydrophilic polymer is polyethylene glycol, polypropylene, polyethylene glycol/polypropylene glycol copolymers, polyoxyethylated glycerol, polyoxyethylated glucose, polyoxyethylated sorbitol, and linear, branched and activated derivatives thereof.
22. The pharmaceutical composition of item 21, wherein the activated derivative is an amino-reactive derivative selected from the group consisting of an aldehyde, an N-hydroxy succinimide, a succinimide, a maleimide, a PNP-carbonate, and a benzotrizole terminated hydrophilic polymer or a thiol-reactive derivative.
23. The pharmaceutical composition of any one of items 20 to 22, wherein the hydrophilic polymer is polyethylene glycol (PEG) or a linear, branched and activated derivative thereof.
24. The pharmaceutical composition of item 23, wherein the polyethylene glycol has a mean molecular weight of 5, 7, 10, 12, 15, 20, 25, 30, 35, 40, 45, 50, 55, 60, 65, or 70 kDa.
25. The pharmaceutical composition of any one of the forgoing items, wherein the lipocalin mutein is selected from the group consisting of muteins of retinol-binding protein (RBP), bilin-binding protein (BBP), apolipoprotein D (APO D), neutrophil gelatinase associated lipocalin (NGAL), tear lipocalin (TLPC), α₂-microglobulin-related protein (A2m), 24p3/uterocalin (24p3), von Ebners gland protein 1 (VEGP 1), von Ebners gland protein 2 (VEGP 2), and Major allergen Can f1 precursor (ALL-1).
26. The pharmaceutical composition of item 25, wherein the lipocalin mutein is selected from the group consisting of human neutrophil gelatinase associated lipocalin (hNGAL), human tear lipocalin (hTLPC), human apolipoprotein D (APO D) and the bilin-binding protein of *Pieris brassicae.*
27. The pharmaceutical composition of item 25 or 26, wherein said lipocalin mutein has at least 50%, 60%, 70%, 75%, 80%, 85%, 90% or 95% sequence homology or sequence identity with human tear lipocalin, human neutrophil gelatinase associated lipocalin, human apolipoprotein D or the bilin-binding protein of *Pieris brassicae.*
28. The pharmaceutical composition of any one of items 25 to 27, wherein the mutein binds a given target with detectable affinity.
29. The pharmaceutical composition of item 28, wherein the target is VEGF, IL-4R alpha, VEGF-R2; CTLA-4 or c-Met.
30. The pharmaceutical composition of any one of items 25 to 29, wherein the lipocalin mutein comprises at least one mutated amino acid residues at any sequence position in the four peptide loops AB, CD, EF, and GH encompassing the natural lipocalin binding pocket.
31. The pharmaceutical composition of item 30, wherein the lipocalin mutein comprises at least 2, 3, 4, 5, 6, 7, 8, 9, 10, 1 1, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 mutated amino acid residues at any sequence position in the four peptide loops AB, CD, EF, and GH encompassing the natural lipocalin binding pocket.
32. The pharmaceutical composition of any one of items 25 to 31, wherein the lipocalin mutein comprises at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 mutated amino acid residues at any sequence positions corresponding to the sequence positions 24-36, 53-66, 79-84, and 103-110 of the linear polypeptide sequence of native mature human tear lipocalin (SWISS-PROT Data Bank Accession Number P31025).
33. The pharmaceutical composition of item 32, wherein the lipocalin mutein comprises at least 2, 3, 4, 5, 6, 7, 8, 9, 10, 11, 12, 13, 14, 15, 16, 17, or 18 mutated amino acid residues at any sequence positions corresponding to sequence positions 26-34, 56-58, 80, 83, 104-106 and 108 of the linear polypeptide sequence of native mature human tear lipocalin.
34. The pharmaceutical composition of any one of items 25 to 33, wherein the lipocalin mutein comprises, consist essentially of or consists of the amino acid sequence set forth in any one of SEQ ID NOs. 1 - 48 and 50 - 110.
35. The pharmaceutical formulation of any one of items 1 to 34, wherein the formulation is in form of an implant or implantable device.
36. The pharmaceutical composition of any one of items 1 to 35, wherein about 40 - about 70% of the released lipocalin mutein are active.
37. A method for controlled systemic or local delivery of a lipocalin mutein to a subject comprising administering to the subject the pharmaceutical formulation according to any one of items 1 to 36.
38. The method of item 37, wherein the controlled local delivery of a lipocalin mutein is facilitated by administration to a confined body compartment or organ of the subject.
39. The method of item 38, wherein the confined body compartment or organ is an eye, a joint, or a bodily lumen or cavity.
40. The method of any one of items 37 to 39, wherein the conjugate or formulation is administered orally, by inhalation, mucosal delivery, subcutaneous injection, intramuscular injection, intravenous injection, intravitreal injection, intraarticular injection, intracranial injection, intraspinal injection, intratumoral injection or implantation.
41. The method of any one of items 37 to 40, wherein the subject is a mammal.
42. The method of item 41, wherein the mammal is a human.
43. A method of making a controlled release composition comprising: combining an organic phase comprising a lipocalin mutein and a polymer with an aqueous phase, and recovering said composition.
44. The method of item 43, further comprising at least one co-solvent in said organic phase.
45. The method of item 44, wherein said at least cosolvent is selected from the group consisting of dimethyl sulfoxide, dimethyl formamide, n-methylpyrrolidinone, PEG200, PEG400, methyl alcohol, ethyl alcohol, isopropyl alcohol, benzyl alcohol, water and mixtures thereof.
46. The method of any one of items 40 to 45, further comprising an emulsifying agent in said aqueous phase.
47. The method of item 46, wherein said emulsifying agent is selected from the group consisting of polyvinyl alcohol, albumin, lecithin, vitamin E-TPGS and polysorbates.
48. The method of item 46 or 47, wherein said emulsifying agent is at a final concentration ranging from about 0.1 to about 10% (w/w).
49. The method of any one of items 43 to 48, wherein said organic phase comprises a solvent selected from the group consisting of dichloromethane, ethyl acetate, benzyl alcohol, acetone, acetic acid and propylene carbonate.
50. The method of any one of items 43 to 49, wherein the aqueous phase comprises an organic ion, wherein said organic ion is present in an aqueous phase to reduce degradation of said lipocalin mutein.
51. The method of item 50, wherein said organic ion is at a final concentration ranging from about 0.1 to 1000 mM.
52. The method of item 50 or 51, wherein said organic ion is selected from the group consisting of carboxylate, sulfate, phosphate, dodecylsulfate, trifluoromethyl-p-toluate, 2-naphthalene sulfonate, 2,3-naphthalene dicarboxylate, 1-hydroxy-2-naphthoate, 3-hydroxy-2-naphthoate, 2-naphthoate, and salicylsalicylate.
53. The method of any one of items 43 to 52, wherein said controlled release composition is selected from the group consisting of microparticles and nanoparticles.
54. The method of item 53, wherein said microparticles and nanoparticles are biodegradable.
55. The method of any one of items 44 to 54, wherein said polymer is selected from the group consisting of polylactides, polyglycolides, poly(lactide-co-glycolide)s, poly(lactic acid), poly (glycolic acid), poly (lactic acid-co-glycolic acid), polycaprolactones, polycarbonates, polyesteramides, polyanhydrides, poly(amino acids), polyorthoesters, polyacetyls, polycyanoacrylates, polyetheresters, polydioxanones, polyalkylene alkylates, copolymers of polyethylene glycol and polyorthoester, biodegradable polyurethanes, and blends and copolymers thereof.
56. The method of any one of items 44 to 55, wherein the organic phase and aqueous phase are combined using an emulsion process.
57. The method of item 56, wherein said emulsion process is selected from the group consisting of oil-in-water and water-oil-water.
58. A controlled release composition made by the method as defined in any one of items 44 to 57.
59. Use of the pharmaceutical formulation according to any one of items 1 to 36 or the controlled release composition according to item 58 for the controlled delivery of the lipocalin mutein, for extending the *in vivo* half-life of the lipocalin mutein, for increasing the bioavailability of the lipocalin mutein, or for decreasing the immunogenicity of the lipocalin mutein upon administration to a subject.
60. Method for treating a disease or disorder, comprising administering the pharmaceutical formulation according to any one of items 1 to 36 or the controlled release composition according to item 58 to a subject in need thereof.
61. The method of item 60, wherein the disease or disorder is connected to increased vascularisation.
62. The method of item 60 or 61, wherein the disease or disorder is selected from the group consisting of cancer, asthma, arthritis, inflammation, chronic obstructive pulmonary disease (COPD), pulmonary hypertension, neovascular wet age-related macular degeneration (AMD), diabetic retinopathy, macular edema, retinopathy of prematurity and retinal vein occlusion.

## Claims

1. A mutein of human tear lipocalin (hTLc) having detectable binding affinity to the human Met receptor tyrosine kinase (c-Met) or a fragment thereof, wherein said mutein has an amino acid sequence as set forth in SEQ ID NO: 49 or wherein said mutein of human tear lipocalin (hTLc) has 70 to 98 % sequence identity with a lipocalin mutein of SEQ ID NO: 49, and wherein said mutein is conjugated to a maleimide-derivatized polyethylenglycol (PEG), which is reacted with free thiol groups of cysteine residues in the mutein, wherein these cysteine side chains may naturally occur in the protein or may be artificially introduced by mutagenesis.

2. The mutein according to claim 1, wherein the mutein has a binding affinity to c-Met with a K_{D} of 20 nM or less.

3. The mutein according to claim 1 or 2, wherein the maleimide-derivatized PEG is branched or linear.

4. The mutein of any one of claims 1 to 3, wherein the maleimide-derivatized PEG has a molecular weight in the range of 300 to 70,000 Da.

5. The mutein of any one of claims 1-4, wherein the mutein is conjugated to PEG20 or PEG40.

6. A pharmaceutical composition comprising the mutein of any one of claims 1-5 and a pharmaceutically acceptable excipient.

7. The mutein of any one of claims 1-5 for use in binding human Met receptor tyrosine kinase (c-Met) or a fragment thereof.

## Patentansprüche

1. Mutein von menschlichem Tränenlipocalin (hTLc) mit nachweisbarer Bindungsaffinität zu menschlicher Met-Rezeptor-Tyrosinkinase (c-Met) oder einem Fragment davon, wobei das Mutein eine Aminosäuresequenz wie in SEQ ID Nr. 49 angegeben aufweist oder wobei das Mutein von menschlichem Tränenlipocalin (hTLc) 70 bis 98 % Sequenzidentität mit einem Lipocalin-Mutein von SEQ ID Nr. 49 aufweist, und wobei das Mutein mit einem Maleimid-derivatisierten Polyethylenglykol (PEG) konjugiert ist, welches mit freien Thiolgruppen von Cysteinresten im Mutein zur Reaktion gebracht wird, wobei diese Cystein-Seitenketten natürlich im Protein vorkommen können oder durch Mutagenese künstlich eingebracht sein können.

2. Mutein nach Anspruch 1, wobei das Mutein eine Bindungsaffinität zu c-Met mit einem K_{D} von 20 nM oder weniger aufweist.

3. Mutein nach Anspruch 1 oder 2, wobei das Maleimid-derivatisierte PEG verzweigt oder linear ist.

4. Mutein nach einem der Ansprüche 1 bis 3, wobei das Maleimid-derivatisierte PEG ein Molekulargewicht im Bereich von 300 bis 70.000 Da aufweist.

5. Mutein nach einem der Ansprüche 1-4, wobei das Mutein mit PEG20 oder PEG40 konjugiert ist.

6. Pharmazeutische Zusammensetzung, umfassend das Mutein nach irgendeinem der Ansprüche 1-5 und einen pharmazeutisch annehmbaren Trägerstoff.

7. Mutein nach einem der Ansprüche 1-5 zur Verwendung bei der Bindung von menschlicher Met-Rezeptor-Tyrosinkinase (c-Met) oder eines Fragments davon.

## Revendications

1. Mutéine de lipocaline lacrymale humaine (hTLc) présentant une affinité de liaison détectable au récepteur tyrosine kinase Met humain (c-Met) ou à un fragment de celui-ci, sachant que ladite mutéine présente une séquence d'acide aminé telle qu'exposée dans SEQ ID N°: 49 ou sachant que ladite mutéine de lipocaline lacrymale humaine (hTLc) a 70 à 98 % d'identité de séquence avec une mutéine de lipocaline de SEQ ID N°: 49, et sachant que ladite mutéine est conjuguée à un polyéthylène glycol (PEG) dérivé du maléimide, lequel est mis en réaction avec des groupes thiol libres de résidus de cystéine dans la mutéine, sachant que ces chaînes latérales de cystéine peuvent survenir naturellement dans la protéine ou être introduits artificiellement par mutagenèse.

2. La mutéine selon la revendication 1, sachant que la mutéine présente une affinité de liaison au c-Met avec un K_{D} de 20 nM ou moins.

3. La mutéine selon la revendication 1 ou 2, sachant le PED dérivé du maléimide est ramifié ou linéaire.

4. La mutéine de l'une quelconque des revendications 1 à 3, sachant que le PEG dérivé du maléimide a un poids moléculaire compris dans la plage de 300 à 70 000 Da.

5. La mutéine de l'une quelconque des revendications 1 à 4, sachant que la mutéine est conjuguée à PEG20 ou PEG40.

6. Composition pharmaceutique comprenant la mutéine de l'une quelconque des revendications 1 à 5 et un excipient pharmaceutiquement acceptable.

7. La mutéine de l'une quelconque des revendications 1 à 5, destinée à être utilisée dans la liaison du récepteur tyrosine kinase Met humain (c-Met) ou d'un fragment de celui-ci.
